(19) Europäisches Patentamt
European Patent Office
Office européen des brevets

(11) EP 4 756 027 A1

# (12) EUROPEAN PATENT APPLICATION

published in accordance with Art. 153(4) EPC

(43) Date of publication:
**10.06.2026 Bulletin 2026/24**

(21) Application number: **24850947.3**

(22) Date of filing: **02.08.2024**

(51) International Patent Classification (IPC):
***C12N 15/113** (2010.01)* ***A61K 31/713** (2006.01)*
***A61P 31/20** (2006.01)*

(52) Cooperative Patent Classification (CPC):
**A61K 31/713; A61P 31/20; C12N 15/113**

(86) International application number:
**PCT/CN2024/109635**

(87) International publication number:
**WO 2025/031302 (13.02.2025 Gazette 2025/07)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **04.08.2023 CN 202310982666**
**04.12.2023 CN 202311654515**
**26.01.2024 CN 202410114631**

(71) Applicant: **Chia Tai Tianqing Pharmaceutical Group Co., Ltd.**
**Lianyungang, Jiangsu 222062 (CN)**

(72) Inventors:
- **XU, Hongjiang**
**Lianyungang, Jiangsu 222062 (CN)**
- **XU, Yaqiong**
**Lianyungang, Jiangsu 222062 (CN)**
- **ZHENG, Jiajia**
**Lianyungang, Jiangsu 222062 (CN)**
- **GE, Xingfeng**
**Lianyungang, Jiangsu 222062 (CN)**
- **YANG, Ling**
**Lianyungang, Jiangsu 222062 (CN)**

(74) Representative: **WSL Patentanwälte Partnerschaft mbB**
**Kaiser-Friedrich-Ring 98**
**65185 Wiesbaden (DE)**

Remarks:
The complete document including Reference Table(s) and the Sequence Listing(s) can be downloaded from the EPO website

# (54) DOUBLE-STRANDED RIBONUCLEIC ACID TARGETING PD-L1

(57) Provided are a small interfering ribonucleic acid targeting PD-L1, a pharmaceutically acceptable salt thereof, or a ligand conjugate thereof. Further provided are a pharmaceutical composition containing the small interfering ribonucleic acid, the pharmaceutically acceptable salt thereof, or the ligand conjugate thereof, and a therapeutic use of the small interfering ribonucleic acid, the pharmaceutically acceptable salt thereof, or the ligand conjugate thereof.

EP 4 756 027 A1

## Description

### CROSS-REFERENCE TO RELATED APPLICATIONS

The present application claims the priority and benefit to Chinese Patent Application No. 202310982666.0 filed with the China National Intellectual Property Administration on Aug. 4, 2023, Chinese Patent Application No. 202311654515.9 filed with the China National Intellectual Property Administration on Dec. 4, 2023, and Chinese Patent Application No. 202410114631.X filed with the China National Intellectual Property Administration on Jan. 26, 2024, which are incorporated herein by reference in their entirety.

### TECHNICAL FIELD

The present application relates to the field of biomedicine, and in particular, to a double-stranded ribonucleic acid (dsRNA), a pharmaceutically acceptable salt thereof, or a ligand conjugate thereof, or a pharmaceutical composition, which can be used for inhibiting PD-L1.

### BACKGROUND

Viral hepatitis B, abbreviated as hepatitis B, is a disease caused by hepatitis B virus (HBV) infection of the body. Hepatitis B virus is a hepatotropic virus, and it is mainly present in and damages hepatocytes, causing inflammation, necrosis, fibrosis, and the like, in hepatocytes. Viral hepatitis B is classified into acute and chronic ones. Acute hepatitis B can be mostly self-healed in adults by their own immune mechanisms. However, chronic hepatitis B (CHB) has become a great challenge for global health care and is also the main cause of chronic liver disease, cirrhosis and hepatic carcinoma (HCC) (Edward J. G., et al., The oral toll-like receptor-7 agonist GS-9620 in patients with chronic hepatitis B virus infection. Journal of Hepatology (2015); 63: 320-328).

Currently approved anti-HBV drugs on the market are mainly immunomodulators (interferon-a, polyethylene glycol interferon-$\alpha$-2a, etc.) and antiviral therapeutic agents (lamivudine, adefovir dipivoxil, entecavir, telbivudine, tenofovir, clevudine, etc.). Among them, the antiviral therapeutic agents belong to the nucleoside or nucleotide drugs, and the action mechanism thereof is to inhibit the synthesis of HBV DNA, but the HBsAg level cannot be directly reduced. As with the extended therapy, the nucleoside or nucleotide drugs show HBsAg clearance rates similar to those observed naturally (Janssen et al., Lancet (2005), 365, 123-129; Marcellin et al., N. Engl.JMed. (2004), 351, 1206-1217; Buster et al., Hepatology (2007), 46, 388-394).

Immune checkpoint inhibitors are immunotherapeutics that have been widely studied in recent years, mainly focusing on the PD-1/PD-L1 pathway. PD-L1 is a transmembrane protein of 40 kDa. Its binding to PD-1 can inhibit the activation of T cells, thereby maintaining immune homeostasis. Generally, the expression of PD-L1 is induced in most lymphocytes, such as epithelial cells, endothelial cells, and some tumor cells. In normal liver tissues, PD-L1 is mainly expressed in the portal vein area and is expressed at a lower rate in both liver parenchymal cells and liver endothelial cells. The viral infection can significantly increase the expression of PD-L1 in hepatocytes. During HBV infections, the expression of PD-L1 on the surface of hepatocytes is up-regulated, and PD-L1 interacts with PD-1 receptors on the surface of T cells, resulting in T cell depletion. Therefore, inhibiting the expression of PD-L1 can enhance the functionality of T cells, help restore immune responses, and trigger the immune-mediated clearance of HBV-infected hepatocytes, which is crucial for the cure of chronic hepatitis B.

Small interfering RNAs (siRNAs) can inhibit the expression of a target gene by inhibiting or blocking the translation or transcription of the target gene in a sequence-specific manner on the basis of the RNA interference (RNAi) mechanism, and exert an inhibitory effect at the mRNA level, thereby achieving the purpose of treating diseases.

### BRIEF SUMMARY

In one aspect, the present application provides a double-stranded ribonucleic acid (dsRNA), a pharmaceutically acceptable salt thereof, or a ligand conjugate thereof, wherein the dsRNA comprises a sense strand and an antisense strand; the sense strand comprises a nucleotide sequence with a length of not greater than 23 nucleotides, and the antisense strand comprises a nucleotide sequence with a length of not greater than 25 nucleotides; the sense strand or the antisense strand is optionally modified; the double-stranded ribonucleic acid (dsRNA), the pharmaceutically acceptable salt thereof, or the ligand conjugate thereof targets PD-L1 mRNA.

In another aspect, the present application provides a double-stranded ribonucleic acid (dsRNA), a pharmaceutically acceptable salt thereof, or a ligand conjugate thereof, wherein the dsRNA comprises a sense strand and an antisense strand; the sense strand comprises at least 15 consecutive nucleotides in the nucleotide sequence set forth in SEQ ID NO. 9 and has a length of not greater than 23 nucleotides, and the antisense strand comprises at least 15

consecutive nucleotides in the nucleotide sequence set forth in SEQ ID NO. 10 and has a length of not greater than 25 nucleotides; or, the sense strand comprises at least 15 consecutive nucleotides in the nucleotide sequence set forth in SEQ ID NO. 11 and has a length of not greater than 23 nucleotides, and the antisense strand comprises at least 15 consecutive nucleotides in the nucleotide sequence set forth in SEQ ID NO. 12 and has a length of not greater than 25 nucleotides; or, the sense strand comprises at least 15 consecutive nucleotides in the nucleotide sequence set forth in SEQ ID NO. 13 and has a length of not greater than 21 nucleotides, and the antisense strand comprises at least 15 consecutive nucleotides in the nucleotide sequence set forth in SEQ ID NO. 14 and has a length of not greater than 23 nucleotides; or, the sense strand comprises at least 15 consecutive nucleotides in the nucleotide sequence set forth in SEQ ID NO. 15 and has a length of not greater than 23 nucleotides, and the antisense strand comprises at least 15 consecutive nucleotides in the nucleotide sequence set forth in SEQ ID NO. 16 and has a length of not greater than 25 nucleotides; or, the sense strand comprises at least 15 consecutive nucleotides in the nucleotide sequence set forth in SEQ ID NO. 17 and has a length of not greater than 23 nucleotides, and the antisense strand comprises at least 15 consecutive nucleotides in the nucleotide sequence set forth in SEQ ID NO. 18 and has a length of not greater than 25 nucleotides; or, the sense strand comprises at least 15 consecutive nucleotides in the nucleotide sequence set forth in SEQ ID NO. 19 and has a length of not greater than 23 nucleotides, and the antisense strand comprises at least 15 consecutive nucleotides in the nucleotide sequence set forth in SEQ ID NO. 20 and has a length of not greater than 25 nucleotides; or, the sense strand comprises at least 15 consecutive nucleotides in the nucleotide sequence set forth in SEQ ID NO. 21 and has a length of not greater than 23 nucleotides, and the antisense strand comprises at least 15 consecutive nucleotides in the nucleotide sequence set forth in SEQ ID NO. 22 and has a length of not greater than 25 nucleotides; or, the sense strand comprises at least 15 consecutive nucleotides in the nucleotide sequence set forth in SEQ ID NO. 23 and has a length of not greater than 23 nucleotides, and the antisense strand comprises at least 15 consecutive nucleotides in the nucleotide sequence set forth in SEQ ID NO. 24 and has a length of not greater than 25 nucleotides; or, the sense strand comprises at least 15 consecutive nucleotides in the nucleotide sequence set forth in SEQ ID NO. 25 and has a length of not greater than 23 nucleotides, and the antisense strand comprises at least 15 consecutive nucleotides in the nucleotide sequence set forth in SEQ ID NO. 26 and has a length of not greater than 25 nucleotides; or, the sense strand comprises at least 15 consecutive nucleotides in the nucleotide sequence set forth in SEQ ID NO. 27 and has a length of not greater than 23 nucleotides, and the antisense strand comprises at least 15 consecutive nucleotides in the nucleotide sequence set forth in SEQ ID NO. 28 and has a length of not greater than 25 nucleotides; or, the sense strand comprises at least 15 consecutive nucleotides in the nucleotide sequence set forth in SEQ ID NO. 29 and has a length of not greater than 21 nucleotides, and the antisense strand comprises at least 15 consecutive nucleotides in the nucleotide sequence set forth in SEQ ID NO. 30 and has a length of not greater than 23 nucleotides; or, the sense strand comprises at least 15 consecutive nucleotides in the nucleotide sequence set forth in SEQ ID NO. 53 and has a length of not greater than 23 nucleotides, and the antisense strand comprises at least 15 consecutive nucleotides in the nucleotide sequence set forth in SEQ ID NO. 54 and has a length of not greater than 25 nucleotides:

sense strand: 5’-AGCAAUAUGACAAUUGAAUGA-3’ (SEQ ID NO. 9),

antisense strand: 5’-UCAUUCAAUUGUCAUAUUGCUAC-3’ (SEQ ID NO. 10);

sense strand: 5’-AUUUACUGUCACGGUUCCCAA-3’ (SEQ ID NO. 11),

antisense strand: 5’-UUGGGAACCGUGACAGUAAUAGC-3’ (SEQ ID NO. 12);

sense strand: 5’-CUAUUUAUUUUGAGUCUGU-3’ (SEQ ID NO. 13),

antisense strand: 5’-ACAGACUCAAAAUAAAUAGGA-3’ (SEQ ID NO. 14);

sense strand: 5’-UGAAGAUAUAUUGUAGUAGAU-3’ (SEQ ID NO. 15),

antisense strand: 5’-AUCUACUACAAUAUAUCUUCAAA-3’ (SEQ ID NO. 16);

sense strand: 5’-UUGUCUCAUGUUUCAUCGUAA-3’ (SEQ ID NO. 17),

antisense strand: 5’-UUACGAUGAAACAUGAGACAAAA-3’ (SEQ ID NO. 18);

sense strand: 5’-CUGUGCAGUAUCUGUUCCAUU-3’ (SEQ ID NO. 19),

antisense strand: 5'-AAUGGAACAGAUACUGCACAGAC-3' (SEQ ID NO. 20);

sense strand: 5'-AAUGAAAGGACUCACUUGGUA-3' (SEQ ID NO. 21),

antisense strand: 5'-UACCAAGUGAGUCCUUUCAUUUG-3' (SEQ ID NO. 22);

sense strand: 5'-GCUGCAUGAUCAGCUAUGGUA-3' (SEQ ID NO. 23),

antisense strand: 5'-UACCAUAGCUGAUCAUGCAGCGG-3' (SEQ ID NO. 24);

sense strand: 5'-GGUGCUUGGUCUCCUCUAUAA-3' (SEQ ID NO. 25),

antisense strand: 5'-UUAUAGAGGAGACCAAGCACCUU-3' (SEQ ID NO. 26);

sense strand: 5'-AUUGGUCAUCCCAGAACUACA-3' (SEQ ID NO. 27),

antisense strand: 5'-UGUAGUUCUGGGAUGACCAAUUC-3' (SEQ ID NO. 28);

sense strand: 5'-UAUUUAUUUUGAGUCUGUG-3' (SEQ ID NO. 29),

antisense strand: 5'-CACAGACUCAAAAUAAAUAGG-3' (SEQ ID NO. 30);

sense strand: 5'-AUUUACUGUCACGGUUCCCAA-3' (SEQ ID NO. 53),

antisense strand: 5'-UUGGGAACCGUGACAGUAAAUGC-3' (SEQ ID NO. 54);

the sense strand or the antisense strand is optionally modified.

In another aspect, the present application provides a pharmaceutical composition comprising the dsRNA, the pharmaceutically acceptable salt thereof, or the ligand conjugate thereof of the present application, and a pharmaceutically acceptable carrier or excipient.

In another aspect, the present application provides a kit for treating and/or preventing hepatitis B virus infection, comprising the dsRNA, the pharmaceutically acceptable salt thereof, or the ligand conjugate thereof, or the pharmaceutical composition of the present application, and optionally an instruction for use.

In another aspect, the present application provides a method for treating and/or preventing hepatitis B virus infection, comprising administering to a subject the dsRNA, the pharmaceutically acceptable salt thereof, or the ligand conjugate thereof, or the pharmaceutical composition of the present application.

In some embodiments, the present application provides a method for treating and/or preventing hepatitis B virus infection, comprising administering to a subject in need of the treatment and/or prevention a therapeutically/prophylactically effective amount of the dsRNA, the pharmaceutically acceptable salt thereof, or the ligand conjugate thereof, or the pharmaceutical composition of the present application.

In another aspect, the present application provides use of the dsRNA, the pharmaceutically acceptable salt thereof, the ligand conjugate thereof, or the pharmaceutical composition of the present application in preparing a medicament for treating and/or preventing hepatitis B virus infection.

In another aspect, the present application provides use of the dsRNA, the pharmaceutically acceptable salt thereof, the ligand conjugate thereof, or the pharmaceutical composition of the present application in treating and/or preventing hepatitis B virus infection.

In another aspect, the present application provides the dsRNA, the pharmaceutically acceptable salt thereof, or the ligand conjugate thereof, or the pharmaceutical composition of the present application for use in treating and/or preventing hepatitis B virus infection.

In some embodiments, the hepatitis B virus infection may be at any stage of the disease, such as acute hepatitis B or chronic hepatitis B, or a liver disease caused by hepatitis B virus infection, including hepatitis, hepatic fibrosis, cirrhosis, hepatic failure, or hepatic cancer. In some embodiments, the hepatitis B virus infection is chronic hepatitis B.

In some embodiments, the dsRNA, the pharmaceutically acceptable salt thereof, the ligand conjugate thereof, or the pharmaceutical composition of the present application is used as a single therapeutic agent for treating and/or preventing hepatitis B virus infection.

In some embodiments, the dsRNA, the pharmaceutically acceptable salt thereof, the ligand conjugate thereof, or the pharmaceutical composition of the present application is used in combination with an additional therapeutic agent for

treating and/or preventing hepatitis B virus infection.

## SUMMARY

The exemplary embodiments of the present application will be described below; however, it will be appreciated by those skilled in the art that the protection scope of the present application is not limited thereto, and that various modifications, alterations, or changes can be made based on the spirit and conception of the present application, and the content with these modifications, alterations, or changes shall still fall within the scope of the present application. The present application provides a double-stranded ribonucleic acid (dsRNA), a pharmaceutically acceptable salt thereof, or a ligand conjugate thereof, wherein the dsRNA comprises a sense strand and an antisense strand; the sense strand comprises at least 15 consecutive nucleotides in the nucleotide sequence set forth in SEQ ID NO. 9 and has a length of not greater than 23 nucleotides, and the antisense strand comprises at least 15 consecutive nucleotides in the nucleotide sequence set forth in SEQ ID NO. 10 and has a length of not greater than 25 nucleotides; or, the sense strand comprises at least 15 consecutive nucleotides in the nucleotide sequence set forth in SEQ ID NO. 11 and has a length of not greater than 23 nucleotides, and the antisense strand comprises at least 15 consecutive nucleotides in the nucleotide sequence set forth in SEQ ID NO. 12 and has a length of not greater than 25 nucleotides; or, the sense strand comprises at least 15 consecutive nucleotides in the nucleotide sequence set forth in SEQ ID NO. 13 and has a length of not greater than 21 nucleotides, and the antisense strand comprises at least 15 consecutive nucleotides in the nucleotide sequence set forth in SEQ ID NO. 14 and has a length of not greater than 23 nucleotides; or, the sense strand comprises at least 15 consecutive nucleotides in the nucleotide sequence set forth in SEQ ID NO. 15 and has a length of not greater than 23 nucleotides, and the antisense strand comprises at least 15 consecutive nucleotides in the nucleotide sequence set forth in SEQ ID NO. 16 and has a length of not greater than 25 nucleotides; or, the sense strand comprises at least 15 consecutive nucleotides in the nucleotide sequence set forth in SEQ ID NO. 17 and has a length of not greater than 23 nucleotides, and the antisense strand comprises at least 15 consecutive nucleotides in the nucleotide sequence set forth in SEQ ID NO. 18 and has a length of not greater than 25 nucleotides; or, the sense strand comprises at least 15 consecutive nucleotides in the nucleotide sequence set forth in SEQ ID NO. 19 and has a length of not greater than 23 nucleotides, and the antisense strand comprises at least 15 consecutive nucleotides in the nucleotide sequence set forth in SEQ ID NO. 20 and has a length of not greater than 25 nucleotides; or, the sense strand comprises at least 15 consecutive nucleotides in the nucleotide sequence set forth in SEQ ID NO. 21 and has a length of not greater than 23 nucleotides, and the antisense strand comprises at least 15 consecutive nucleotides in the nucleotide sequence set forth in SEQ ID NO. 22 and has a length of not greater than 25 nucleotides; or, the sense strand comprises at least 15 consecutive nucleotides in the nucleotide sequence set forth in SEQ ID NO. 23 and has a length of not greater than 23 nucleotides, and the antisense strand comprises at least 15 consecutive nucleotides in the nucleotide sequence set forth in SEQ ID NO. 24 and has a length of not greater than 25 nucleotides; or, the sense strand comprises at least 15 consecutive nucleotides in the nucleotide sequence set forth in SEQ ID NO. 25 and has a length of not greater than 23 nucleotides, and the antisense strand comprises at least 15 consecutive nucleotides in the nucleotide sequence set forth in SEQ ID NO. 26 and has a length of not greater than 25 nucleotides; or, the sense strand comprises at least 15 consecutive nucleotides in the nucleotide sequence set forth in SEQ ID NO. 27 and has a length of not greater than 23 nucleotides, and the antisense strand comprises at least 15 consecutive nucleotides in the nucleotide sequence set forth in SEQ ID NO. 28 and has a length of not greater than 25 nucleotides; or, the sense strand comprises at least 15 consecutive nucleotides in the nucleotide sequence set forth in SEQ ID NO. 29 and has a length of not greater than 21 nucleotides, and the antisense strand comprises at least 15 consecutive nucleotides in the nucleotide sequence set forth in SEQ ID NO. 30 and has a length of not greater than 23 nucleotides; or, the sense strand comprises at least 15 consecutive nucleotides in the nucleotide sequence set forth in SEQ ID NO. 53 and has a length of not greater than 23 nucleotides, and the antisense strand comprises at least 15 consecutive nucleotides in the nucleotide sequence set forth in SEQ ID NO. 54 and has a length of not greater than 25 nucleotides: sense strand: 5’-AGCAAUAUGACAAUUGAAUGA-3’ (SEQ ID NO. 9),

antisense strand: 5’-UCAUUCAAUUGUCAUAUUGCUAC-3’ (SEQ ID NO. 10);

sense strand: 5’-AUUUACUGUCACGGUUCCCAA-3’ (SEQ ID NO. 11),

antisense strand: 5’-UUGGGAACCGUGACAGUAAUAGC-3’ (SEQ ID NO. 12);

sense strand: 5’-CUAUUUAUUUUGAGUCUGU-3’ (SEQ ID NO. 13),

antisense strand: 5’-ACAGACUCAAAAUAAAUAGGA-3’ (SEQ ID NO. 14);

sense strand: 5'-UGAAGAUAUAUUGUAGUAGAU-3' (SEQ ID NO. 15),

antisense strand: 5'-AUCUACUACAAUAUAUCUUCAAA-3' (SEQ ID NO. 16);

sense strand: 5'-UUGUCUCAUGUUUCAUCGUAA-3' (SEQ ID NO. 17),

antisense strand: 5'-UUACGAUGAAACAUGAGACAAAA-3' (SEQ ID NO. 18);

sense strand: 5'-CUGUGCAGUAUCUGUUCCAUU-3' (SEQ ID NO. 19),

antisense strand: 5'-AAUGGAACAGAUACUGCACAGAC-3' (SEQ ID NO. 20);

sense strand: 5'-AAUGAAAGGACUCACUUGGUA-3' (SEQ ID NO. 21),

antisense strand: 5'-UACCAAGUGAGUCCUUUCAUUUG-3' (SEQ ID NO. 22);

sense strand: 5'-GCUGCAUGAUCAGCUAUGGUA-3' (SEQ ID NO. 23),

antisense strand: 5'-UACCAUAGCUGAUCAUGCAGCGG-3' (SEQ ID NO. 24);

sense strand: 5'-GGUGCUUGGUCUCCUCUAUAA-3' (SEQ ID NO. 25),

antisense strand: 5'-UUAUAGAGGAGACCAAGCACCUU-3' (SEQ ID NO. 26);

sense strand: 5'-AUUGGUCAUCCCAGAACUACA-3' (SEQ ID NO. 27),

antisense strand: 5'-UGUAGUUCUGGGAUGACCAAUUC-3' (SEQ ID NO. 28);

sense strand: 5'-UAUUUAUUUUGAGUCUGUG-3' (SEQ ID NO. 29),

antisense strand: 5'-CACAGACUCAAAAUAAAUAGG-3' (SEQ ID NO. 30);

sense strand: 5'-AUUUACUGUCACGGUUCCCAA-3' (SEQ ID NO. 53),

antisense strand: 5'-UUGGGAACCGUGACAGUAAAUGC-3' (SEQ ID NO. 54);

the sense strand or the antisense strand is optionally modified.

In some embodiments, the sense strand comprises at least 15 consecutive nucleotides in the nucleotide sequence set forth in SEQ ID NO. 9, 11, 15, 17, 19, 21, 23, 25, 27, or 53 and has a length of not greater than 23 nucleotides, wherein the at least 15 consecutive nucleotides may be selected from the group consisting of 15, 16, 17, 18, 19, 20, 21, 22, and 23 consecutive nucleotides.

In some embodiments, the antisense strand comprises at least 15 consecutive nucleotides in the nucleotide sequence set forth in SEQ ID NO. 10, 12, 16, 18, 20, 22, 24, 26, 28, or 54 and has a length of not greater than 25 nucleotides, wherein the at least 15 consecutive nucleotides may be selected from the group consisting of 15, 16, 17, 18, 19, 20, 21, 22, 23, 24, and 25 consecutive nucleotides.

In some embodiments, the sense strand comprises at least 15 consecutive nucleotides in the nucleotide sequence set forth in SEQ ID NO. 13 or 29 and has a length of not greater than 21 nucleotides, wherein the at least 15 consecutive nucleotides may be selected from the group consisting of 15, 16, 17, 18, 19, 20, and 21 consecutive nucleotides.

In some embodiments, the antisense strand comprises at least 15 consecutive nucleotides in the nucleotide sequence set forth in SEQ ID NO. 14 or 30 and has a length of not greater than 23 nucleotides, wherein the at least 15 consecutive nucleotides may be selected from the group consisting of 15, 16, 17, 18, 19, 20, 21, 22, and 23 consecutive nucleotides.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand; the sense strand comprises the nucleotide sequence set forth in SEQ ID NO. 9 and has a length of not greater than 23 nucleotides, and the antisense strand comprises the nucleotide sequence set forth in SEQ ID NO. 10

and has a length of not greater than 25 nucleotides; or, the sense strand comprises the nucleotide sequence set forth in SEQ ID NO. 11 and has a length of not greater than 23 nucleotides, and the antisense strand comprises the nucleotide sequence set forth in SEQ ID NO. 12 and has a length of not greater than 25 nucleotides; or, the sense strand comprises the nucleotide sequence set forth in SEQ ID NO. 13 and has a length of not greater than 21 nucleotides, and the antisense strand comprises the nucleotide sequence set forth in SEQ ID NO. 14 and has a length of not greater than 23 nucleotides; or, the sense strand comprises the nucleotide sequence set forth in SEQ ID NO. 15 and has a length of not greater than 23 nucleotides, and the antisense strand comprises the nucleotide sequence set forth in SEQ ID NO. 16 and has a length of not greater than 25 nucleotides; or, the sense strand comprises the nucleotide sequence set forth in SEQ ID NO. 17 and has a length of not greater than 23 nucleotides, and the antisense strand comprises the nucleotide sequence set forth in SEQ ID NO. 18 and has a length of not greater than 25 nucleotides; or, the sense strand comprises the nucleotide sequence set forth in SEQ ID NO. 19 and has a length of not greater than 23 nucleotides, and the antisense strand comprises the nucleotide sequence set forth in SEQ ID NO. 20 and has a length of not greater than 25 nucleotides; or, the sense strand comprises the nucleotide sequence set forth in SEQ ID NO. 21 and has a length of not greater than 23 nucleotides, and the antisense strand comprises the nucleotide sequence set forth in SEQ ID NO. 22 and has a length of not greater than 25 nucleotides; or, the sense strand comprises the nucleotide sequence set forth in SEQ ID NO. 23 and has a length of not greater than 23 nucleotides, and the antisense strand comprises the nucleotide sequence set forth in SEQ ID NO. 24 and has a length of not greater than 25 nucleotides; or, the sense strand comprises the nucleotide sequence set forth in SEQ ID NO. 25 and has a length of not greater than 23 nucleotides, and the antisense strand comprises the nucleotide sequence set forth in SEQ ID NO. 26 and has a length of not greater than 25 nucleotides; or, the sense strand comprises the nucleotide sequence set forth in SEQ ID NO. 27 and has a length of not greater than 23 nucleotides, and the antisense strand comprises the nucleotide sequence set forth in SEQ ID NO. 28 and has a length of not greater than 25 nucleotides; or, the sense strand comprises the nucleotide sequence set forth in SEQ ID NO. 29 and has a length of not greater than 21 nucleotides, and the antisense strand comprises the nucleotide sequence set forth in SEQ ID NO. 30 and has a length of not greater than 23 nucleotides; or, the sense strand comprises the nucleotide sequence set forth in SEQ ID NO. 53 and has a length of not greater than 23 nucleotides, and the antisense strand comprises the nucleotide sequence set forth in SEQ ID NO. 54 and has a length of not greater than 25 nucleotides:

sense strand: 5'-AGCAAUAUGACAAUUGAAUGA-3' (SEQ ID NO. 9),

antisense strand: 5'-UCAUUCAAUUGUCAUAUUGCUAC-3' (SEQ ID NO. 10);

sense strand: 5'-AUUUACUGUCACGGUUCCCAA-3' (SEQ ID NO. 11),

antisense strand: 5'-UUGGGAACCGUGACAGUAAUAGC-3' (SEQ ID NO. 12);

sense strand: 5'-CUAUUUAUUUUGAGUCUGU-3' (SEQ ID NO. 13),

antisense strand: 5'-ACAGACUCAAAAUAAAUAGGA-3' (SEQ ID NO. 14);

sense strand: 5'-UGAAGAUAUAUUGUAGUAGAU-3' (SEQ ID NO. 15),

antisense strand: 5'-AUCUACUACAAUAUAUCUUCAAA-3' (SEQ ID NO. 16);

sense strand: 5'-UUGUCUCAUGUUUCAUCGUAA-3' (SEQ ID NO. 17),

antisense strand: 5'-UUACGAUGAAACAUGAGACAAAA-3' (SEQ ID NO. 18);

sense strand: 5'-CUGUGCAGUAUCUGUUCCAUU-3' (SEQ ID NO. 19),

antisense strand: 5'-AAUGGAACAGAUACUGCACAGAC-3' (SEQ ID NO. 20);

sense strand: 5'-AAUGAAAGGACUCACUUGGUA-3' (SEQ ID NO. 21),

antisense strand: 5'-UACCAAGUGAGUCCUUUCAUUUG-3' (SEQ ID NO. 22);

sense strand: 5'-GCUGCAUGAUCAGCUAUGGUA-3' (SEQ ID NO. 23),

antisense strand: 5'-UACCAUAGCUGAUCAUGCAGCGG-3' (SEQ ID NO. 24);

sense strand: 5'-GGUGCUUGGUCUCCUCUAUAA-3' (SEQ ID NO. 25),

antisense strand: 5'-UUAUAGAGGAGACCAAGCACCUU-3' (SEQ ID NO. 26);

sense strand: 5'-AUUGGUCAUCCCAGAACUACA-3' (SEQ ID NO. 27),

antisense strand: 5'-UGUAGUUCUGGGAUGACCAAUUC-3' (SEQ ID NO. 28);

sense strand: 5'-UAUUUAUUUUGAGUCUGUG-3' (SEQ ID NO. 29),

antisense strand: 5'-CACAGACUCAAAAUAAAUAGG-3' (SEQ ID NO. 30);

sense strand: 5'-AUUUACUGUCACGGUUCCCAA-3' (SEQ ID NO. 53),

antisense strand: 5'-UUGGGAACCGUGACAGUAAAUGC-3' (SEQ ID NO. 54);

the sense strand or the antisense strand is optionally modified.

In some embodiments, the present application provides a double-stranded ribonucleic acid (dsRNA), a pharmaceutically acceptable salt thereof, or a ligand conjugate thereof, wherein the dsRNA comprises a sense strand and an antisense strand; the sense strand is the nucleotide sequence set forth in SEQ ID NO. 9, and the antisense strand is the nucleotide sequence set forth in SEQ ID NO. 10; or, the sense strand is the nucleotide sequence set forth in SEQ ID NO. 11, and the antisense strand is the nucleotide sequence set forth in SEQ ID NO. 12; or, the sense strand is the nucleotide sequence set forth in SEQ ID NO. 13, and the antisense strand is the nucleotide sequence set forth in SEQ ID NO. 14; or, the sense strand is the nucleotide sequence set forth in SEQ ID NO. 15, and the antisense strand is the nucleotide sequence set forth in SEQ ID NO. 16; or, the sense strand is the nucleotide sequence set forth in SEQ ID NO. 17, and the antisense strand is the nucleotide sequence set forth in SEQ ID NO. 18; or, the sense strand is the nucleotide sequence set forth in SEQ ID NO. 19, and the antisense strand is the nucleotide sequence set forth in SEQ ID NO. 20; or, the sense strand is the nucleotide sequence set forth in SEQ ID NO. 21, and the antisense strand is the nucleotide sequence set forth in SEQ ID NO. 22; or, the sense strand is the nucleotide sequence set forth in SEQ ID NO. 23, and the antisense strand is the nucleotide sequence set forth in SEQ ID NO. 24; or, the sense strand is the nucleotide sequence set forth in SEQ ID NO. 25, and the antisense strand is the nucleotide sequence set forth in SEQ ID NO. 26; or, the sense strand is the nucleotide sequence set forth in SEQ ID NO. 27, and the antisense strand is the nucleotide sequence set forth in SEQ ID NO. 28; or, the sense strand is the nucleotide sequence set forth in SEQ ID NO. 29, and the antisense strand is the nucleotide sequence set forth in SEQ ID NO. 30; or, the sense strand comprises the nucleotide sequence set forth in SEQ ID NO. 53, and the antisense strand comprises the nucleotide sequence set forth in SEQ ID NO. 54:

sense strand: 5'-AGCAAUAUGACAAUUGAAUGA-3' (SEQ ID NO. 9),

antisense strand: 5'-UCAUUCAAUUGUCAUAUUGCUAC-3' (SEQ ID NO. 10);

sense strand: 5'-AUUUACUGUCACGGUUCCCAA-3' (SEQ ID NO. 11),

antisense strand: 5'-UUGGGAACCGUGACAGUAAUAGC-3' (SEQ ID NO. 12);

sense strand: 5'-CUAUUUAUUUUGAGUCUGU-3' (SEQ ID NO. 13),

antisense strand: 5'-ACAGACUCAAAAUAAAUAGGA-3' (SEQ ID NO. 14);

sense strand: 5'-UGAAGAUAUAUUGUAGUAGAU-3' (SEQ ID NO. 15),

antisense strand: 5'-AUCUACUACAAUAUAUCUUCAAA-3' (SEQ ID NO. 16);

sense strand: 5'-UUGUCUCAUGUUUCAUCGUAA-3' (SEQ ID NO. 17),

antisense strand: 5'-UUACGAUGAAACAUGAGACAAAA-3' (SEQ ID NO. 18);

sense strand: 5'-CUGUGCAGUAUCUGUUCCAUU-3' (SEQ ID NO. 19),

antisense strand: 5'-AAUGGAACAGAUACUGCACAGAC-3' (SEQ ID NO. 20);

sense strand: 5'-AAUGAAAGGACUCACUUGGUA-3' (SEQ ID NO. 21),

antisense strand: 5'-UACCAAGUGAGUCCUUUCAUUUG-3' (SEQ ID NO. 22);

sense strand: 5'-GCUGCAUGAUCAGCUAUGGUA-3' (SEQ ID NO. 23),

antisense strand: 5'-UACCAUAGCUGAUCAUGCAGCGG-3' (SEQ ID NO. 24);

sense strand: 5'-GGUGCUUGGUCUCCUCUAUAA-3' (SEQ ID NO. 25),

antisense strand: 5'-UUAUAGAGGAGACCAAGCACCUU-3' (SEQ ID NO. 26);

sense strand: 5'-AUUGGUCAUCCCAGAACUACA-3' (SEQ ID NO. 27),

antisense strand: 5'-UGUAGUUCUGGGAUGACCAAUUC-3' (SEQ ID NO. 28);

sense strand: 5'-UAUUUAUUUUGAGUCUGUG-3' (SEQ ID NO. 29),

antisense strand: 5'-CACAGACUCAAAAUAAAUAGG-3' (SEQ ID NO. 30);

sense strand: 5'-AUUUACUGUCACGGUUCCCAA-3' (SEQ ID NO. 53),

antisense strand: 5'-UUGGGAACCGUGACAGUAAAUGC-3' (SEQ ID NO. 54);

the sense strand or the antisense strand is optionally modified.

The present application further provides a dsRNA, a pharmaceutically acceptable salt thereof, or a ligand conjugate thereof, wherein the dsRNA comprises a sense strand and an antisense strand; the sense strand and the antisense strand have at least 90%, 91%, 92%, 93%, 94%, 95%, 96%, 97%, 98%, or 99% sequence identity to the full length of the nucleotide sequences of the sense strands and the antisense strands described above, respectively.

In some embodiments, the dsRNA, the pharmaceutically acceptable salt thereof, or the ligand conjugate thereof of the present application is an RNAi drug.

In some embodiments, the dsRNA, the pharmaceutically acceptable salt thereof, or the ligand conjugate thereof of the present application is an siRNA or a ligand conjugate thereof.

In a specific embodiment, the dsRNA, the pharmaceutically acceptable salt thereof, or the ligand conjugate thereof has a double-stranded region formed by the complementation of the sense strand and the antisense strand, and optionally an overhang at the 5' and/or 3' end of the sense strand and/or the antisense strand. In a preferred embodiment, the dsRNA, the pharmaceutically acceptable salt thereof, or the ligand conjugate thereof has a double-stranded region formed by the complementation of the sense strand and the antisense strand, and an overhang at the 5' and/or 3' end of the sense strand and/or the antisense strand. In a preferred embodiment, the dsRNA, the pharmaceutically acceptable salt thereof, or the ligand conjugate thereof has a double-stranded region formed by the complementation of the sense strand and the antisense strand, and an overhang at the 3' end of the antisense strand. In a preferred embodiment, the dsRNA, the pharmaceutically acceptable salt thereof, or the ligand conjugate thereof has a double-stranded region formed by the complementation of the sense strand and the antisense strand, and an overhang at the 3' end of the sense strand.

## Double-Stranded Region

In some embodiments, the double-stranded region of the dsRNA has a length of 19-23 nucleotide pairs. For example, the double-stranded region of the dsRNA has a length of 19, 20, 21, 22, or 23 nucleotide pairs.

## Overhang

In some embodiments, the sense strand or the antisense strand optionally comprises an overhang at the 5' end and/or 3' end.

In some embodiments, the overhang comprises 1, 2, 3, 4, or 5 nucleotides. In some embodiments, the overhang comprises 1 or 2 nucleotides.

In some embodiments, the sense strand optionally comprises an overhang at the 5' end and/or 3' end. In some embodiments, the sense strand optionally comprises an overhang of 1, 2, 3, 4, or 5 nucleotides at the 5' end and/or 3' end. In some embodiments, the sense strand optionally comprises an overhang of 1 or 2 nucleotides at the 5' end and/or 3' end.

In some embodiments, the antisense strand optionally comprises an overhang at the 5' end and/or 3' end. In some embodiments, the antisense strand optionally comprises an overhang of 1, 2, 3, 4, or 5 nucleotides at the 5' end and/or 3' end. In some embodiments, the antisense strand optionally comprises an overhang of 1 or 2 nucleotides at the 5' end and/or 3' end.

In some embodiments, the overhang is selected from the group consisting of unmodified or modified A, G, C, U, and T.

In a specific embodiment, the overhang may be the 1, 2, 3, 4, or 5 nucleotides, preferably the 1 or 2 nucleotides, at the 5' end and/or 3' end of the sense strand or the antisense strand. In a specific embodiment, the dsRNA, the pharmaceutically acceptable salt thereof, or the ligand conjugate thereof comprises an additional sequence as the overhang, and the additional sequence may comprise 1, 2, 3, 4, or 5 nucleotides, preferably 1 or 2 nucleotides, linked to the 5' end and/or 3' end of the sense strand or the antisense strand.

For example, in the case where the ribonucleotides at the 5' end and/or 3' end of the sense strand or the antisense strand serve as the overhang: when the sense strand has 21 nucleotides, the antisense strand has 23 nucleotides, and positions 1-21 of the sense strand are complementary to positions 1-21 of the antisense strand, the nucleotides at positions 22 and 23 of the antisense strand constitute the overhang located at the 3' end of the antisense strand, that is, the 3' end of the sense strand is a blunt end; when the sense strand has 21 nucleotides, the antisense strand has 21 nucleotides, and positions 1-20 of the sense strand are complementary to positions 1-20 of the antisense strand, the nucleotides at positions 21 of the sense strand and the antisense strand constitute the overhangs at the 3' end of the sense strand and the antisense strand; when the sense strand has 21 nucleotides, the antisense strand has 23 nucleotides, and positions 1-21 of the sense strand are complementary to positions 3-23 of the antisense strand, the nucleotides at positions 1 and 2 of the antisense strand constitute the overhang located at the 5' end of the antisense strand, that is, the 5' end of the sense strand is a blunt end.

In some embodiments, in the case where the nucleotides at the 5' end and/or 3' end of the sense strand or the antisense strand serve as the overhang, the antisense strand optionally comprises an overhang at the 5' end and/or 3' end, and the overhang is selected from the group consisting of unmodified or modified ribonucleotide sequences GA, CA, AC, GC, AA, UG, GG, UU, and UC. In some embodiments, the antisense strand optionally comprises an overhang at the 3' end, and the overhang is selected from the group consisting of unmodified or modified ribonucleotide sequences GA, CA, AC, GC, AA, UG, GG, UU, and UC.

In a specific embodiment, the dsRNA, the pharmaceutically acceptable salt thereof, or the ligand conjugate thereof comprises an additional sequence at the 5' end and/or 3' end of the antisense strand or the sense strand as the overhang, and the additional sequence may comprise 1, 2, 3, 4, or 5 nucleosides, preferably 1 or 2 nucleotides, at the 5' end and/or the 3' end.

In some embodiments, the overhang is an additional sequence optionally located at the 5' end and/or 3' end of the antisense strand, and the overhang is selected from the group consisting of unmodified or modified GA, CA, AC, GC, AA, UG, GG, UU, and UC. In some embodiments, the overhang is an additional sequence located at the 3' end of the antisense strand, and the overhang is selected from the group consisting of unmodified or modified GA, CA, AC, GC, AA, UG, GG, UU, and UC.

In some embodiments, the additional sequence as the overhang comprises unmodified or modified U or T.

In some embodiments, when the additional sequence as the overhang has 1 nucleotide, the overhang is selected from the group consisting of unmodified or modified U and T. In some embodiments, when the additional sequence as the overhang has 1 nucleotide, the overhang is selected from the group consisting of 2'-O-methyl-modified U (hereinafter referred to as u) and deoxythymine nucleotide (hereinafter referred to as dT).

In some embodiments, when the additional sequence as the overhang has 2 nucleotides, the overhang is selected from the group consisting of unmodified or modified UU and TT. In some embodiments, when the additional sequence as the overhang has 2 nucleotides, the overhang is selected from the group consisting of uu and dTdT.

In some embodiments, the overhang is an additional sequence optionally located at the 5' end and/or 3' end of the sense strand, and the overhang is selected from the group consisting of uu and dTdT.

In some embodiments, the overhang is an additional sequence optionally located at the 5' end and/or 3' end of the antisense strand, and the overhang is selected from the group consisting of uu and dTdT.

In some embodiments, the overhang is linked to an adjacent nucleotide via a phosphate group or a phosphorothioate group.

In some embodiments, one or more nucleotides in the overhang are linked via a phosphate group or a phosphorothioate group.

**Modification**

In some embodiments, the sense strand or the antisense strand is optionally modified.
In some embodiments, one or more nucleotides of the sense strand or the antisense strand are modified.
In some embodiments, each nucleotide may be modified by the same or different modifications. The modification may include: one or more changes in one or two of non-linked phosphate oxygens and/or one or more of linked phosphate oxygens; changes in components of ribose (e.g., 2' hydroxyl in ribose); total replacement of the phosphate moiety with a "dephosphorylation" linker; modification of naturally occurring bases; and modification of the ribose-phosphate backbone.
In some embodiments, before each nucleotide modification, the base is selected from the group consisting of naturally occurring bases, e.g., A, U, C, G, and T, and the modification occurring on a nucleotide base refers to a chemical modification on the basis of the backbone of A, U, C, G, and T.
In some embodiments, 1, 2, 3, 4, 5, 6, 7, 8, 9, 10, 11, 12, 13, 14, 15, 16, 17, 18, 19, 20, 21, 22, or 23 nucleotides of the sense strand are modified, or a number of nucleotides within a range formed by any of the foregoing values, e.g., 1-23, 1-22, 1-21, 1-20, 1-19, 1-18, 1-17, 1-16, 1-15, 1-14, 1-13, 1-12, 1-11, or 1-10 nucleotides, are modified.
In some embodiments, 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, or 23 or more nucleotides of the sense strand are modified; typically, 10 or more, 11 or more, 12 or more, 13 or more, 14 or more, 15 or more, 16 or more, 17 or more, 18 or more, 19 or more, 20 or more, or 21 or more nucleotides of the sense strand are modified.
In some embodiments, all of the nucleotides in the sense strand are modified.
In some embodiments, 1, 2, 3, 4, 5, 6, 7, 8, 9, 10, 11, 12, 13, 14, 15, 16, 17, 18, 19, 20, 21, 22, 23, 24, or 25 nucleotides of the antisense strand are modified, or a number of nucleotides within a range formed by any of the foregoing values, e.g., 1-25, 1-24, 1-23, 1-22, 1-21, 1-20, 1-19, 1-18, 1-17, 1-16, 1-15, 1-14, 1-13, 1-12, 1-11, or 1-10 nucleotides, are modified.
In some embodiments, 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides of the antisense strand are modified; typically, 10 or more, 11 or more, 12 or more, 13 or more, 14 or more, 15 or more, 16 or more, 17 or more, 18 or more, 19 or more, 20 or more, 21 or more, 22 or more, or 23 or more nucleotides of the antisense strand are modified.
In some embodiments, all of the nucleotides in the antisense strand are modified.
In some embodiments, all of the nucleotides in the sense strand and all of the nucleotides in the antisense strand are modified.
In some embodiments, the modification is selected from the group consisting of glycosyl modification of nucleotides, modification of bases, modification of linkages between nucleotides, and terminal modification. In some embodiments, the modification is selected from the group consisting of glycosyl modification of nucleotides, modification of linkages between nucleotides, and terminal modification.
In some embodiments, the glycosyl modification of the nucleotide is selected from the group consisting of dehydroxylation, fluorination, amination, alkylation, hydroxyalkylation, and hydroxyalkenylation.
In some embodiments, the glycosyl modification of the nucleotide occurs at the 2' position of the glycosyl.
In some embodiments, the glycosyl modification is selected from the group consisting of 2'-dehydroxylation, 2'-fluoro, 2'-amino, 2'-alkyl, 2'-O-alkyl, 2'-O-ether, and 2'-O-alkenyl. In some embodiments, the glycosyl modification is selected from the group consisting of 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-ethyl-O-methyl, and 2'-O-allyl.
In some embodiments, the modification of linkages between nucleotides is selected from the group consisting of phosphorothioate (PS), phosphorodithioate (PS2), methylphosphonate (MP), methoxypropylphosphonate (MOP), and aminophosphonate. In some embodiments, the modification of linkages between nucleotides is selected from the group consisting of phosphorothioate (PS). In some embodiments, the nucleotides are linked via phosphorothioate diester bonds.
In some embodiments, the terminal modification is selected from the group consisting of a modification on phosphate group or hydroxyl group at the terminus. In some embodiments, the terminal modification is selected from the group consisting of a 5'-end modification and a 3'-end modification. In some embodiments, the terminal modification is selected from the group consisting of 5'-phosphate, 5'-methylphosphonate (5'-MP), 5'-phosphorothioate (5'-PS), and 5'-(*E*)-vinylphosphonate (5'-(E)-VP) on the ribose group of the nucleotide at the terminus. In some embodiments, the terminal modification is selected from the group consisting of 5'-phosphate and 5'-(*E*)-vinylphosphate on the ribose group of the nucleotide at the terminus.
In some embodiments, the modification is selected from the group consisting of 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-allyl, phosphorothioate, methylphosphonate, phosphoramidate, 5'-phosphate, 3'-phosphate, 5'-(*E*)-vinylphosphate, and 3'-(*E*)-vinylphosphate.

In some embodiments, the modification is selected from the group consisting of 2'-dehydroxylation, 2'-fluoro, 2'-O-methyl, 2'-O-ethyl, 2'-O-ethyl-O-methyl, 2'-O-allyl, phosphorothioate, and 5'-(*E*)-vinylphosphate.

In some embodiments, the modification is selected from the group consisting of 2'-fluoro, 2'-O-methyl, phosphorothioate, and 5'-(*E*)-vinylphosphate.

As will be described in detail below, when referring to the nucleotide at position n of the sense strand and the antisense strand, the counting starts from the 5' end unless otherwise specified.

In some embodiments, a phosphorothioate modification is present between the first two nucleotides and/or between the first three nucleotides at one or both of the two ends of the two nucleic acid strands of the dsRNA, the pharmaceutically acceptable salt thereof, or the ligand conjugate thereof. For example, for the sense strand, the phosphorothioate modification is present between the nucleotides at positions 1 and 2 of the sense strand, and/or between the nucleotides at positions 2 and 3 of the sense strand, and/or between the nucleotides at positions 1 and 2 and the nucleotides at positions 2 and 3 of the sense strand.

In some embodiments, in the absence of the additional sequence as the overhang, the phosphorothioate modification is present between the nucleotides at positions 1 and 2 of the sense strand and/or between the nucleotides at positions 2 and 3 of the sense strand.

In some embodiments, in the absence of the additional sequence as the overhang, the phosphorothioate modification is present between the nucleotides at positions 1 and 2 of the antisense strand, between the nucleotides at positions 2 and 3 of the antisense strand, between the nucleotides at positions 1 and 2 of the 3' end of the antisense strand, and/or between the nucleotides at positions 2 and 3 of the 3' end of the antisense strand.

In some embodiments, in the presence of the additional sequence as the overhang, the phosphorothioate modification may be located between nucleotides in the additional sequence.

In some embodiments, the nucleotide at the 5'-end of the antisense strand is modified by 5'-(*E*)-vinylphosphate.

In some embodiments, 2, 3, 4, or 5 nucleotides of the sense strand are modified by 2'-fluoro. In some embodiments, 14, 15, 16, 17, 18, or 19 nucleotides of the sense strand are modified by 2'-O-methyl. In some embodiments, in the sense strand, 3 or 4 nucleotides are modified by 2'-fluoro, and 15, 16, 17, or 18 nucleotides are modified by 2'-O-methyl.

In some embodiments, 0, 1, or 2 of the nucleotides at positions 1 to 6 or positions 1 to 8 of the sense strand are modified by 2'-fluoro.

In some embodiments, 4, 5, 6, 7, or 8 of the nucleotides at positions 1 to 6 or positions 1 to 8 of the sense strand are modified by 2'-O-methyl.

In some embodiments, 2 or 3 of the nucleotides at positions 7 to 9 or positions 9 to 11 of the sense strand are modified by 2'-fluoro.

In some embodiments, 0 or 1 of the nucleotides at positions 7 to 9 or positions 9 to 11 of the sense strand is modified by 2'-O-methyl.

In some embodiments, 0 or 1 of the nucleotides at positions 10 to 19 or positions 12 to 21 of the sense strand is modified by 2'-fluoro.

In some embodiments, 8, 9, 10, 11, or 12 of the nucleotides at positions 10 to 19 or positions 12 to 21 of the sense strand are modified by 2'-O-methyl.

In some embodiments, the nucleotides at positions 7, 8, and 9 of the sense strand are modified by 2'-fluoro, and 0, 1, or 2 of the nucleotides at positions 1 to 6 and/or 10 to 19 of the sense strand are also modified by 2'-fluoro. In some embodiments, the nucleotides at positions 7, 8, and 9 of the sense strand are modified by 2'-fluoro, and 0, 1, or 2 of the nucleotides at positions 1 to 6 and 10 to 19 of the sense strand are also modified by 2'-fluoro. In some embodiments, the nucleotides at positions 7, 8, and 9 of the sense strand are modified by 2'-fluoro, and 0 of nucleotides at positions 1 to 6 and 10 to 19 of the sense strand are modified by 2'-fluoro.

In some embodiments, the nucleotides at positions 9, 10, and 11 of the sense strand are modified by 2'-fluoro, and 0, 1, or 2 of the nucleotides at positions 1 to 8 and/or 12 to 21 of the sense strand are also modified by 2'-fluoro. In some embodiments, the nucleotides at positions 9, 10, and 11 of the sense strand are modified by 2'-fluoro, and 0, 1, or 2 of the nucleotides at positions 1 to 8 and 12 to 21 of the sense strand are also modified by 2'-fluoro. In some embodiments, the nucleotides at positions 9, 10, and 11 of the sense strand are modified by 2'-fluoro, and 0 of the nucleotides at positions 1 to 8 and 12 to 21 of the sense strand are modified by 2'-fluoro.

In some embodiments, 2, 3, 4, 5, 6, or 7 nucleotides of the antisense strand are modified by 2'-fluoro.

In some embodiments, 14, 15, 16, 17, 18, 19, 20, or 21 nucleotides of the antisense strand are modified by 2'-O-methyl.

In some embodiments, 3 or 4 of the nucleotides at positions 2, 6, 14, and 16 of the antisense strand are modified by 2'-fluoro. In some embodiments, 4 of the nucleotides at positions 2, 6, 14, and 16 of the antisense strand are modified by 2'-fluoro.

In some embodiments, 3, 4, 5, or 6 of the nucleotides at positions 2, 6, 8, 9, 14, and 16 of the antisense strand are modified by 2'-fluoro. In some embodiments, 3 or 4 of the nucleotides at positions 2, 6, 14, and 16 of the antisense strand

are modified by 2'-fluoro. In some embodiments, 4 or 6 of the nucleotides at positions 2, 6, 8, 9, 14, and 16 of the antisense strand are modified by 2'-fluoro.

In some embodiments, the nucleotides at positions 2, 6, 14, and 16 of the antisense strand are modified by 2'-fluoro, and any one or two of the nucleotides at positions 1, 3 to 5, 7 to 13, 15, 17 to 21, and/or 17 to 23 of the antisense strand are also modified by 2'-fluoro. In some embodiments, the nucleotides at positions 2, 6, 14, and 16 of the antisense strand are modified by 2'-fluoro, and any one or two of the nucleotides at positions 1, 3 to 5, 7 to 13, 15, 17 to 21, and/or 17 to 23 of the antisense strand are also modified by 2'-fluoro. In some embodiments, the nucleotides at positions 2, 6, 14, and 16 of the antisense strand are modified by 2'-fluoro, and 0 of the nucleotides at positions 1, 3 to 5, 7 to 13, 15, 17 to 21, and/or 17 to 23 of the antisense strand are modified by 2'-fluoro.

In some embodiments, 14, 15, 16, 17, 18, or 19 of the nucleotides at positions 1, 3 to 5, 7 to 13, 15, 17 to 21, and/or 17 to 23 of the antisense strand are modified by 2'-O-methyl.

In some embodiments, the nucleotides at positions 2, 6, 14, and 16 of the antisense strand are modified by 2'-fluoro, 0 of the nucleotides at positions 1, 3 to 5, 7 to 13, 15, 17 to 21, and/or 17 to 23 of the antisense strand are modified by 2'-fluoro, and 14, 15, 16, 17, 18, or 19 of the nucleotides at positions 1, 3 to 5, 7 to 13, 15, 17 to 21, and/or 17 to 23 of the antisense strand are modified by 2'-O-methyl.

In some embodiments, 2, 3, 4, or 5 nucleotides of the sense strand are modified by 2'-fluoro, and 14, 15, 16, 17, 18, or 19 nucleotides of the sense strand are modified by 2'-O-methyl; and/or

2, 3, 4, 5, 6, or 7 nucleotides of the antisense strand are modified by 2'-fluoro, and 14, 15, 16, 17, 18, 19, 20, or 21 nucleotides of the antisense strand are modified by 2'-O-methyl.

In one specific embodiment, the nucleotides at positions 7, 8, and 9 or positions 9, 10, and 11 of the sense strand are modified by 2'-fluoro, and the other nucleotides of the sense strand are modified by 2'-O-methyl; and the nucleotides at positions 2, 6, 14, and 16 of the antisense strand are modified by 2'-fluoro, and the other nucleotides of the antisense strand are modified by 2'-O-methyl.

In one specific embodiment, the nucleotides at positions 7, 8, and 9 or positions 9, 10, and 11 of the sense strand are modified by 2'-fluoro, and the other nucleotides of the sense strand are modified by 2'-O-methyl; and the nucleotides at positions 2, 6, 14, and 16 of the antisense strand are modified by 2'-fluoro, and the other nucleotides of the antisense strand are modified by 2'-O-methyl; and a phosphorothioate modification is present between the nucleotides at positions 1 and 2 of the sense strand, between the nucleotides at positions 2 and 3 of the sense strand, between the nucleotides at positions 1 and 2 of the antisense strand, between the nucleotides at positions 2 and 3 of the antisense strand, between the nucleotides at positions 1 and 2 of the 3' end of the antisense strand, and/or between the nucleotides at positions 3 and 4 of the antisense strand. Optionally, the nucleotide at the 5'-end of the antisense strand is modified by 5'-(*E*)-vinylphosphate.

## Ligand

In some embodiments, the ligand comprises a branched group and a linker. In some embodiments, the branched group may comprise a targeting group.

In some embodiments, the ligand is linked to the dsRNA via the linker. Herein, a dsRNA to which a ligand is linked is referred to as a dsRNA ligand conjugate.

In some embodiments, the ligand is linked to one or more targeting groups via the branched group. In some embodiments, the branched group comprises the targeting group.

In some embodiments, the ligand comprises at least one targeting group. In some embodiments, the ligand comprises one, two, three, four, or five targeting groups. In some embodiments, the ligand comprises two, three, or four targeting groups. In some embodiments, the ligand comprises three targeting groups.

In some embodiments, the targeting group is selected from the group consisting of a GalNAc group.

In some embodiments, the ligand comprises one, two, three, four, or five GalNAc groups. In some embodiments, the ligand comprises two, three, or four GalNAc groups. In some embodiments, the ligand comprises three GalNAc groups.

In some embodiments, the branched group is selected from the group consisting of:

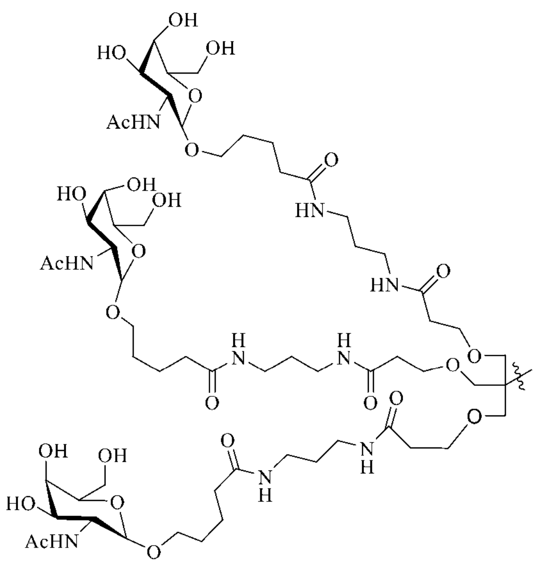

and

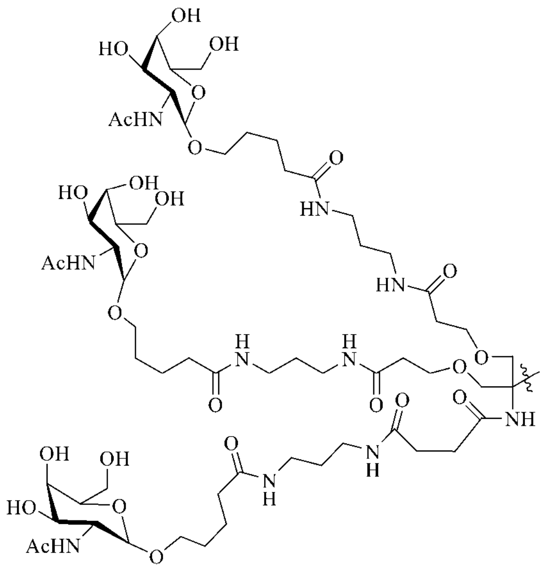

.

In some embodiments, the linker is selected from the group consisting of:

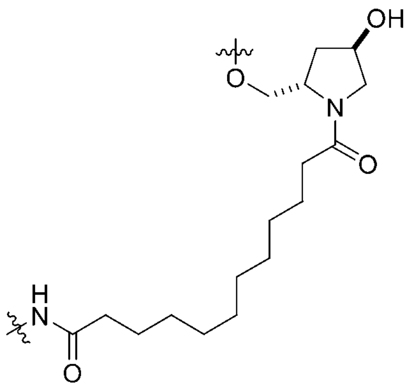

and

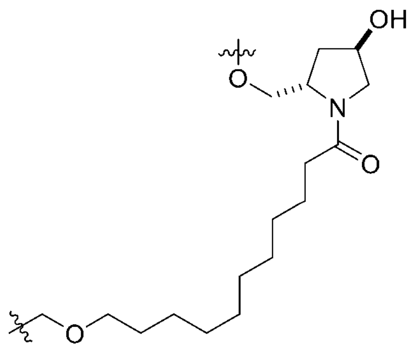

.

In some embodiments, the ligand is selected from the group consisting of:

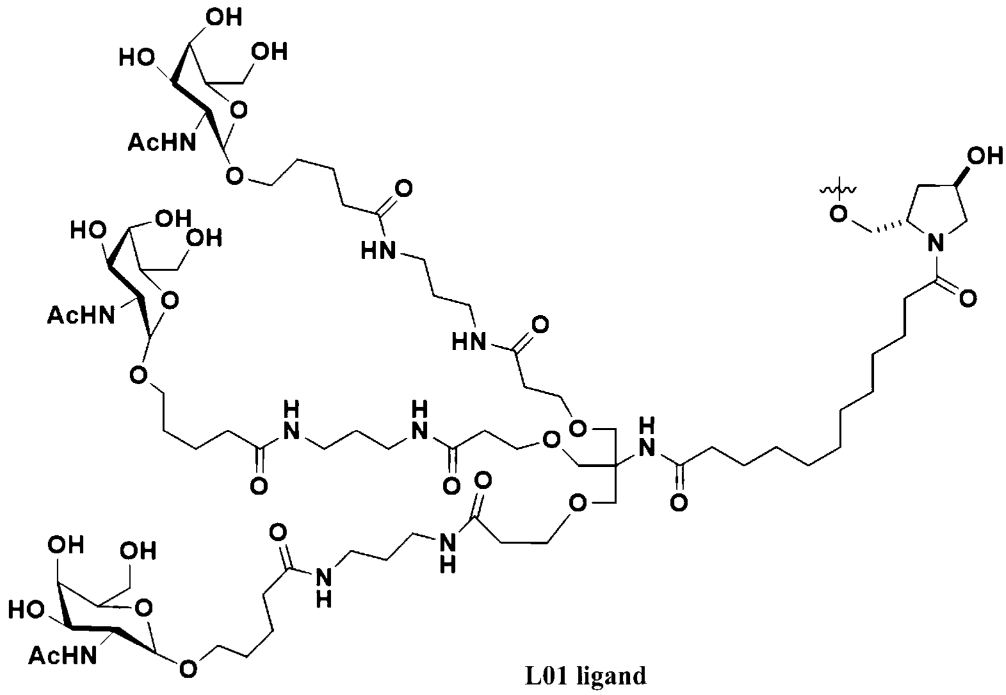

L01 ligand

and

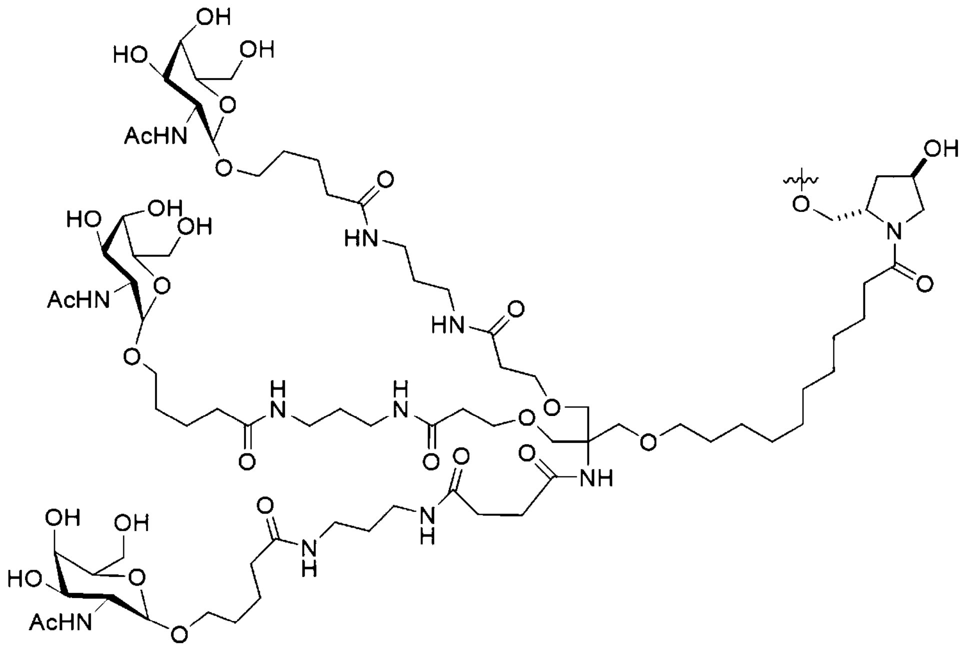

L02 ligand

In some embodiments, the ligand is linked to the sense strand or the antisense strand. In some embodiments, the ligand is linked to the 5' or 3' end of the sense strand or the antisense strand. In some embodiments, the ligand is linked to the 5' or 3' end of the sense strand. In some embodiments, the ligand is linked to the 3' end of the sense strand.

In some embodiments, the ligand is linked to the sense strand or the antisense strand of the dsRNA via a phosphate group or a phosphorothioate group. In some embodiments, the ligand is linked to the sense strand via a phosphate group or a phosphorothioate group. In some embodiments, the ligand is linked to the 3' end of the sense strand via a phosphate group or a phosphorothioate group.

In some embodiments, the dsRNA ligand conjugate is as follows:

3' end of sense strand

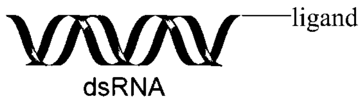


formula 101

In some embodiments, the dsRNA ligand conjugate is selected from the group consisting of:

3' end of sense strand X is O⁻, S⁻, OH, or SH

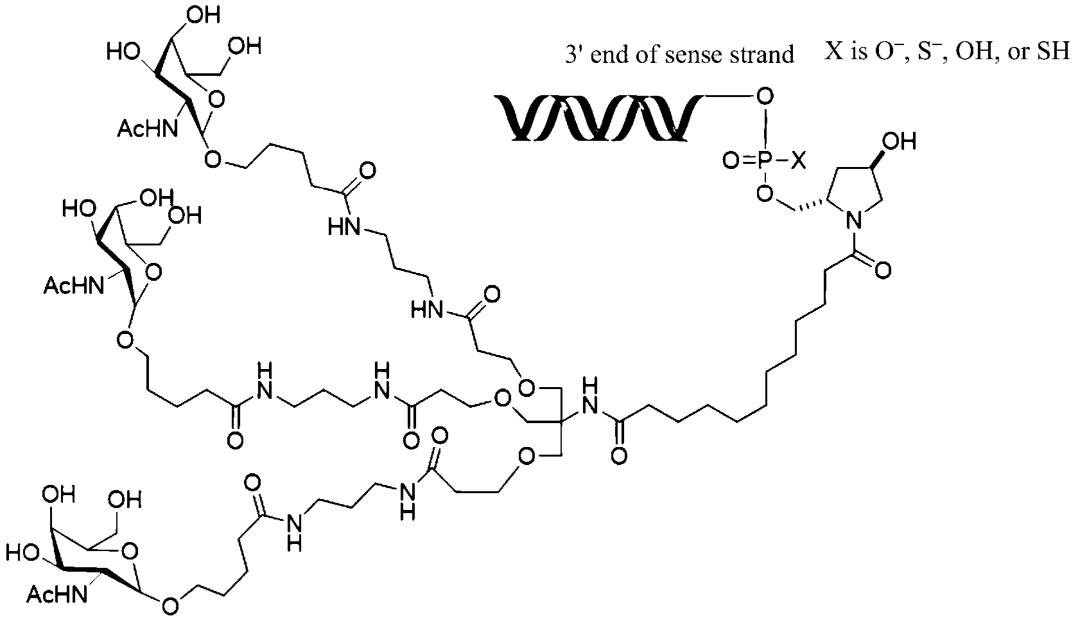

formula 102

, and

3' end of sense strand X is O⁻, S⁻, OH, or SH

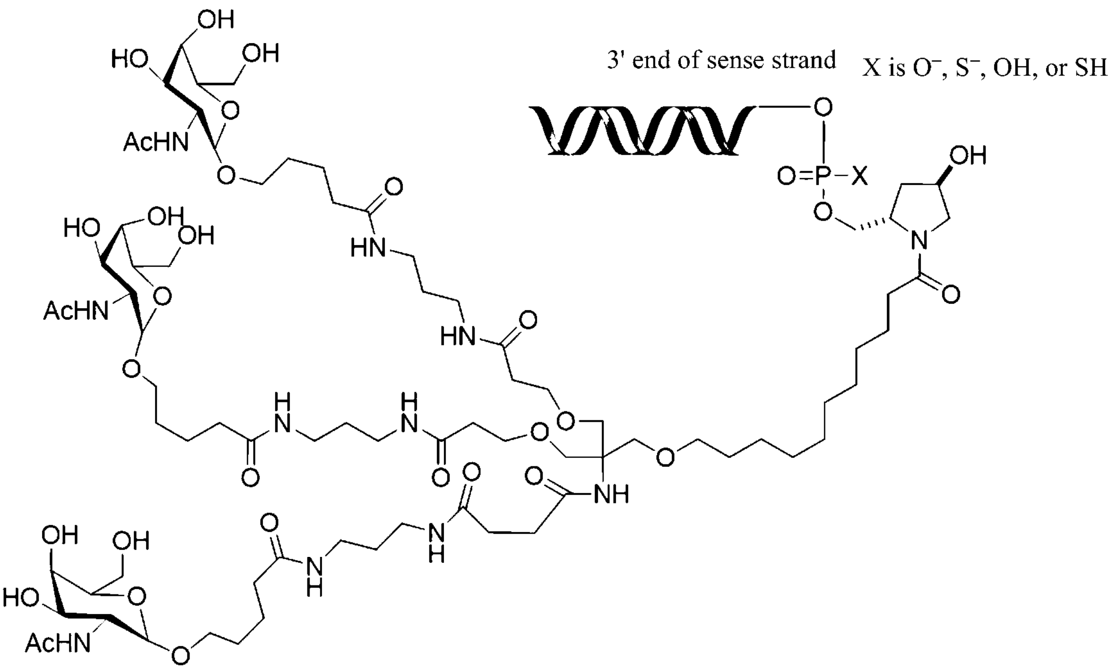

formula 103

.

In some embodiments, the dsRNA ligand conjugate is selected from the group consisting of:

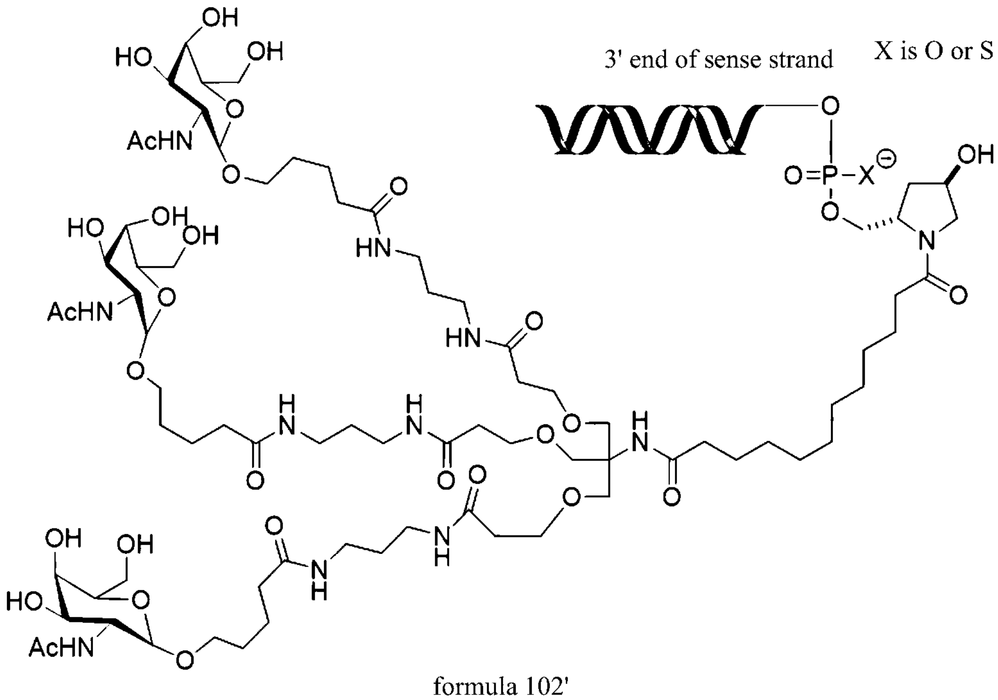


formula 102'

,

and

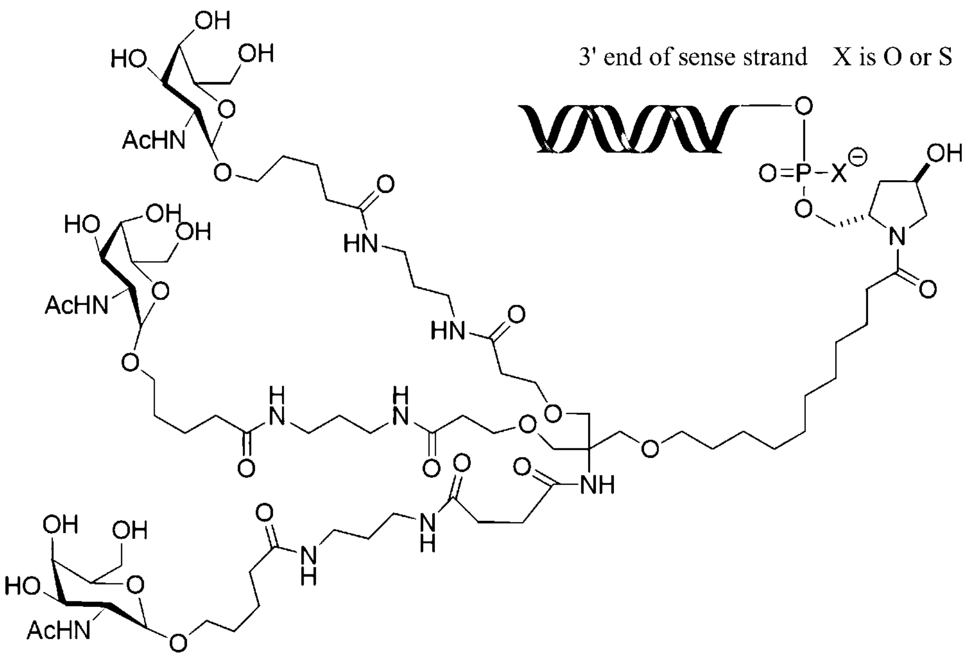


formula 103'

In some embodiments, the unmodified sense strand of the dsRNA is selected from the group consisting of:

Table 1. Unmodified sense strands

| SEQ ID NO | Sequence in the 5' to 3' direction |
|---|---|
| 9 | AGCAAUAUGACAAUUGAAUGA |
| 11 | AUUUACUGUCACGGUUCCCAA |
| 13 | CUAUUUAUUUUGAGUCUGU |

(continued)

| SEQ ID NO | Sequence in the 5' to 3' direction |
|---|---|
| 15 | UGAAGAUAUAUUGUAGUAGAU |
| 17 | UUGUCUCAUGUUUCAUCGUAA |
| 19 | CUGUGCAGUAUCUGUUCCAUU |
| 21 | AAUGAAAGGACUCACUUGGUA |
| 23 | GCUGCAUGAUCAGCUAUGGUA |
| 25 | GGUGCUUGGUCUCCUCUAUAA |
| 27 | AUUGGUCAUCCCAGAACUACA |
| 29 | UAUUUAUUUUGAGUCUGUG |
| 53 | AUUUACUGUCACGGUUCCCAA |

In some embodiments, the unmodified antisense strand of the dsRNA is selected from the group consisting of:

Table 2. Unmodified antisense strands

| SEQ ID NO | Sequence in the 5' to 3' direction |
|---|---|
| 10 | UCAUUCAAUUGUCAUAUUGCUAC |
| 12 | UUGGGAACCGUGACAGUAAUAGC |
| 14 | ACAGACUCAAAAUAAAUAGGA |
| 16 | AUCUACUACAAUAUAUCUUCAAA |
| 18 | UUACGAUGAAACAUGAGACAAAA |
| 20 | AAUGGAACAGAUACUGCACAGAC |
| 22 | UACCAAGUGAGUCCUUUCAUUUG |
| 24 | UACCAUAGCUGAUCAUGCAGCGG |
| 26 | UUAUAGAGGAGACCAAGCACCUU |
| 28 | UGUAGUUCUGGGAUGACCAAUUC |
| 30 | CACAGACUCAAAAUAAAUAGG |
| 54 | UUGGGAACCGUGACAGUAAAUGC |

In some embodiments, the modified sense strand of the dsRNA is selected from the group consisting of:

Table 3. Modified sense strands

| SEQ ID NO | Sequence in the 5' to 3' direction |
|---|---|
| 31 | asgscaauauGfAfCfaauugaauga |
| 33 | asusuuacugUfCfAfcgguucccaa |
| 35 | csusauuuAfUfUfuugagucugu |
| 37 | usgsaagauaUfAfUfuguaguagau |
| 39 | ususgucucaUfGfUfuucaucguaa |
| 41 | csusgugcagUfAfUfcuguuccauu |
| 43 | asasugaaagGfAfCfucacuuggua |
| 45 | gscsugcaugAfUfCfagcuauggua |
| 47 | gsgsugcuugGfUfCfuccucuauaa |
| 49 | asusuggucaUfCfCfcagaacuaca |

(continued)

| SEQ ID NO | Sequence in the 5' to 3' direction |
|---|---|
| 51 | usasuuuaUfUfUfugagucugug |
| 55 | asusuuacugUfCfAfcgguucccaa |

In some embodiments, the modified antisense strand of the dsRNA is selected from the group consisting of:

Table 4. Modified antisense strands

| SEQ ID NO | Sequence in the 5' to 3' direction |
|---|---|
| 32 | usCfsauuCfaauugucAfuAfuugcusasc |
| 34 | usUfsgggAfaccgugaCfaGfuaauasgsc |
| 36 | asCfsagaCfucaaaauAfaAfuagsgsa |
| 38 | asUfscuaCfuacaauaUfaUfcuucasasa |
| 40 | usUfsacgAfugaaacaUfgAfgacaasasa |
| 42 | asAfsuggAfacagauaCfuGfcacagsasc |
| 44 | usAfsccaAfgugagucCfuUfucauususg |
| 46 | usAfsccaUfagcugauCfaUfgcagcsgsg |
| 48 | usUfsauaGfaggagacCfaAfgcaccsusu |
| 50 | usGfsuagUfucugggaUfgAfccaaususc |
| 52 | csAfscagAfcucaaaaUfaAfauasgsg |
| 56 | usUfsgggAfaccgugaCfaGfuaaausgsc |

The dsRNA of the present application may be formed by comprising any one of the sense strands described above and any one of the antisense strands described above.

For example, the dsRNA comprises any one of the following sense strands: SEQ ID NO. 9, SEQ ID NO. 11, SEQ ID NO. 13, SEQ ID NO. 15, SEQ ID NO. 17, SEQ ID NO. 19, SEQ ID NO. 21, SEQ ID NO. 23, SEQ ID NO. 25, SEQ ID NO. 27, SEQ ID NO. 29, SEQ ID NO. 53, SEQ ID NO. 31, SEQ ID NO. 33, SEQ ID NO. 35, SEQ ID NO. 37, SEQ ID NO. 39, SEQ ID NO. 41, SEQ ID NO. 43, SEQ ID NO. 45, SEQ ID NO. 47, SEQ ID NO. 49, SEQ ID NO. 51, or SEQ ID NO. 55; and the dsRNA comprises any one of the following antisense strands: SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, SEQ ID NO. 28, SEQ ID NO. 30, SEQ ID NO. 54, SEQ ID NO. 32, SEQ ID NO. 34, SEQ ID NO. 36, SEQ ID NO. 38, SEQ ID NO. 40, SEQ ID NO. 42, SEQ ID NO. 44, SEQ ID NO. 46, SEQ ID NO. 48, SEQ ID NO. 50, SEQ ID NO. 52, or SEQ ID NO. 56.

In some embodiments, the dsRNA (or siRNA, unmodified) is selected from the group consisting of:

Table 5. siRNAs (unmodified)

| dsRNA No. | Sense strand SEQ ID NO | Sequence in the 5' to 3' direction | Antisense strand SEQ ID NO | Sequence in the 5' to 3' direction |
|---|---|---|---|---|
| AD01 | 9 | AGCAAUAUGACAAUUGAAUGA | 10 | UCAUUCAAUUGUCAUAUUGCUAC |
| AD02 | 11 | AUUUACUGUCACGGUUCCCAA | 12 | UUGGGAACCGUGACAGUAAUAGC |
| AD03 | 13 | CUAUUUAUUUUGAGUCUGU | 14 | ACAGACUCAAAAUAAAUAGGA |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| AD04 | 15 | UGAAGAUAUAUUG UAGUAGAU | 16 | AUCUACUACAAUAUAUCUUC AAA |
| AD05 | 17 | UUGUCUCAUGUUU CAUCGUAA | 18 | UUACGAUGAAACAUGAGACA AAA |
| AD06 | 19 | CUGUGCAGUAUCU GUUCCAUU | 20 | AAUGGAACAGAUACUGCACA GAC |
| AD07 | 21 | AAUGAAAGGACUC ACUUGGUA | 22 | UACCAAGUGAGUCCUUUCAU UUG |
| AD08 | 23 | GCUGCAUGAUCAG CUAUGGUA | 24 | UACCAUAGCUGAUCAUGCAG CGG |
| AD09 | 25 | GGUGCUUGGUCUC CUCUAUAA | 26 | UUAUAGAGGAGACCAAGCAC CUU |
| AD10 | 27 | AUUGGUCAUCCCA GAACUACA | 28 | UGUAGUUCUGGGAUGACCAA UUC |
| AD11 | 29 | UAUUUAUUUUGAG UCUGUG | 30 | CACAGACUCAAAAUAAAUAG G |
| AD12 | 53 | AUUUACUGUCACG GUUCCCAA | 54 | UUGGGAACCGUGACAGUAAA UGC |

In some embodiments, the dsRNA (modified) is selected from the group consisting of:

Table 6. dsRNAs (modified)

| dsRN A No. | Sense strand SEQ ID NO | Sequence in the 5' to 3' direction | Antisense strand SEQ ID NO | Sequence in the 5' to 3' direction |
|---|---|---|---|---|
| M01 | 31 | asgscaauauGfAfCfaauugaau ga | 32 | usCfsauuCfaauugucAfuAfuugc usasc |
| M02 | 33 | asusuuacugUfCfAfcgguucc caa | 34 | usUfsgggAfaccgugaCfaGfuaaua sgsc |
| M03 | 35 | csusauuuAfUfUfuugagucug u | 36 | asCfsagaCfucaaaauAfaAfuagsg sa |
| M04 | 37 | usgsaagauaUfAfUfuguagua gau | 38 | asUfscuaCfuacaauaUfaUfcuuca sasa |
| M05 | 39 | ususgucucaUfGfUfuucaucg uaa | 40 | usUfsacgAfugaaacaUfgAfgacaa sasa |

(continued)

| dsRN A No. | Sense strand SEQ ID NO | Sequence in the 5' to 3' direction | Antisense strand SEQ ID NO | Sequence in the 5' to 3' direction |
|---|---|---|---|---|
| M06 | 41 | csusgugcagUfAfUfcuguucc auu | 42 | asAfsuggAfacagauaCfuGfcacag sasc |
| M07 | 43 | asasugaaagGfAfCfucacuugg ua | 44 | usAfsccaAfgugagucCfuUfucauu susg |
| M08 | 45 | gscsugcaugAfUfCfagcuaug gua | 46 | usAfsccaUfagcugauCfaUfgcagc sgsg |
| M09 | 47 | gsgsugcuugGfUfCfuccucua uaa | 48 | usUfsauaGfaggagacCfaAfgcacc susu |
| M10 | 49 | asusuggucaUfCfCfcagaacua ca | 50 | usGfsuagUfucugggaUfgAfccaa ususc |
| M11 | 51 | usasuuuaUfUfUfugagucugu g | 52 | csAfscagAfcucaaaaUfaAfauasg sg |
| M12 | 55 | asusuuacugUfCfAfcgguucc caa | 56 | usUfsgggAfaccgugaCfaGfuaaau sgsc |

In some embodiments, the siRNA (or dsRNA) ligand conjugate is selected from the group consisting of:

Table 7. dsRNA ligand conjugates

| dsRNA No. | Sense strand SEQ ID NO | Sequence in the 5' to 3' direction | Antisense strand SEQ ID NO | Sequence in the 5' to 3' direction |
|---|---|---|---|---|
| AD01L | 9 | AGCAAUAUGACAAUUGAAUGAL | 10 | UCAUUCAAUUGUCAUAUUGCUAC |
| AD02L | 11 | AUUUACUGUCACGGUUCCCAAL | 12 | UUGGGAACCGUGACAGUAAUAGC |
| AD03L | 13 | CUAUUUAUUUUGAGUCUGUL | 14 | ACAGACUCAAAAUAAAUAGGA |
| AD04L | 15 | UGAAGAUAUAUUGUAGUAGAUL | 16 | AUCUACUACAAUAUAUCUUCAAA |
| AD05L | 17 | UUGUCUCAUGUUUCAUCGUAAL | 18 | UUACGAUGAAACAUGAGACAAAA |
| AD06L | 19 | CUGUGCAGUAUCUGUUCCAUUL | 20 | AAUGGAACAGAUACUGCACAGAC |
| AD07L | 21 | AAUGAAAGGACUCACUUGGUAL | 22 | UACCAAGUGAGUCCUUUCAUUUG |
| AD08L | 23 | GCUGCAUGAUCAGCUAUGGUAL | 24 | UACCAUAGCUGAUCAUGCAGCGG |
| AD09L | 25 | GGUGCUUGGUCUCCUCUAUAAL | 26 | UUAUAGAGGAGACCAAGCACCUU |
| AD10L | 27 | AUUGGUCAUCCCAGAACUACAL | 28 | UGUAGUUCUGGGAUGACCAAUUC |
| AD11L | 29 | UAUUUAUUUUGAGUCUGUGL | 30 | CACAGACUCAAAAUAAAUAGG |
| AD12L | 53 | AUUUACUGUCACGGUUCCCAAL | 54 | UUGGGAACCGUGACAGUAAAUGC |
| M01L | 31 | asgscaauauGfAfCfaauugaaugaL | 32 | usCfsauuCfaauugucAfuAfuugcusasc |
| M02L | 33 | asusuuacugUfCfAfcgguucccaaL | 34 | usUfsgggAfaccgugaCfaGfuaauasgsc |
| M03L | 35 | csusauuuAfUfUfuugagucuguL | 36 | asCfsagaCfucaaaauAfaAfuagsgsa |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| M04L | 37 | usgsaagauaUfAfUfuguaguagauL | 38 | asUfscuaCfuacaauaUfaUfcuucasasa |
| M05L | 39 | ususgucucaUfGfUfuucaucguaaL | 40 | usUfsacgAfugaaacaUfgAfgacaasasa |
| M06L | 41 | csusgugcagUfAfUfcuguuccauuL | 42 | asAfsuggAfacagauaCfuGfcacagsasc |
| M07L | 43 | asasugaaagGfAfCfucacuugguaL | 44 | usAfsccaAfgugagucCfuUfucauususg |
| M08L | 45 | gscsugcaugAfUfCfagcuaugguaL | 46 | usAfsccaUfagcugauCfaUfgcagcsgsg |
| M09L | 47 | gsgsugcuugGfUfCfuccucuauaaL | 48 | usUfsauaGfaggagacCfaAfgcaccsusu |
| M10L | 49 | asusuggucaUfCfCfcagaacuacaL | 50 | usGfsuagUfucugggaUfgAfccaaususc |
| M11L | 51 | usasuuuaUfUfUfugagucugugL | 52 | csAfscagAfcucaaaaUfaAfauasgsg |
| M12L | 55 | asusuuacugUfCfAfcgguucccaaL | 56 | usUfsgggAfaccgugaCfaGfuaaausgsc |

L denotes the ligand as described above. In some embodiments, the ligand is selected from the group consisting of the L01 ligand and the L02 ligand. The sequence ID number of the sense strand refers to the sequence ID number of the nucleic acid sequence in the sense strand. The L at the 3' end of the sense strand sequence of the serial number of the dsRNA ligand conjugate indicates that in the dsRNA ligand conjugate, a ligand is linked to the 3' end of the sense strand sequence.

Pharmaceutically Acceptable Salt

In some embodiments, the salt described above is selected from the group consisting of a base addition salt, an acid addition salt, and combinations thereof.

In some embodiments, the base addition salt is selected from the group consisting of sodium, potassium, calcium, ammonium, organic amine, magnesium salts and combinations thereof, and the acid addition salt is selected from the group consisting of salts derived from inorganic acids, salts derived from organic acids and combinations thereof. In some embodiments, the inorganic acid is selected from the group consisting of hydrochloric acid, hydrobromic acid, nitric acid, carbonic acid, bicarbonate radical, phosphoric acid, monohydrogen phosphate, dihydrogen phosphate, sulfuric acid, hydrogen sulfate, hydroiodic acid, phosphorous acid and combinations thereof, and the organic acid is selected from the group consisting of acetic acid, propionic acid, isobutyric acid, maleic acid, malonic acid, benzoic acid, succinic acid, suberic acid, fumaric acid, lactic acid, mandelic acid, phthalic acid, benzenesulfonic acid, *p*-toluenesulfonic acid, citric acid, tartaric acid, methanesulfonic acid and combinations thereof.

In some embodiments, the pharmaceutically acceptable salt is selected from the group consisting of a sodium salt.

Beneficial Effects

The double-stranded ribonucleic acid, the pharmaceutically acceptable salt thereof, or the ligand conjugate thereof of the present application has good PD-L1 inhibitory activity, *in vitro* silencing activity, and activity to inhibit or reduce PD-L1 mRNA expression *in vivo* (in human PD-L1 model mice or AAV-hPD-L1 mice). The double-stranded ribonucleic acid, the pharmaceutically acceptable salt thereof, or the ligand conjugate thereof of the present application has good stability and can function continuously *in vivo* for a long term. The double-stranded ribonucleic acid, the pharmaceutically acceptable salt thereof, or the ligand conjugate thereof of the present application has low off-target effects, mild adverse effects, and good safety. Therefore, the present application has good pharmaceutical prospects.

**Definitions and Description**

Unless otherwise stated, the following terms and phrases used herein are intended to have the following meanings. A certain term or phrase, unless otherwise specifically defined, shall not be construed as uncertain or unclear, but interpreted according to the meaning as understood by those of ordinary skill in the art. When referring to a trade name, it is intended to refer to its corresponding commercial product or its active ingredient.

In the present application, unless otherwise stated, the terms "comprise", "comprises", and "comprising" or equivalents thereof are open-ended statements and mean that elements, components, or steps that are not specified may be included in addition to those listed.

When any variable occurs more than once in a compound, nucleotide, or single- or double-stranded structure, the definition of the variable in each case is independent. For example, the modifications described herein occur independently, i.e., unless otherwise indicated, modifications in the sense strand do not influence modifications of the antisense strand, modifications in one nucleotide do not influence modification in another nucleotide, and modifications on the glycosyl of one nucleotide do not influence modification at another position on the glycosyl of the same nucleotide. The influence includes the presence of the modification and the type of the modification.

The PD-L1 gene sequence of the present application can be acquired from published databases, such as Genbank, e.g., the human CD274 reference sequence (NM_014143.4).

As is known in the art, the term "interfering RNA" or "RNAi" or "interfering RNA sequence" refers to a single-stranded RNA (e.g., a mature miRNA) or a double-stranded RNA (e.g., a duplex RNA such as siRNA, aiRNA, or pre-miRNA) that is capable of reducing or inhibiting the expression of a target gene or sequence (e.g., by mediating the degradation of mRNA complementary to the interfering RNA sequence or inhibiting the translation or transcription of mRNA complementary to the interfering RNA sequence) when the interfering RNA and the target gene or sequence are in the same cell. Interfering RNAs may have substantial or complete identity to a target gene or sequence, or may comprise mismatch regions (i.e., mismatch sequences).

The double-stranded ribonucleic acid of the present application serves as an interfering RNA. Hereinafter, the double-stranded ribonucleic acid of the present application is sometimes referred to as siRNA.

As is known in the art, the term "mismatch region" or "mismatch sequence" refers to a portion of the interfering RNA (e.g., siRNA, aiRNA, miRNA) sequence that does not have 100% complementarity to its target sequence. The interfering RNA (e.g., siRNA, aiRNA, miRNA) may have at least 1, 2, 3, 4, 5, 6, or more mismatch regions. The mismatch region may be consecutive or may be separated by 1, 2, 3, 4, 5, 6, 7, 8, 9, 10, 11, 12, or more nucleotides. The mismatch region may comprise a single nucleotide or may comprise 2, 3, 4, 5, 6, or more nucleotides.

The term "identity" refers to the similarity between two nucleotide sequences or between two amino acid sequences. The identity of sequences preferably relates to the percentage of nucleotides or amino acids that have the same position in two or more sequences of the same length. Specifically, the "% identity" of two amino acid sequences or two nucleotide sequences can be determined by aligning the sequences for optimal alignment (e.g., gaps may be introduced in either sequence for optimal alignment with the other sequence) and comparing the amino acids or nucleotides at the corresponding positions. Gaps are generally regarded as non-identical positions regardless of the actual position in the alignment. The "optimal alignment" is generally an alignment of two sequences that results in the highest percentage identity. The percentage identity is determined by the number of identical nucleotides in the sequences being compared (i.e., % identity = number of identical positions/total number of positions $\times$ 100). The sequence identity of the present application is at least 80%, 85%, 90%, or 95%, preferably at least 90%, and nonlimiting examples include: 80%, 81%, 82%, 83%, 84%, 85%, 86%, 87%, 88%, 89%, 90%, 91%, 92%, 93%, 94%, 95%, 96%, 97%, 98%, 99%, and 100%. The determination of percentage identity between two sequences can be accomplished by using a mathematical algorithm known to those skilled in the art.

As is known in the art, interfering RNA includes "small interfering RNA (siRNA)", which has a length of, for example, about 15-60, 15-50, 15-40, 15-30, 15-25, 19-25, 19-23, or 19-21 nucleotides. The double-stranded ribonucleic acid of the present application may serve as such an siRNA and may have a corresponding length.

As used herein, the nucleotide positions of the sense strand or the antisense strand are counted from the 5' end. For a dsRNA with 21 nucleotides in the sense strand and 23 nucleotides in the antisense strand, for example, in 5' - AGCAAUAUGACAAUUGAAUGA - 3' (SEQ ID NO: 9), position 1 is A, and position 21 is A; in 5' - UCAUUCAAUUGUCAUAUUGCUAC - 3' (SEQ ID NO: 10), position 1 is U, and position 23 is C. For a dsRNA with 21 or more nucleotides in the sense strand and 23 or more nucleotides in the antisense strand, for example, if several nucleotides are added to the end of the sense strand on the basis of SEQ ID NO: 9, and several nucleotides are added to the end of the antisense strand on the basis of SEQ ID NO: 10, the method for counting the nucleotide positions of the sense strand or the antisense strand will not be changed, that is, position 1 of the sense strand is still position 1 of SEQ ID NO: 9, and position 1 of the antisense strand is still position 1 of SEQ ID NO: 10.

As is known in the art, and unless otherwise indicated, the term "complementary" used to describe the relationship between a first nucleic acid sequence and a second nucleic acid sequence refers to the ability of an oligonucleotide or polynucleotide containing the first nucleic acid sequence to hybridize with an oligonucleotide or polynucleotide containing the second nucleic acid sequence and form a double-stranded structure under specific conditions. As described herein, "complementary" sequences may also include double-stranded structures formed by non-Watson-Crick base pairing and/or base pairing formed by non-natural or modified nucleotides, or may be double-stranded structures formed entirely by non-Watson-Crick base pairing and/or base pairing formed by non-natural or modified nucleotides, so long as the above requirements with respect to their ability to hybridize are met.

As is known in the art, "completely complementary" sequences include base pairing of an oligonucleotide or polynucleotide comprising the first nucleic acid sequence with an oligonucleotide or polynucleotide comprising the second nucleic acid sequence over the entire length of the first nucleic acid sequence and the second nucleic acid sequence.

As is known in the art, the "substantially complementary" means that two nucleic acid sequences are completely complementary or at least 85% (e.g., 85%, 86%, 87%, 88%, 89%, 90%, 91%, 92%, 93%, 94%, 95%, 96%, 97%, 98%, 99%, or 100%) of overlapping nucleotides are complementary.

The terms "complementary", "fully complementary" and "substantially complementary" used herein may be used in accordance with the base pairing between the sense strand and the antisense strand of the dsRNA, or between the antisense strand of the dsRNA and a target sequence, as will be understood from the context of their use.

In the art, "G", "C", "A", "T", and "U" generally represent the bases of guanine, cytosine, adenine, thymine, and uracil, respectively, but it is also generally known in the art that "G", "C", "A", "T", and "U" each generally also represent nucleotides that contain guanine, cytosine, adenine, thymine, and uracil as the base, respectively, which is a common way of representing deoxyribonucleic acid sequences and/or ribonucleic acid sequences. Therefore, in the context of the present disclosure, the meanings represented by "G", "C", "A", "T", and "U" include the various possible cases described above. However, it will be appreciated that the term "ribonucleotide" or "nucleotide" may also refer to a modified nucleotide (as further detailed elsewhere herein) or having an alternative replacement moiety, and those skilled in the art will recognize that guanine, cytosine, adenine, and uracil can be replaced by other moieties without substantially altering the base pairing properties of an oligonucleotide, including a nucleotide having such a replacement moiety. For example, without limitation, a nucleotide comprising inosine as its base may pair with nucleotides comprising adenine, cytosine, or uracil. Thus, nucleotides comprising uracil, guanine, or adenine may be replaced in the nucleotide sequences of dsRNA characterized herein by a nucleotide containing, for example, inosine. In another example, adenine and cytosine anywhere in the oligonucleotide can be replaced by guanine and uracil, respectively, to form G-U wobble base pairing with the target mRNA. Sequences comprising such replacement moieties are suitable for use in the compositions and methods characterized herein.

As is known in the art, the "double-stranded ribonucleic acid", "double-stranded RNA", or "dsRNA" are used interchangeably. The term "dsRNA" comprises two antiparallel and complementary nucleic acid strands having a "sense" or "antisense" orientation relative to a target RNA (e.g., the PD-L1 gene). In the examples of the present application, the dsRNA may degrade the target RNA (e.g., mRNA) through the RNA interference (RNAi) mechanism.

The double-stranded ribonucleic acid of the present application comprises a sense strand and an antisense strand. The term "sense strand" refers to a single strand of the dsRNA double strands that is substantially complementary to a region of the antisense strand. The term "antisense strand" refers to a single strand of the dsRNA double strands that is substantially complementary to a region of the target sequence. Mismatches may occur within the molecule or in the terminal regions when the sense strand is not completely complementary to the antisense strand. In general, the most tolerated mismatches are in the terminal regions. Mismatches may occur within the molecule or in the terminal regions when the antisense strand is not completely complementary to the target sequence. In general, the most tolerated mismatches are in the terminal regions.

As is known in the art, the two strands of the dsRNA can have the same or different numbers of nucleotides. The length of the double-stranded region formed by complementation may be any length that permits the degradation of the target RNA, and possible lengths are in the range of about 9 to 36 nucleotide pairs, e.g., 15 to 30 pairs, 16 to 28 pairs, 19 to 21 pairs, etc.

As is known in the art, outside of the double-stranded region, the dsRNA may comprise one or more nucleotide overhangs, each referring to at least one unpaired nucleotide/nucleoside analog. For example, when the 3' end of one strand of the dsRNA exceeds the 5' end of the other strand (or vice versa), an overhang is present. The nucleotides of the "overhang" may comprise 0-5 nucleotides, where "0" indicates no "overhang", and "5" indicates 5 additional (i.e., unpaired with the other single strand) nucleotides on one single strand of the dsRNA double strands. These optional "overhangs" may be located at the 5' and/or 3' end of either single strand of the dsRNA. In some embodiments, the "overhang" comprises 0-5 nucleotides. In some embodiments, the "overhang" comprises 0-2 nucleotides. In some embodiments, the "overhang" at the 3' and/or 5' end of the sense strand of the dsRNA has 0-2 nucleotides. In some embodiments, the "overhang" at the 3' and/or 5' end of the antisense strand of the dsRNA has 0-2 nucleotides. The nucleotides forming the "overhang" may be A, G, C, U, or T, or modified structures thereof. The nucleotides forming the "overhang" may be U, T, or dT, or modified structures thereof. In some embodiments, the "overhang" includes, but is not limited to, "TT", "dTdT", "UU", or corresponding modified structures thereof, e.g., 2'-methoxy-modified UU, i.e., uu. In some embodiments, the "overhang" at the 3' and/or 5' end of the antisense strand of the dsRNA is substantially complementary to the target RNA. In some embodiments, the "overhang" at the 3' and/or 5' end of the antisense strand of the dsRNA is completely complementary to the target RNA. In some embodiments, the "overhang" at the 3' end of the antisense strand of the dsRNA is completely complementary to the target RNA. In some embodiments, the "overhang" at the 3' end of the antisense strand of the dsRNA is selected from the group consisting of unmodified and modified GA. The term "blunt end" means that there are no unpaired nucleotides at the end of the dsRNA, i.e., no nucleotide overhang. A dsRNA with "blunt"

ends at both ends is a dsRNA with a double-stranded region over the entire length, i.e., no nucleotide overhang at either end of the molecule.

In the present application, the terms "phosphorothioate linkage" and "phosphorothioate group" are used interchangeably.

In the present application, the dsRNA or any single strand thereof is optionally modified, and both unmodified and modified ribonucleic acids fall within the protection scope of the present application. The modification does not cause significant weakening or loss of the function of the dsRNA to inhibit the expression of the PD-L1 gene. The modification of the dsRNA or any single strand thereof may be located at the 5' end and/or 3' end, a nucleotide, or an internucleotide linkage. The synthesis or modification can be performed by methods well known in the art.

In the present application, that the sense strand or the antisense strand is optionally modified means that any nucleotide of the sense strand or the antisense strand is optionally modified.

In the present application, the modification of the nucleotide includes, but is not limited to, those occurring on the glycosyl of the nucleotide, including one or more substituted or removed glycosyl moiety groups, such as the removal of the hydroxy group at a carbonyl group, or the occurrence of fluorination, amination, alkylation, hydroxyalkylation, or hydroxyalkenylation. Modifications on glycosyl may occur at various positions on the sugar ring. Illustratively, modifications on the glycosyl of the nucleotide include, but are not limited to, 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-ethyl-O-methyl, or 2'-O-allyl, and the structures of the modifications and modified nucleoside may be as follows:

| Modification | Nucleoside structure | Modification | Nucleoside structure |
|---|---|---|---|
| 2'-Dehydroxylation | 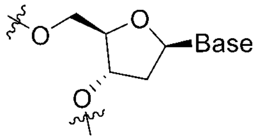 | 2'-Fluoro | 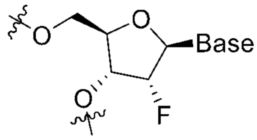 |
| 2'-Amino | 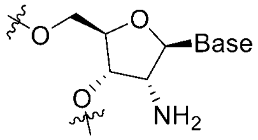 | 2'-Methyl | 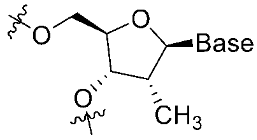 |
| 2'-Ethyl | 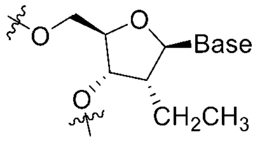 | 2'-Methyl-O-methyl | 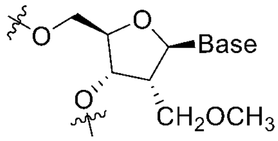 |
| 2'-Ethyl-O-methyl | 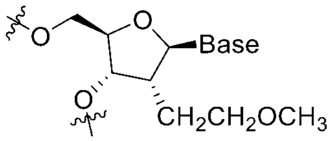 | 2'-O-methyl | 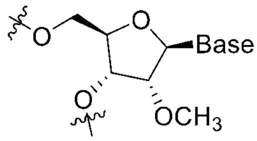 |
| 2'-O-ethyl | 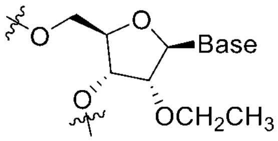 | 2'-O-ethyl-O-methyl | 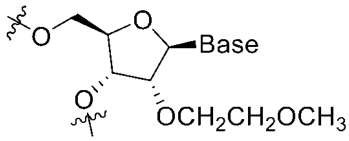 |
| 2'-O-allyl | 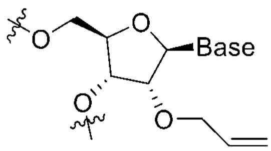 | | |

wherein Base represents a base.

In the present application, the modification of the linkage between nucleotides includes substitution or replacement of atoms or functional groups of phosphate groups, such as phosphorothioate (PS), phosphorodithioate (PS2), methylphosphonate (MP), methoxypropylphosphonate (MOP), and aminophosphonate.

In the present application, uppercase G, C, A, U, and T each generally represent a nucleotide containing guanine, cytosine, adenine, uracil, and thymine as the base, where the letter combination dT represents a deoxyribonucleotide with thymine as the base. Lowercase g, c, a, and u represent that the ribosyl groups of the nucleotides represented by their corresponding uppercase letters are modified by 2'-methoxy, that is, g, c, a, and u represent 2'-O-methyl G, 2'-O-methyl C, 2'-O-methyl A, and 2'-O-methyl U, respectively. Uppercase letters with a lowercase f on the right side indicate that the ribosyl group of the nucleotide represented by the corresponding uppercase letter is modified by 2'-fluoro, i.e., Gf, Cf, Af, and Uf represent 2'-fluoro G, 2'-fluoro C, 2'-fluoro A, and 2'-fluoro U, respectively. Lowercase s indicates that the two nucleotide residues adjacent to s are linked by a phosphorothioate group; for example, "csu" indicates that residues c and u are linked by a phosphorothioate group. VP- indicates that the nucleotide to the right of the hyphen is a (*E*)-vinylphosphate modified nucleotide; for example, "VP-u" indicates a (*E*)-vinylphosphate modified 2'-O-methyl U.

In the present application, the modification at the 5' end and/or 3' end refers to a modification occurring at the 5' end and/or 3' end of the dsRNA or any single strand thereof, e.g., phosphorylation, conjugation, reverse linkage, or the like. For example, the modification at the 5' end includes, but is not limited to, 5'-phosphate, 5'-methylphosphonate (5'-MP), 5'-phosphorothioate (5'-PS), or 5'-(*E*)-vinylphosphonate (5'-(E)-VP), and the structures of the modifications and modified nucleotide may be as follows:

| Modification | Nucleotide structure | Modification | Nucleotide structure |
|---|---|---|---|
| 5'-Phosphate | 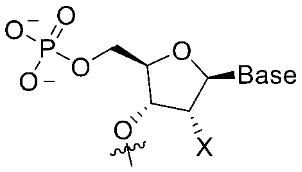 | 5'-Methylphosphonate | 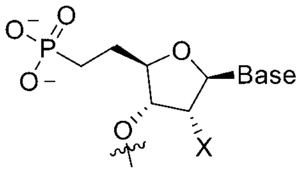 |
| 5'-Phosphorothioate | 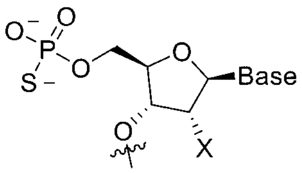 | 5'-(*E*)-vinylphosphonate | 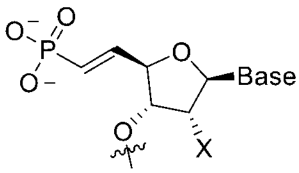 |

wherein Base represents a base, and X is selected from the group consisting of a 2' modification on hydroxy or glycosyl.

In the present application, the ligand is a group linked to the dsRNA, comprising a branched group and a linker. The dsRNA, the linker, and the branched group are linked in sequence (e.g., as shown in formula 104). The branched group comprises at least one (e.g., one, two, three, four, or five) pharmaceutically acceptable targeting group that targets the dsRNA to a particular tissue or enhances cellular uptake. The targeting group is, for example, but is not limited to, GalNAc (N-acetylgalactosamine, e.g., as shown in formula 105) group. A number of targeting groups are connected in series or in parallel via the branched group. GalNAc group may be monovalent, divalent, trivalent, or tetravalent. The monovalent, divalent, trivalent, and tetravalent described herein refer to that after the dsRNA molecule and the ligand containing GalNAc as the targeting group form the dsRNA ligand conjugate, the molar ratio of the dsRNA molecule to the GalNAc molecule in the dsRNA ligand conjugate is 1:1, 1:2, 1:3, and 1:4, respectively. In some embodiments, when the dsRNA of the present application is conjugated to a ligand comprising GalNAc, the GalNAc molecule is trivalent or tetravalent. In some embodiments, when the dsRNA of the present application is conjugated to a ligand comprising GalNAc, the GalNAc molecule is trivalent.

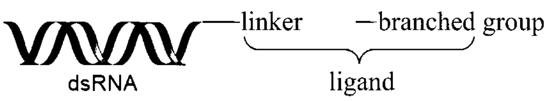


formula 104

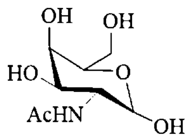

formula 105

In the present application, the ligand may be linked to the phosphate group, the 2'-hydroxyl, the 3'-hydroxyl, or the base of the nucleotide. The ligand may be linked to any nucleotide of the dsRNA, including but not limited to the 5' or 3' terminal nucleotide or the non-terminal intermediate nucleotide of the sense strand or the antisense strand. When the ligand is linked to the end of the dsRNA strand, the ligand may be linked to a phosphate group of a nucleotide; when the ligand is linked to an intermediate nucleotide of the dsRNA, the ligand may be linked to the sugar ring or the base of the nucleotide.

In the present application, for the types or preparation methods of the ligands, reference may be made to methods known in the art, including but not limited to ligands and preparation methods thereof described in WO2009082607, WO2014025805, WO2015006740, and WO2021249484, which are incorporated herein by reference in their entirety. Exemplary ligands include, but are not limited to, L01 or L02 described above. The L01 ligand described herein is the same as the L96 ligand in the prior art.

In the present application, unless otherwise stated, the "conjugation" means that two or more chemical moieties, each having a particular function, are non-covalently or covalently linked to each other; accordingly, the "conjugate" refers to a compound formed by non-covalent or covalent linking of the various chemical moieties. In the present application, conjugates that are linked covalently are preferred.

In the present application, the ligand is linked to the 5' or 3' end of the sense strand or the antisense strand. Preferably, the ligand is linked to the 5' or 3' end of the sense strand. More preferably, the ligand is linked to the 3' end of the sense strand. Illustratively, the dsRNA and the ligand are linked to form the dsRNA ligand conjugate, as shown in formula 102 or formula 103 below:

3' end of sense strand X is $O^-$, $S^-$, OH, or SH

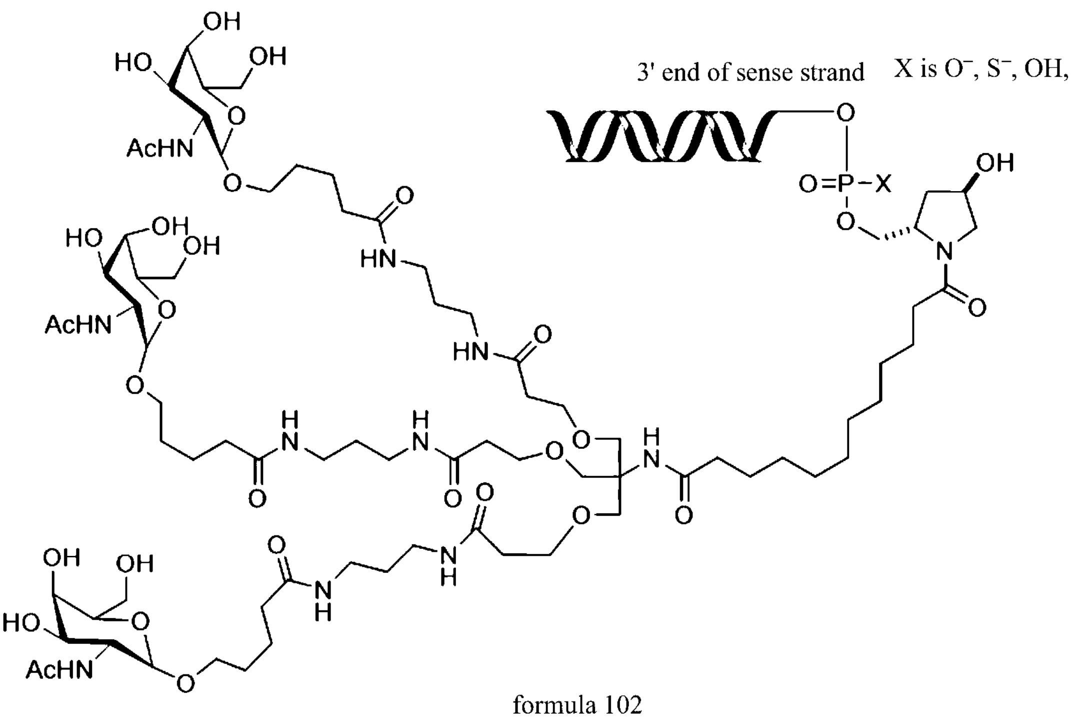

formula 102

,

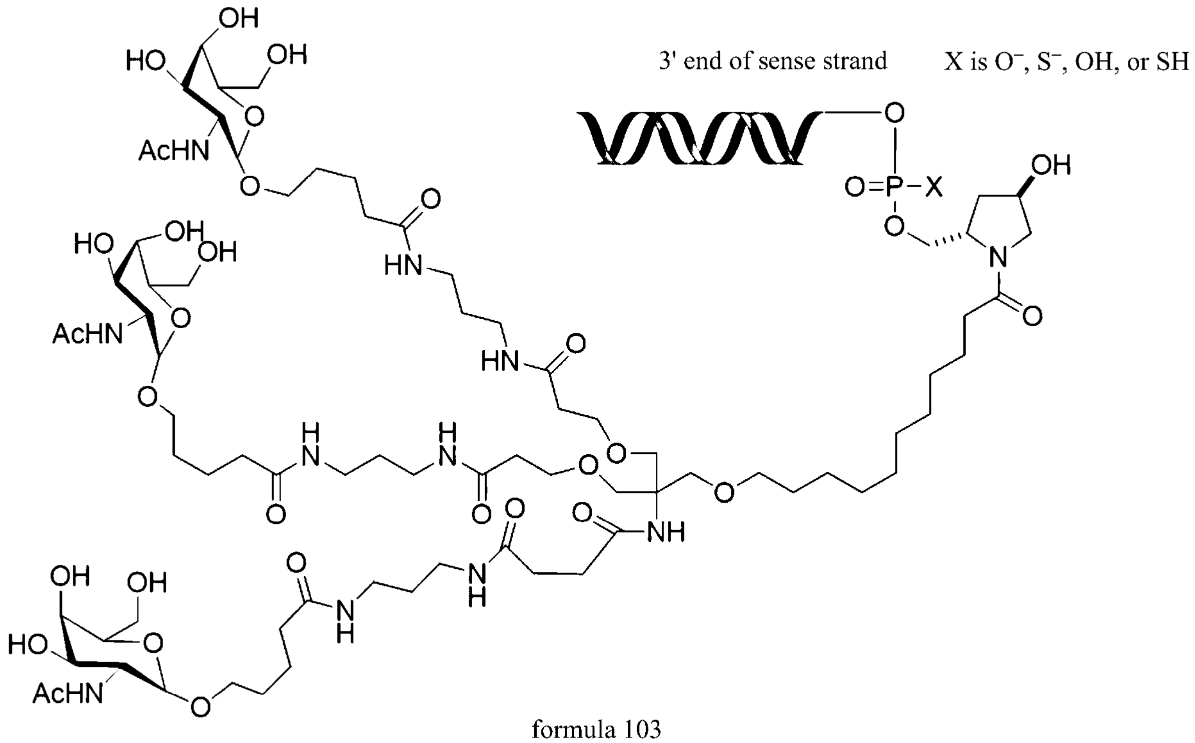


formula 103

The compound of the present application (e.g., the double-stranded ribonucleic acid, the pharmaceutically acceptable salt thereof, or the ligand conjugate thereof of the present application) may have particular geometrically isomeric or stereoisomeric forms, all of which are encompassed within the scope of the present application. All such compounds are contemplated herein, including (R)- and (S)- enantiomers, diastereoisomers, and racemic mixtures and other mixtures thereof, such as an enantiomer or diastereoisomer enriched mixture, all of which are encompassed within the scope of the present application. Additional asymmetric carbon atoms may be present in substituents such as alkyl. All these isomers and mixtures thereof are encompassed within the scope of the present application.

Unless otherwise stated, the term "enantiomer" or "optical isomer" refers to stereoisomers that are mirror images of each other.

Unless otherwise stated, the term "diastereoisomer" refers to stereoisomers whose molecules have two or more chiral centers and are not mirror images of each other.

Unless otherwise stated, the absolute configuration of a stereogenic center is represented by a wedged solid bond (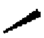) and a wedged dashed bond (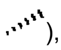), and the relative configuration of a stereogenic center is represented by a straight solid bond (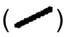) and a straight dashed bond (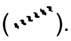). A wavy line (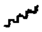) represents a wedged solid bond (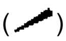) or a wedged dashed bond (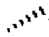), or a wavy line (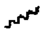) represents a straight solid bond (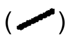) and/or a straight dashed bond (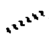).

Unless otherwise stated, the term "enriched with one isomer", "isomer enriched", "enriched with one enantiomer", or "enantiomer enriched" means that the content of one of the isomers or enantiomers is less than 100% and greater than or equal to 60%, or greater than or equal to 70%, or greater than or equal to 80%, or greater than or equal to 90%, or greater than or equal to 95%, or greater than or equal to 96%, or greater than or equal to 97%, or greater than or equal to 98%, or greater than or equal to 99%, or greater than or equal to 99.5%, or greater than or equal to 99.6%, or greater than or equal to 99.7%, or greater than or equal to 99.8%, or greater than or equal to 99.9%.

Unless otherwise stated, the term "isomeric excess" or "enantiomeric excess" refers to the difference between the relative percentages of two isomers or enantiomers. For example, if the content of one of the isomers or enantiomers is 90% and the content of the other isomer or enantiomer is 10%, the isomeric or enantiomeric excess (ee) is 80%. Optically active (R)- and (S)-isomers and D and L isomers can be prepared by chiral synthesis or chiral reagents or other conventional techniques. An enantiomer of a certain compound of the present application can be prepared by asymmetric synthesis or derivatization using a chiral additive, wherein the resulting diastereoisomeric mixture is separated and the auxiliary group is cleaved so as to provide the desired pure enantiomer. Alternatively, when the molecule contains a basic functional group (for example, amino) or an acidic functional group (for example, carboxyl), the compound reacts with an

appropriate optically active acid or base to form a salt of the diastereoisomer, which is then subjected to diastereoisomeric resolution through conventional methods well known in the art to give the pure enantiomer. Furthermore, the enantiomer and the diastereoisomer are generally isolated through chromatography using a chiral stationary phase, optionally in combination with chemical derivatization (e.g., carbamate generated from amines). The compound of the present application may contain an unnatural proportion of atomic isotope at one or more of the atoms that constitute the compound. For example, the compound may be labeled with a radioisotope, such as tritium ($^{3}H$), iodine-125 ($^{125}I$), or C-14 ($^{14}C$). For another example, hydrogen can be substituted by deuterium to form a deuterated drug, and the bond formed by deuterium and carbon is firmer than that formed by common hydrogen and carbon. Compared with an undeuterated drug, the deuterated drug has the advantages of reduced adverse effects, increased stability, enhanced efficacy, prolonged biological half-life, and the like. All isotopic variations of the compound of the present application, whether radioactive or not, are encompassed within the scope of the present application.

The term "treat", "treating", or "treatment" refers to administering the compound or formulation of the present application (e.g., the double-stranded ribonucleic acid, the pharmaceutically acceptable salt thereof, the ligand conjugate thereof, or the pharmaceutical composition of the present application) to ameliorate or eliminate a disease or one or more symptoms associated with the disease, and includes: (i) inhibiting the disease or disease state, i.e., arresting its development; and (ii) alleviating the disease or disease state, i.e., causing regression of the disease or disease state.

The term "prevent", "preventing", or "prevention" means administering the compound or formulation of the present application (e.g., the double-stranded ribonucleic acid, the pharmaceutically acceptable salt thereof, the ligand conjugate thereof, or the pharmaceutical composition of the present application) to prevent a disease or one or more symptoms associated with the disease, including preventing the occurrence of the disease or disease state in a subject, particularly when such a subject is predisposed to the disease state but has not yet been diagnosed with it.

The terms "subject" and "patient" are used interchangeably herein and refer to an animal that has been the subject of treatment, observation, or experiment. In some embodiments, the subject is a mammal, preferably a primate, and more preferably a human.

The term "therapeutically effective amount" refers to an amount of the compound of the present application (e.g., the double-stranded ribonucleic acid, the pharmaceutically acceptable salt thereof, or the ligand conjugate thereof of the present application) for (i) treating or preventing a specific disease, condition, or disorder; (ii) alleviating, ameliorating, or eliminating one or more symptoms of a specific disease, condition, or disorder, or (iii) preventing or delaying onset of one or more symptoms of a specific disease, condition, or disorder described herein. The amount of the compound of the present application composing the "therapeutically effective amount" varies depending on the compound, the disease state and its severity, the route of administration, and the age of the subject to be treated, but may be determined routinely by those skilled in the art in accordance with their knowledge and the present application.

The therapeutic dose of the compound of the present application may be determined by, for example, the specific use of the treatment, the route of administration of the compound, the health and condition of a patient, and the judgment of a prescribing physician. The proportion or concentration of the compound of the present application in a pharmaceutical composition may not be constant and depends on a variety of factors including dosages, chemical properties (e.g., hydrophobicity), and routes of administration. For example, the compound of the present application may be provided for parenteral administration by a buffered normal saline containing about 0.1-10% w/v of the compound. Certain typical dosages range from about 1 μg/kg body weight/day to about 1 g/kg body weight/day. In some certain embodiments, the dose ranges from about 0.01 mg/kg body weight/day to about 100 mg/kg body weight/day. The dose is likely to depend on variables such as the type and degree of progression of the disease or disorder, the general health condition of a specific patient, the relative biological potency of the compound selected, the excipient formulation, and its route of administration. Effective doses can be extrapolated from dose-response curves derived from *in vitro* or animal model test systems.

The term "pharmaceutically acceptable" is used herein for those compounds, materials, compositions, and/or dosage forms that are, within the scope of sound medical judgment, suitable for use in contact with the tissues of human beings and other animals without excessive toxicity, irritation, allergic response, or other problems or complications, and commensurate with a reasonable benefit/risk ratio.

The pharmaceutically acceptable salt, for example, may be a metal salt, an ammonium salt, a salt formed with an organic base, a salt formed with an inorganic acid, a salt formed with an organic acid, a salt formed with a basic or acidic amino acid, and the like.

As used herein, the pharmaceutically acceptable salt includes a pharmaceutically acceptable salt of the double-stranded ribonucleic acid and a pharmaceutically acceptable salt of the double-stranded ribonucleic acid ligand conjugate.

The term "pharmaceutical composition" refers to a mixture consisting of one or more of the compounds of the present application (e.g., the double-stranded ribonucleic acid, the pharmaceutically acceptable salt thereof, or the ligand conjugate thereof of the present application), and a pharmaceutically acceptable carrier or excipient. The pharmaceutical composition is intended to facilitate the administration of the compound of the present application to an organic entity. As used herein, the terms "pharmaceutical composition" and "formulation" have the same meaning and are used interchangeably. The carrier or excipient used herein includes any and all solvents, diluents, or other liquid excipients,

dispersing or suspending agents, surfactants, isotonizing agents, thickening or emulsifying agents, preservatives, solid binders, lubricants, and the like, suitable for the desired particular dosage form. Various carriers or excipients used to formulate pharmaceutically acceptable compositions and methods for their preparation are known in the art. The use of any conventional carrier media other than those incompatible with the compounds of the present application (e.g., those producing any undesirable biological effects or otherwise interacting in a deleterious manner with any other component of the pharmaceutically acceptable composition) is encompassed within the scope of the present application. In some specific embodiments, the carrier or excipient used herein is a carrier or excipient conventionally used in the field of dsRNA administration.

The pharmaceutical composition of the present application may be prepared by combining the compound of the present application with a suitable pharmaceutically acceptable carrier or excipient, and may be formulated, for example, into a solid, semisolid, liquid, or gaseous formulation such as tablet, pill, capsule, powder, granule, ointment, emulsion, suspension, suppository, injection, inhalant, gel, microsphere, and aerosol.

Typical routes of administration of the compound or the pharmaceutical composition thereof of the present application include, but are not limited to, oral, rectal, topical, inhalational, parenteral, sublingual, intravaginal, intranasal, intraocular, intraperitoneal, intramuscular, subcutaneous and intravenous administrations.

The pharmaceutical composition of the present application may be manufactured using methods well known in the art, such as conventional mixing, dissolving, granulating, dragee-making, levigating, emulsifying, and lyophilizing. The kit of the present application comprises the double-stranded ribonucleic acid, the pharmaceutically acceptable salt thereof, the ligand conjugate thereof, or the pharmaceutical composition of the present application, and optionally an instruction for use of the double-stranded ribonucleic acid, the pharmaceutically acceptable salt thereof, the ligand conjugate thereof, or the pharmaceutical composition of the present application in treating and/or preventing hepatitis B virus infection.

The solvents used herein are commercially available.

Unless otherwise specified, the solvent ratios used in column chromatography and preparative thin-layer silica gel chromatography in the present application are volume ratios.

It is known in the art that modified nucleotide groups can be introduced into the dsRNA described herein by using nucleoside monomers with corresponding modifications. Methods for preparing correspondingly modified nucleoside monomers and methods for introducing modified nucleotide groups into dsRNAs are known to those skilled in the art. All modified nucleoside monomers are commercially available or can be prepared using known methods.

It is known in the art that the desired ribonucleic acid can be acquired by conventional ribonucleic acid preparation methods (e.g., solid-phase synthesis and liquid-phase synthesis) in the art; for example, the desired ribonucleic acid can be synthesized by phosphoramidite-based solid-phase synthesis techniques. The method for preparing the double-stranded ribonucleic acid, the pharmaceutically acceptable salt thereof, or the ligand conjugate thereof of the present application comprises the following steps: sequentially linking nucleotide monomers in the 3' to 5' direction according to the type or order of nucleotides in the sense strand or the antisense strand of the double-stranded ribonucleic acid to synthesize the sense strand and the antisense strand. The ligation of each nucleotide monomer involves four reactions: deprotection, coupling, capping, and oxidation or sulfurization. Those skilled in the art can determine conventional reaction conditions and types and amounts of reagents, or make adjustments according to the experimental conditions to achieve the deprotection, coupling, capping, and oxidation or sulfurization reactions.

In some embodiments, for the synthesis of the ligand-containing ribonucleic acid, the ligand may be linked to the ribonucleic acid by a coupling reaction during or after the synthesis of the oligoribonucleotide, or the ligand may be firstly linked to a solid support and then the nucleoside monomer is sequentially linked to the ligand-solid support in the direction from 3' to 5'.

Methods for purification and desalting are well known to those skilled in the art. For example, the purification of the ribonucleic acid may be achieved by preparative ion chromatography methods. For another example, the desalting of the ribonucleic acid may be achieved by a reversed-phase chromatography purification method or an ultrafiltration/centrifugation method.

Methods for annealing are well known to those skilled in the art. For example, the sense strand and the antisense strand may be mixed in a molar ratio of 1:1, heated to 70-95 °C, and then cooled to room temperature to form a double-stranded structure.

During the synthesis, the concentration of the ribonucleic acid may be detected by, for example, ion exchange chromatography, or the molecular weight may be determined by liquid chromatography-mass spectrometry, or the concentration may be determined by microspectrophotometer to control the synthesis quality. Such detection methods are well known to those skilled in the art.

Unless otherwise specified, terms in the singular form shall be deemed to include the plural form and vice versa. Unless otherwise specified, the word "a" or "an" refers to "at least one". Unless otherwise stated, "or" is used to mean "and/or".

All patents, patent applications, and other identified publications are explicitly incorporated herein by reference

for the purpose of description and disclosure. These publications are provided solely because they were disclosed prior to the filing date of the present application. All statements as to the dates of these documents or descriptions as to the content of these documents are based on the information available to the applicant and do not constitute any admission as to the correctness of the dates or the content of these documents. Moreover, in any country or region, any reference to these publications herein should not be construed as an admission that the publications form part of the commonly recognized knowledge in the art.

## DETAILED DESCRIPTION

The present application is described in detail below by way of examples, which, however, are not intended to disadvantageously limit the scope of the present application in any way. The compounds of the present application can be prepared by a variety of synthetic methods well known to those skilled in the art, including the specific embodiments listed below, embodiments formed by combinations thereof with other chemical synthetic methods, and equivalents thereof known to those skilled in the art. The preferred embodiments include, but are not limited to, the examples of the present application. It will be apparent to those skilled in the art that various changes and modifications can be made to the specific embodiments without departing from the spirit and scope of the present application.

The prepared double-stranded ribonucleic acid, pharmaceutically acceptable salt thereof, or ligand conjugate thereof can be verified to be the target product by mass spectrometry or other detection methods.

Reagents and materials

Commercially available protective monomers used in synthesis

| Protective monomer | CAS |
|---|---|
| 5'-O-(4,4-Dimethoxytrityl)-2'-O-[(tert-butyl)dimethylsilyl]-N-isobutyrylguanosine-3'-(2-cyanoethyl-N,N-diisopropyl)phosphoramidite | 147201-04-5 |
| N-Benzoyl-5'-O-(4,4-dimethoxytrityl)-2'-O-[(tert-butyl)dimethylsilyl]adenosine-3'-(2-cyanoethyl-N,N-diisopropyl)phosphoramidite | 104992-55-4 |
| N-Acetyl-5'-O-(4,4-dimethoxytrityl)-2'-O-[(tert-butyl)dimethylsilyl]cytidine-3'-(2-cyanoethyl-N,N-diisopropyl)phosphoramidite | 121058-88-6 |
| 5'-0-[bis(4-Methoxyphenyl)phenylmethyl]-2'-O-[(1,1-dimethylethyl)dimethylsilyl]-uridine, 3'-[2-cyanoethyl N,N-bis(1-methylethyl)phosphoramidite] | 118362-03-1 |
| 5'-O-[bis(4-Methoxyphenyl)phenylmethyl]-2'-deoxythymidine, 3'-[2-cyanoethyl N,N-bis(1-methylethyl)phosphoramidite] | 98796-51-1 |
| N-Benzoyl-5'-O-[bis(4-methoxyphenyl)phenylmethyl]-2'-deoxy-2'-fluoroadenosine-3'-[2-cyanoethyl-N,N-diisopropyl]phosphoramidite | 136834-22-5 |
| N-Acetyl-5'-O-(4,4-dimethoxytrityl)-2'-deoxy-2'-fluorocytidine-3'-(2-cyanoethyl-N,N-diisopropyl) phosphoramidite | 159414-99-0 |
| 5'-O-[bis(4-Methoxyphenyl)phenylmethyl]-2'-deoxy-2'-fluoro-N-(2-methyl-1-oxopropyl)-guanosine-3'-(2-cyanoethyl N,N-diisopropyl)phosphoramidite | 144089-97-4 |
| 5'-O-(4,4-Dimethoxytrityl)-2'-deoxy-2'-fluorouridine-3'-(2-cyanoethyl-N,N-diisopropyl)phosphoramidite | 146954-75-8 |
| N-Benzoyl-5'-O-(4,4-dimethoxytrityl)-2'-O-methyladenosine-3'-(2-cyanoethyl-N,N-diisopropyl) phosphoramidite | 110782-31-5 |
| N-Acetyl-5'-O-(4,4-dimethoxytrityl)-2'-O-methylcytidine-3'-(2-cyanoethyl-N,N-diisopropyl)phosphoramidite | 199593-09-4 |
| N-Acetyl-5'-O-(4,4-dimethoxytrityl)-2'-O-methylguanosine-3'-(2-cyanoethyl-N,N-diisopropyl)phosphoramidite | 909033-40-5 |
| 5'-O-(4,4-Dimethoxytrityl)-2'-O-methyluridine-3'-(2-cyanoethyl-N,N-diisopropyl)phosphoramidite | 110764-79-9 |

Reagents used in synthesis

| | |
|---|---|
| Use | |
| Washing | Anhydrous acetonitrile |
| Decapping agent DBK | 3% TCA/DCM (3% trichloroacetic acid/DCM) |
| Activating agent ACT | 0.25 mol/L ETT (5-ethylthiotetrazole)/ACN |
| Capping A | 25% NMI (N-methylmorpholine)/ACN |
| Capping B | 20% acetic anhydride, 30% 2,6-lutidine/ ACN |
| Oxidant | 0.025 mol/L 12/(pyridine/water/acetonitrile = 9/1/10) |
| Sulfuration reagent | 0.2 mol/L PADS (phenylacetyl disulfide)/ACN |

The present application uses the following abbreviations:
DCM represents dichloromethane; ACN represents acetonitrile; DIC represents diisopropylcarbodiimide; DMAP represents dimethylaminopyridine; DMT represents di(p-methoxytrityl); MEM represents 2-methoxyethoxymethyl.

**Preparation Example 1. Ligation of GalNAc ligand (ligand containing GalNAc group as targeting group) to solid support**

5 mL of decapping agent DBK was added to 1.0 g of amino-CPG (amino load: 30-40 μmol/g). The mixture was reacted for 1 min, and the reagents were removed. The procedure described above was repeated 4 times. Based on the load of 35 μmol, 2 eq. of GalNAc ligand/acetonitrile solution was added, and then a condensation reagent consisting of 2.5 eq. of DIC and 2.5 eq. of DMAP was added. The mixture was reacted at 25-30 °C for 24 h. After the reaction was completed, the reaction mixture was filtered to remove the solvent and washed with anhydrous acetonitrile before the GalNAc ligand and a condensation reagent were added according to the above amounts. The system was reacted for another 24 h. After the reaction was completed, the solid support was washed with anhydrous acetonitrile, and a 4 mL 1:1 reagent mixture of capping A and capping B was added. The system was reacted for 2 min before the removal of reagents, and then 4 mL of the reagent mixture described above was added. The system was reacted for another 2 min before the removal of reagents. The mixture was washed with anhydrous acetonitrile and dried *in vacuo* at 30 °C for 1 h for later use. The GalNAc ligand in this example was L01 ligand.

**Preparation Example 2. Preparation of double-stranded ribonucleic acid**

**2.1.1 Synthesis of GalNAc ligand-ssRNA (sense strand)**

On the K&A nucleic acid synthesizer, the GalNAc ligand-solid support described above was placed into the synthetic column, and the ssRNA was synthesized according to the standard phosphoramidite-based technique. The specific procedures include:

1) DMT removal (deprotection): The resin was washed twice with acetonitrile, the DMT on the resin was removed with 3% trichloroacetic acid/DCM, and the resin was washed 4-5 times with acetonitrile.

2) Condensation (coupling): Monomers were added to the synthetic column, and a condensation reagent was added for condensation at room temperature for 10 min. The column was washed 4-5 times with acetonitrile.

3) Blocking (capping): Unreacted hydroxyl was blocked first with Capping A and then with Capping B. The column was washed 4-5 times with acetonitrile.

4) Oxidation: An oxidant was added, and the oxidation was performed for 2 min, followed by 4-5 washings with acetonitrile.

The procedures of 1) to 4) were repeated until the synthesis of all sequences was completed. Finally, DMT was removed with a decapping reagent DBK, and the system was washed 4-5 times with acetonitrile. Phosphorothioate bond formation: The oxidant was replaced with a sulfuration reagent, and the sulfuration time was set to 10 min, so as to complete the phosphorothioate bond formation.

### 2.1.2 Synthesis of ssRNA (sense strand without ligand)

On a K&A nucleic acid synthesizer, the universal CPG solid support was placed in the synthesis column, and the ssRNA was synthesized according to the standard phosphoramidite technique. The specific procedures include:

1) DMT removal (deprotection): The resin was washed twice with acetonitrile, the DMT on the resin was removed with 3% trichloroacetic acid/DCM, and the resin was washed 4-5 times with acetonitrile.

2) Condensation (coupling): Monomers were added to the synthetic column, and a condensation reagent was added for condensation at room temperature for 10 min. The column was washed 4-5 times with acetonitrile.

3) Blocking (capping): Unreacted hydroxyl was blocked first with Capping A and then with Capping B. The column was washed 4-5 times with acetonitrile.

4) Oxidation: An oxidant was added, and the oxidation was performed for 2 min, followed by 4-5 washings with acetonitrile.

The procedures of 1) to 4) were repeated until the synthesis of all sequences was completed. Finally, DMT was removed with a decapping reagent DBK, and the system was washed 4-5 times with acetonitrile.

Phosphorothioate bond formation: The oxidant was replaced with a sulfuration reagent, and the sulfuration time was set to 10 min, so as to complete the phosphorothioate bond formation.

### 2.2 Synthesis of asRNA (antisense strand without ligand)

The CPG solid support described above was placed into a synthesis column on the K&A nucleic acid synthesizer and synthesized by referring to the method described in 2.1.1 or 2.1.2.

### 2.3 Separation of ssRNA or asRNA from solid support

The cleavage reagent was aqueous ammonia:ethanol = 3:1. 5 mL of the cleavage reagent was added as per 100 mg of the solid support, and the mixture was stirred at 65-70 °C for 3 h. After the reaction was completed, the reaction mixture was cooled to room temperature, precipitated in ice n-butanol, and placed in a refrigerator at -20 °C for 30 min. The centrifugation was performed to give a precipitate, and n-butanol was added for washing; this procedure was repeated twice. Finally, the precipitate was washed with acetone and centrifuged to give a precipitate. The precipitate was dried *in vacuo* to give a crude product, which was then detected by mass spectrometry.

The LC-MS (negative ion mode) conditions are as follows:

Column: HILIC column

Mobile phase A: 20 mM ammonium formate, pH 6.2

Mobile Phase B: 20 mM ammonium formate + 95% ACN

Column temperature: 45 °C

Flow rate: 1 mL/min.

### 2.4. Annealing to form siRNA (dsRNA)

The crude single-stranded RNA products were dissolved in water, and the support was removed by filtration through a membrane. The concentrations of the sense strand and the antisense strand were determined using a microspectrophotometer. The sense strand and the antisense strand were added (in a molar ratio of 1:1), mixed, denatured at 94 °C for 4 min, and annealed by naturally cooling to room temperature.

### 2.5 Purification of siRNA (dsRNA)

The crude product was purified by DNAPac RP 10×150 mm 4 μm chromatographic column (mobile phase A: 0.1 mol/L triethylamine, pH 8.0; mobile phase B: 0.1 mol/L triethylamine, pH 8.0 + 50% ACN). The detection wavelengths were

215 nm and 260 nm. The main peak was collected and concentrated by rotary evaporation to remove most of the solvent, so as to give the target product.

### 2.6. Desalting and content assay

The mixture was centrifuged in a 3K ultrafiltration centrifuge tube at 12000× g for 12 min, concentrated by ultrafiltration, and desalted, and after the resultant mixture was concentrated by rotary evaporation, water was added for solvent exchange; the procedures were repeated 5 or more times.

Content assay: The concentration of the concentrated sample was determined on a microspectrophotometer.

### Preparation Example 3

Solid-phase synthesis: (antisense strand)

A calculated amount of anhydrous acetonitrile was added to a proper amount of the monomer to prepare a 0.2 M monomer solution.

1) A proper amount of Unylinker Support was loaded onto the Fineline70P synthesis column according to the column loading requirements in the SOP.
2) Referring to the standard operation procedures of the synthesis instrument, the monomer solutions and the reagent solutions were connected to the synthesis instrument, and the nitrogen pressure valve was opened to run the solid-phase synthesis command.
3) After the solid-phase synthesis was completed, the pipeline at the upper end of the column was removed, a nitrogen pipeline was connected, the solvent in the column was drained under a positive pressure, and the column was purged for 5 min.
4) The lower port was closed, the valve at the plunger cavity layer was opened, and nitrogen was injected; the plunger rod was pushed to the opening, and the pressure was relieved; the screw rod and the valve were removed, and the wet Oligo-Solid-support was poured into a clean crystallizing dish.
5) The residue was dried *in vacuo* until a constant weight was obtained, with the net weight of the dry product being 123.6 g.

2. Digestion

1) 123 g of the intermediate prepared above and 1230 mL of 28 wt% concentrated aqueous ammonia were added to a 3 L stainless steel reaction kettle, and the stirring speed was set to 100 rpm.
2) The system was heated for 12 h, with the external temperature being controlled at 60-85 °C and the internal temperature being controlled at 50-60 °C.
3) After the reaction was completed, the reaction mixture was poured into the Fineline70P chromatographic column and filtered under positive pressure to give a filtrate. The filter cake was rinsed with purified water and filtered again. All the filtrates were combined to give a digestion (2353.4 g, total OD value: 1375420.4).

3. Purification

1) The column efficiency test was conducted.
2) The column was sterilized.
3) Sample, eluents, and sample collection bottles were prepared. The weight and total OD of the samples to be purified were recorded, the prepared eluent was connected to the corresponding pipeline of the instrument, and the corresponding valve was opened.
4) The program was run manually. Before the manual running, it was necessary to ensure that both pumps A and B were turned on, the column was washed and equilibrated, and the eluent percentage was adjusted according to the purity of the crude product and the results of the pilot experiment in the elution process.
5) After the running was completed, the column was washed.
6) According to the in-process control results, the collected purified solutions were combined and stored in a refrigerator at 2-8 °C.

4. Ultrafiltration

5. The sense strand was synthesized according to the method for synthesizing the antisense strand.

6. Annealing

1) 1402.5 g of the antisense strand solution and 1356.2 g of the sense strand solution were poured into a flask. 200 g of purified water was added to the glass bottles containing the solutions for rinsing and then poured into the reaction flask.

2) The reaction system was stirred and heated, and the temperature was set to 110 °C. The internal temperature of the reaction system was raised to 87-91 °C and held for 30 min. The heating was stopped, and the internal temperature of the reaction system was reduced to about 25 °C. A sample was taken to determine the purity of double strands, and the content of the double strands was 94.44%.

3) The liquid was collected into a sterilized 5 L PP material barrel. The reaction flask was rinsed with a small amount of purified water, and the liquids were combined. The net weight of the liquid was 2978.7 g.

7. The mixture was lyophilized to give a lyophilized solid (71.6 g).

The double-stranded ribonucleic acids are shown below:

Table 8. Double-stranded ribonucleic acids

| dsRNA No. | Sense strand SEQ ID NO | Sequence in the 5' to 3' direction | Antisense strand SEQ ID NO | Sequence in the 5' to 3' direction |
|---|---|---|---|---|
| AD01 | 9 | AGCAAUAUGACAAUUGAAUGA | 10 | UCAUUCAAUUGUCAUAUUGCUAC |
| AD02 | 11 | AUUUACUGUCACGGUUCCCAA | 12 | UUGGGAACCGUGACAGUAAUAGC |
| AD03 | 13 | CUAUUUAUUUUGAGUCUGU | 14 | ACAGACUCAAAAUAAAUAGGA |
| AD04 | 15 | UGAAGAUAUAUUGUAGUAGAU | 16 | AUCUACUACAAUAUAUCUUCAAA |
| AD05 | 17 | UUGUCUCAUGUUUCAUCGUAA | 18 | UUACGAUGAAACAUGAGACAAAA |
| AD06 | 19 | CUGUGCAGUAUCUGUUCCAUU | 20 | AAUGGAACAGAUACUGCACAGAC |
| AD07 | 21 | AAUGAAAGGACUCACUUGGUA | 22 | UACCAAGUGAGUCCUUUCAUUUG |
| AD08 | 23 | GCUGCAUGAUCAGCUAUGGUA | 24 | UACCAUAGCUGAUCAUGCAGCGG |
| AD09 | 25 | GGUGCUUGGUCUCCUCUAUAA | 26 | UUAUAGAGGAGACCAAGCACCUU |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| AD10 | 27 | AUUGGUCAUCCCA GAACUACA | 28 | UGUAGUUCUGGGAUGACC AAUUC |
| AD11 | 29 | UAUUUAUUUUGAG UCUGUG | 30 | CACAGACUCAAAAUAAAU AGG |
| AD12 | 53 | AUUUACUGUCACG GUUCCCAA | 54 | UUGGGAACCGUGACAGUA AAUGC |
| M01 | 31 | asgscaauauGfAfCfaau ugaauga | 32 | usCfsauuCfaauugucAfuAfuug cusasc |
| M02 | 33 | asusuuacugUfCfAfcgg uucccaa | 34 | usUfsgggAfaccgugaCfaGfuaa uasgsc |
| M03 | 35 | csusauuuAfUfUfuugag ucugu | 36 | asCfsagaCfucaaaauAfaAfuags gsa |
| M04 | 37 | usgsaagauaUfAfUfugu aguagau | 38 | asUfscuaCfuacaauaUfaUfcuuc asasa |
| M05 | 39 | ususgucucaUfGfUfuuc aucguaa | 40 | usUfsacgAfugaaacaUfgAfgac aasasa |
| M06 | 41 | csusgugcagUfAfUfcug uuccauu | 42 | asAfsuggAfacagauaCfuGfcaca gsasc |
| M07 | 43 | asasugaaagGfAfCfuca cuuggua | 44 | usAfsccaAfgugagucCfuUfuca uususg |
| M08 | 45 | gscsugcaugAfUfCfagc uauggua | 46 | usAfsccaUfagcugauCfaUfgca gcsgsg |
| M09 | 47 | gsgsugcuugGfUfCfucc ucuauaa | 48 | usUfsauaGfaggagacCfaAfgcac csusu |
| M10 | 49 | asusuggucaUfCfCfcag aacuaca | 50 | usGfsuagUfucugggaUfgAfcca aususc |
| M11 | 51 | usasuuuaUfUfUfugagu cugug | 52 | csAfscagAfcucaaaaUfaAfauas gsg |
| M12 | 55 | asusuuacugUfCfAfcgg uucccaa | 56 | usUfsgggAfaccgugaCfaGfuaa ausgsc |
| M01L | 31 | asgscaauauGfAfCfaau ugaaugaL | 32 | usCfsauuCfaauugucAfuAfuug cusasc |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| M02L | 33 | asusuuacugUfCfAfcgg uucccaaL | 34 | usUfsgggAfaccgugaCfaGfuaa uasgsc |
| M03L | 35 | csusauuuAfUfUfuugag ucuguL | 36 | asCfsagaCfucaaaauAfaAfuags gsa |
| M04L | 37 | usgsaagauaUfAfUfugu aguagauL | 38 | asUfscuaCfuacaauaUfaUfcuuc asasa |
| M05L | 39 | ususgucucaUfGfUfuuc aucguaaL | 40 | usUfsacgAfugaaacaUfgAfgac aasasa |
| M06L | 41 | csusgugcagUfAfUfcug uuccauuL | 42 | asAfsuggAfacagauaCfuGfcaca gsasc |
| M07L | 43 | asasugaaagGfAfCfuca cuugguaL | 44 | usAfsccaAfgugagucCfuUfuca uususg |
| M08L | 45 | gscsugcaugAfUfCfagc uaugguaL | 46 | usAfsccaUfagcugauCfaUfgca gcsgsg |
| M09L | 47 | gsgsugcuugGfUfCfucc ucuauaaL | 48 | usUfsauaGfaggagacCfaAfgcac csusu |
| M10L | 49 | asusuggucaUfCfCfcag aacuacaL | 50 | usGfsuagUfucugggaUfgAfcca aususc |
| M11L | 51 | usasuuuaUfUfUfugagu cugugL | 52 | csAfscagAfcucaaaaUfaAfauas gsg |
| M12L | 55 | asusuuacugUfCfAfcgg uucccaaL | 56 | usUfsgggAfaccgugaCfaGfuaa ausgsc |
| M01L01 | 31 | asgscaauauGfAfCfaau ugaaugaL01 | 32 | usCfsauuCfaauugucAfuAfuug cusasc |
| M02L01 | 33 | asusuuacugUfCfAfcgg uucccaaL01 | 34 | usUfsgggAfaccgugaCfaGfuaa uasgsc |
| M03L01 | 35 | csusauuuAfUfUfuugag ucuguL01 | 36 | asCfsagaCfucaaaauAfaAfuags gsa |
| M04L01 | 37 | usgsaagauaUfAfUfugu aguagauL01 | 38 | asUfscuaCfuacaauaUfaUfcuuc asasa |
| M05L01 | 39 | ususgucucaUfGfUfuuc aucguaaL01 | 40 | usUfsacgAfugaaacaUfgAfgac aasasa |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| M06L01 | 41 | csusgugcagUfAfUfcug uuccauuL01 | 42 | asAfsuggAfacagauaCfuGfcaca gsasc |
| M07L01 | 43 | asasugaaagGfAfCfuca cuugguaL01 | 44 | usAfsccaAfgugagucCfuUfuca uususg |
| M08L01 | 45 | gscsugcaugAfUfCfagc uaugguaL01 | 46 | usAfsccaUfagcugauCfaUfgca gcsgsg |
| M09L01 | 47 | gsgsugcuugGfUfCfucc ucuauaaL01 | 48 | usUfsauaGfaggagacCfaAfgcac csusu |
| M10L01 | 49 | asusuggucaUfCfCfcag aacuacaL01 | 50 | usGfsuagUfucugggaUfgAfcca ususc |
| M11L01 | 51 | usasuuuaUfUfUfugagu cugugL01 | 52 | csAfscagAfcucaaaaUfaAfauas gsg |
| M12L01 | 55 | asusuuacugUfCfAfcgg uucccaaL01 | 56 | usUfsgggAfaccgugaCfaGfuaa ausgsc |
| M01L02 | 31 | asgscaauauGfAfCfaau ugaaugaL02 | 32 | usCfsauuCfaauugucAfuAfuug cusasc |
| M02L02 | 33 | asusuuacugUfCfAfcgg uucccaaL02 | 34 | usUfsgggAfaccgugaCfaGfuaa uasgsc |
| M03L02 | 35 | csusauuuAfUfUfuugag ucuguL02 | 36 | asCfsagaCfucaaaauAfaAfuags gsa |
| M04L02 | 37 | usgsaagauaUfAfUfugu aguagauL02 | 38 | asUfscuaCfuacaauaUfaUfcuuc asasa |
| M05L02 | 39 | ususgucucaUfGfUfuuc aucguaaL02 | 40 | usUfsacgAfugaaacaUfgAfgac aasasa |
| M06L02 | 41 | csusgugcagUfAfUfcug uuccauuL02 | 42 | asAfsuggAfacagauaCfuGfcaca gsasc |
| M07L02 | 43 | asasugaaagGfAfCfuca cuugguaL02 | 44 | usAfsccaAfgugagucCfuUfuca uususg |
| M08L02 | 45 | gscsugcaugAfUfCfagc uaugguaL02 | 46 | usAfsccaUfagcugauCfaUfgca gcsgsg |
| M09L02 | 47 | gsgsugcuugGfUfCfucc ucuauaaL02 | 48 | usUfsauaGfaggagacCfaAfgcac csusu |

(continued)

| dsRNA No. | Sense strand SEQ ID NO | Sequence in the 5' to 3' direction | Antisense strand SEQ ID NO | Sequence in the 5' to 3' direction |
|---|---|---|---|---|
| M10L02 | 49 | asusuggucaUfCfCfcag aacuacaL02 | 50 | usGfsuagUfucugggaUfgAfcca aususc |
| M11L02 | 51 | usasuuuaUfUfUfugagu cugugL02 | 52 | csAfscagAfcucaaaaUfaAfauas gsg |
| M12L02 | 55 | asusuuacugUfCfAfcgg uucccaaL02 | 56 | usUfsgggAfaccgugaCfaGfuaa ausgsc |

Uppercase G, C, A, and U generally represent nucleotides containing guanine, cytosine, adenine, and uracil as bases, respectively; lowercase g, c, a, and u represent 2'-methoxy-modified ribosyl groups of the nucleotides represented by the corresponding uppercase letters, respectively, i.e., g, c, a, and u represent 2'-O-methyl G, 2'-O-methyl C, 2'-O-methyl A, and 2'-O-methyl U, respectively; uppercase letters with a lowercase f on the right side represent 2'-fluoro-modified ribosyl groups of the nucleotides represented by the corresponding uppercase letters, i.e., Gf, Cf, Af, and Uf represent 2'-fluoro G, 2'-fluoro C, 2'-fluoro A, and 2'-fluoro U, respectively; lowercase s indicates that the two nucleotide residues adjacent to s are linked by a phosphorothioate group; for example, "csu" indicates that residues c and u are linked by a phosphorothioate group, and L represents the ligand, e.g., the L01 ligand and the L02 ligand with the following structures:

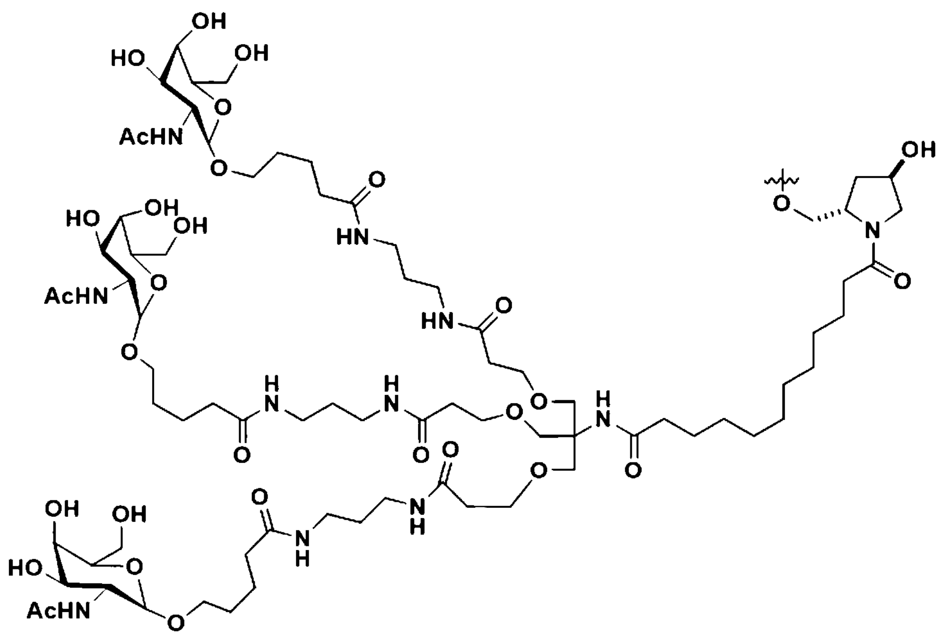

**L01 ligand**

or

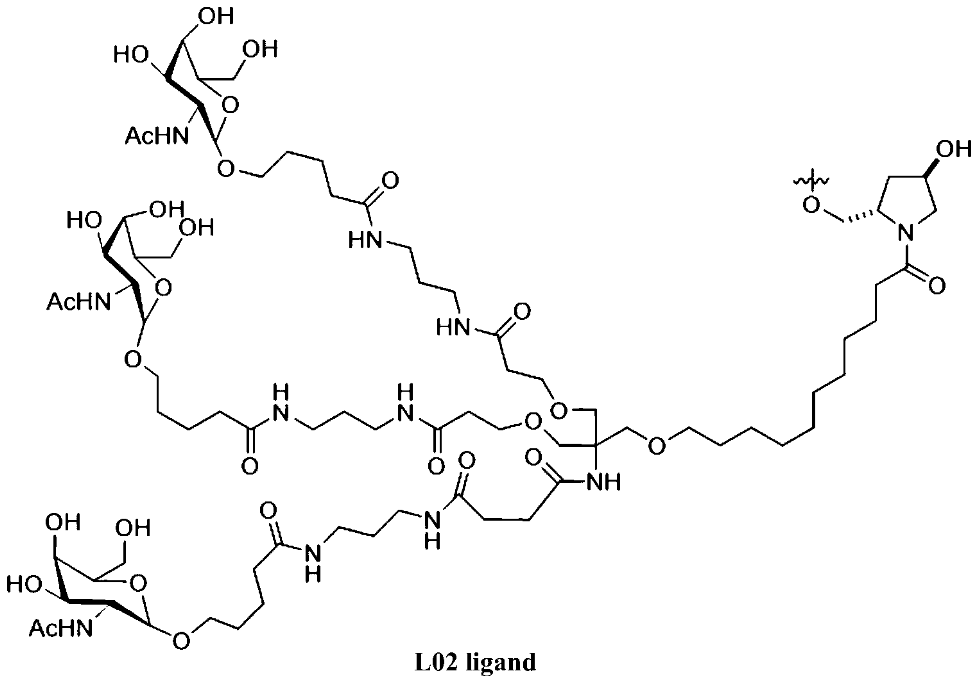

**L02 ligand**

The double-stranded ribonucleic acid, the pharmaceutically acceptable salt thereof, or the ligand conjugate thereof (the ligand is selected from the group consisting of an L01 ligand and an L02 ligand) described above may be prepared according to the method described herein or a method known in the art.

The mass spectrometry data for the double-stranded ribonucleic acid, the pharmaceutically acceptable salt thereof, or the ligand conjugate thereof of the present application were as expected, which confirmed that the target products were obtained.

**Experimental Example 1. *In vitro* screening A**

**1. Cell culture and plasmid transfection by dual luciferase method**

Cos7 cells (purchased from Nanjing Cobioer Biosciences Co., Ltd.) were cultured in DMEM (Viva Cell, Cat. No. C3113-0500) complete medium containing 10% fetal bovine serum (Gibco, Cat. No. 10099-141) in an incubator at 37 °C with 5% $CO_2$/95% air. When the cells grew to near confluence, they were trypsinized and released from the flask. Cos7 cells were seeded in a 96-well plate at $1.5 \times 10^4$ cells/well and cultured overnight. The intact human CD274 reference sequence (NM_014143.4) was cloned into dual-luciferase pmirGLO vector (Promega, Cat. No. E1330) (Nanjing GenScript Biotech Co., Ltd., C3872GD150) using a construct with an insert of about 3634 bp in length, and the pmirGLO plasmid and siRNA were transfected into $1.5 \times 10^4$ cells using LipofectamineTM 3000 (Invitrogen, Cat. No. L3000015) and LipofectamineTM RNAiMAX (Invitrogen, Cat. No. 13778150) transfection reagents. The transfection method is as follows: 0.3 μL of LipofectamineTM 3000 transfection reagent was added to 5 μL of OPTI-MEM medium (Thermo, Cat. No. 11058021), and the mixture was mixed thoroughly by pipetting; the pmirGLO plasmid at 50 ng/well and P3000 at 0.2 μL/well were added to 5 μL of OPTI-MEM medium (Thermo, Cat. No. 11058021), and the mixture was thoroughly mixed by pipetting; the diluted plasmid (in a ratio of 1:1) were added to the OPTI-MEM medium containing the transfection reagent, and the mixture was thoroughly mixed by pipetting and left to stand at room temperature for 5 min; the complex (a mixture of the diluted transfection reagent Lipofectamine 3000 and the diluted plasmid and p3000) was added to the culture wells before 90 μL of DMEM complete culture medium was added to each well, and the mixture was incubated for another 8 h; the medium was exchanged; the siRNA was diluted to 2 μM and 0.2 μM with enzyme-free water; 0.3 μL of Lipofectamine™ RNAiMAX transfection reagent was added to 10 μL of OPTI-MEM culture medium, and the mixture was thoroughly mixed by pipetting; then 0.5 μL of siRNA was added to the OPTI-MEM culture medium containing the transfection reagent, and the mixture was thoroughly mixed by pipetting and left to stand at room temperature for 5 min; the complex was added to the culture wells before 90 μL of DMEM complete medium was added to each well, the plate was incubated for another 48 h, and then the luciferase was detected using the Dual-Glo® luciferase assay system (Promega, Cat. No. E2940); A single-dose study was performed at final duplex concentrations of 10 nM and 1 nM. The siRNAs used for transfection were the sequences set forth in AD01, AD02, AD03, AD04, AD05, AD06, AD07, AD08, AD09, AD10, AD11, and AD12.

**2. Luciferase assay**

Firefly (transfection control) luciferase and Renilla (fused to PD-L1 target sequence) luciferase were measured 48h after the siRNA transfection. First, the broth was removed from the cell culture. The firefly luciferase activity was then measured by adding 75 μL of Dual-luciferase reagent equal to the volume of PBS (pH 7.4) to each well and mixing. The mixture was incubated at room temperature for 10 min, and then luminescence (500 nm) was measured on a multi-functional microplate reader (TECAN) to detect the firefly luciferase signal. Renilla luciferase activity was measured by adding 75 μL of Stop & Glo reagent at room temperature to each well, incubating the plate for 10 min, and measuring the luminescence again to determine the Renilla luciferase signal. The Stop & glo reagent quenched the firefly luciferase signal and allowed the renilla luciferase reaction to continue to emit light. The siRNA activity was determined by normalizing the firefly (control) signal by the Renilla (PD-L1) signal in each well. The magnitude of siRNA activity was then assessed in comparison to cells transfected with the same vector, without siRNA treatment or with non-targeting siRNA treatment. All transfections were performed in triplicate.

**3. Cell culture and transfection for QuantiGene method:**

HCC4006 cells (purchased from Nanjing Cobioer Biosciences Co., Ltd.) were cultured in RPMI 1640 (Bio-Channel, Cat. No. BC-M-017-500mL) complete medium containing 10% fetal bovine serum in an incubator at 37 °C with 5% $CO_2$/95% air. When the cells grew to near confluence, they were trypsinized and released from the flask. HCC4006 cells were seeded in a 96-well plate at $2 \times 10^5$ cells/well and cultured overnight. The siRNA transfection method is as follows: the siRNA was diluted to 2 μM and 0.02 μM with enzyme-free water; 0.3 μL of Lipofectamine RNAiMAX transfection reagent was added to 10 μL of OPTI-MEM culture medium, and the mixture was thoroughly mixed by pipetting; then 0.5 μL of siRNA was added to the OPTI-MEM culture medium containing the transfection reagent, and the mixture was thoroughly mixed by pipetting and left to stand at room temperature for 5 min; the complex was added to the culture wells before 90 μL of RPMI 1640 complete medium was added to each well, and the plate was incubated for another 48 h. A single-dose study was performed at final duplex concentrations of 10 nM and 0.1 nM. For the dose-response study, the sample was serially 3-fold diluted from 10 nM for 8 times. The siRNAs used for transfection were the sequences set forth in M01, M02, M03, M04, M05, M06, M07, M08, M09, M10, M11, and M12.

**4. Detection by QuantiGene method**

48 h after the siRNA transfection, PD-L1 mRNA expression levels in the cells were detected using QuantiGene™ Sample Processing Kit (Thermo, Cat. No. QS0103), QuantiGene™ Probe Set (Thermo, Cat. No. QGS-1000), and QuantiGene™ Signalplex Assay Kit (Thermo, Cat. No. QS0384). The procedures were performed according to the instructions. The chemiluminescence values (RLU) were read on a multi-functional microplate reader (TECAN), and the inhibition rate (%) = [1 - siRNA group (RLU)/control group (RLU)] × 100. The concentration-inhibition rate (%) data were processed using EXCEL software, and the inhibition effect EC50 of siRNA on PD-L1 was calculated by a four-parameter nonlinear regression model. All transfections were performed in duplicate.

The results are shown in Tables 9, 10, and 11.

Table 9. Silencing efficiency (%) by dual-luciferase method

| siRNA No. | 10nM | | lnM | |
|---|---|---|---|---|
| | Avg | SD | Avg | SD |
| AD01 | 69.4% | 7.6% | 56.7% | 1.6% |
| AD02 | 74.6% | 1.3% | 65.2% | 1.3% |
| AD03 | 88.9% | 1.8% | 83.4% | 1.2% |
| AD04 | 67.0% | 4.9% | 46.6% | 2.9% |
| AD05 | 62.9% | 2.8% | 59.2% | 1.4% |
| AD06 | 60.2% | 3.9% | 60.7% | 2.6% |
| AD07 | 67.8% | 8.2% | 58.0% | 0.0% |
| AD08 | 70.1% | 6.1% | 68.5% | 2.1% |
| AD09 | 66.4% | 2.1% | 56.9% | 1.3% |
| AD10 | 66.1% | 1.7% | 59.5% | 6.6% |

(continued)

| siRNA No. | 10nM | | InM | |
|---|---|---|---|---|
| | Avg | SD | Avg | SD |
| AD11 | 84.1% | 0.6% | 70.7% | 5.1% |
| AD12 | 91.4% | 0.8% | 84.9% | 3.5% |

Table 10. Silencing efficiency (%) in single-dose study by QuantiGene method

| siRNA No. | 10nM | | 0.1nM | |
|---|---|---|---|---|
| | Avg | SD | Avg | SD |
| M01 | 75.5% | 8.4% | 51.5% | 3.2% |
| M02 | 54.9% | 8.7% | 20.0% | 1.8% |
| M03 | 69.2% | 4.2% | 48.9% | 0.8% |
| M04 | 74.5% | 3.6% | 50.6% | 14.7% |
| M05 | 71.1% | 6.7% | 60.6% | 4.3% |
| M06 | 65.8% | 8.8% | 47.0% | 8.5% |
| M07 | 59.8% | 7.8% | 4.2% | 8.9% |
| M08 | 42.5% | 13% | 0.5% | 1.2% |
| M09 | 63.8% | 4.2% | 46.6% | 10.7% |
| M10 | 57.7% | 4.6% | 47.6% | 6.6% |
| M11 | 68.0% | 1.8% | 31.3% | 11.9% |

Table 11. $EC_{5o}$ by QuantiGene method

| siRNA No. | $EC_{50}$(pM) | Maximum inhibition rate |
|---|---|---|
| M01 | 971.6 | 71.8% |
| M02 | 297.0 | 42.5% |
| M03 | 88.5 | 70.4% |
| M04 | 800.5 | 71.0% |
| M05 | 41.5 | 69.0% |
| M06 | 182.5 | 67.0% |
| M07 | 645.9 | 39.2% |
| M08 | - | - |
| M09 | 499.4 | 66.0% |
| M11 | 136.1 | 65.0% |

The *in vitro* screening results show that the siRNAs of the present application have good *in vitro* silencing activity.

**Experimental Example 2. *In vitro* screening B**

HCC4006 cells (purchased from Nanjing Cobioer Biosciences Co., Ltd.) were cultured in RPMI 1640 complete medium (Bio-Channel, Cat. No. BC-M-017-500mL) containing 10% fetal bovine serum in an incubator at 37 °C with 5% $CO_2$/95% air. When the cells grew to near confluence, they were trypsinized and released from the flask. HCC4006 cells were seeded in a 48-well plate at $5 \times 10^4$ cells/well and cultured overnight. The siRNA transfection method is as follows: the siRNA (20 μM) was diluted to 2 μM, 0.4 μM, 0.08 μM, 0.016 μM, 0.0032 μM, and 0.00064 μM with enzyme-free water; 1.25 μL of Lipofectamine RNAiMAX (Invitrogen, Cat. No. 13778150) transfection reagent was added to 25 μL of OPTI-MEM culture medium (Thermo, Cat. No. 11058021), and the mixture was thoroughly mixed by pipetting; then 1.25 μL of

siRNA dilutions of different concentrations were added to the 25 μL of OPTI-MEM culture medium containing the transfection reagent, and the mixture was thoroughly mixed by pipetting and left to stand at room temperature for 5 min; the complex was added to the culture wells before 225 μL of RPMI 1640 complete medium was added to each well, and the plate was incubated for another 48 h. A single-dose study was performed at serially 5-fold diluted duplex concentrations from 10 nM for 6 times. The siRNAs used for transfection were the sequences set forth in M03 and M12, and all transfections were performed in duplicate.

**1. Detection by qRT-PCR**

48 h after the siRNA transfection, the total cellular RNA was extracted using the TaKaRa MiniBEST Universal RNA Extraction Kit (TAKARA, Cat. No. 9767) according to the procedures for total RNA extraction in the instructions.

For the extracted total cellular RNA, 500 ng of total RNA was taken as a template for reverse transcription, and 10 μL of reverse transcription reaction system was prepared according to the reverse transcription operation procedures in the kit instructions using the reagents provided by the PrimeScript™ RT Master Mix (Takara, Cat. No. RR036A) to perform reverse transcription on the total cellular RNA. The reverse transcription conditions are as follows: the reverse transcription reaction system was incubated at 37 °C for 15 min and then incubated at 85 °C for 5 s, and after the reaction was completed, a cDNA-containing solution was obtained.

For each reverse transcription reaction system, the cDNA-containing solution described above was taken as the qPCR template, and 20 μL of the qPCR reaction system was prepared using the reagent provided by the ChamQ SYBRqPCR Master Mix kit (Vazyme Biotech Co., Ltd., Cat. No. Q331). The PCR primer sequences for amplifying the target gene PD-L1 and the internal reference gene GAPDH are shown in Table 12, and the final concentration of each primer was 10 μM. The qPCR reaction system was placed on a LightCycler 480 fluorescent quantitative PCR system and amplified using the three-step method. The amplification program was pre-denatured at 95 °C for 3 min, denatured at 95 °C for 10 s, and annealed at 60 °C for 30 s. After the denaturation and annealing process described above were repeated for 40 times, a PCR amplification product containing the amplified target gene PD-L1 and internal reference gene GAPDH was obtained. The PCR amplification products were then incubated at 95 °C for 5 s, 65 °C for 1 min, and 97 °C for 15 s sequentially. The real-time fluorescent quantitative PCR system separately acquired the dissolution curves of the target gene PD-L1 and the internal reference gene GAPDH in the PCR amplification products to give the Ct values of the target gene PD-L1 and the internal reference gene GAPDH.

Table 12. Primer sequences

| Gene name | SEQ ID NO. | Nucleotide sequence (5' → 3') |
|---|---|---|
| Human PD-L1 | 3 | TGGCTCCCAGAATTACCAAG |
| | 4 | TATGGTGGTGCCGACTACAA |
| Human GAPDH | 57 | GGTCGGAGTCAACGGATTT |
| | 58 | CCAGCATCGCCCCACTTGA |

The expression level of PD-L1 mRNA was calculated using the relative quantification method, i.e., the ΔΔCt value method.

The calculation method is as follows:

$$\Delta Ct\ (siRNA) = Ct\ (PD\text{-}L1) - Ct\ (GAPDH)$$

$$\Delta Ct\ (NC) = Ct\ (PD\text{-}L1) - Ct\ (GAPDH)$$

$$\Delta\Delta Ct\ (siRNA) = \Delta Ct\ (siRNA) - \Delta Ct\ (mean\ NC)$$

$$\Delta\Delta Ct\ (NC) = \Delta Ct\ (NC) - \Delta Ct\ (mean\ NC)$$

$$\text{PD-L1 mRNA relative expression level} = 2^{(-\Delta\Delta Ct\ (siRNA))} \times 100\%,$$

$$\text{inhibition rate of siRNA on PD-L1 mRNA} = (1 - 2^{(-\Delta\Delta Ct\ (siRNA))}) \times 100\%;$$

The concentration-inhibition rate (%) data were processed using EXCEL software, and the inhibition effect $EC_{50}$ of siRNA on PD-L1 was calculated by a four-parameter nonlinear regression model.
The results are shown in Table 13.

Table 13. $EC_{50}$ values determined by qRT-PCR

| dsRNA No. | $EC_{50}$(pM) | Maximum inhibition rate |
|---|---|---|
| M03 | 115.2 | 77.3% |
| M12 | 56.4 | 53.0% |

**Experimental Example 3. Activity evaluation A in human PD-L1 model mice**

Male human PD-L1 mice (purchased from Shanghai Model Organisms Center, Inc.) aged 6-8 weeks were randomized by weight, with 3 mice in each group. The groups were designated as a PBS group and various sample groups (dsRNA ligand conjugate of the present application).
Each sample group (dsRNA ligand conjugate of the present application) received a 1 mg/mL solution prepared in 1× PBS (pH 7.4).
Each mouse in the PBS group was subcutaneously injected with 10 mL/kg body weight of 1× PBS (pH 7.4), and each mouse in the sample groups (dsRNA ligand conjugate of the present application) was subcutaneously injected with the corresponding solution prepared as described above. The administration volume for each animal was 10 mL/kg body weight, and the administration dose was 10 mg/kg body weight. The day of the first dose was designated as day 0. All mice were sacrificed 14 days after the treatment, and the liver was collected and preserved in 1.5 mL sterile EP tubes containing 1 mL of animal tissue RNA stabilizing solution (Shanghai Beyotime Biotech Inc., Cat. No. R0118). After being preserved in a refrigerator at 4 °C for 24 h, the liver was transferred to -80 °C for storage. Then, the liver tissue of each mouse was homogenized using a tissue homogenizer, and the total RNA of the liver tissue of each mouse was separately extracted using the TaKaRa MiniBEST Universal RNA Extraction Kit (TAKARA, Cat. No. 9767) according to the procedures for total RNA extraction in the instructions.
For the total RNA extracted from the liver tissue of each mouse, 1 μg of the total RNA was taken as the template for reverse transcription, and 20 μL of reverse transcription reaction system was prepared according to the reverse transcription operation procedures in the kit instructions using the reagents provided by the HiScript III 1st Strand cDNA Synthesis Kit (+ gDNA wiper) (Vazyme Biotech Co., Ltd., Cat. No. R312), so as to perform reverse transcription on the obtained liver total RNA. The reverse transcription conditions are as follows: the reverse transcription reaction system was incubated at 37 °C for 15 min and then incubated at 85 °C for 5 s, and after the reaction was completed, a cDNA-containing solution was obtained.
For each reverse transcription reaction system, the cDNA-containing solution described above was taken as the qPCR template, and 20 μL of the qPCR reaction system was prepared using the reagent provided by the ChamQ SYBRqPCR Master Mix kit (Vazyme Biotech Co., Ltd., Cat. No. Q311). The PCR primer sequences for amplifying the target gene PD-L1 and GAPDH are shown in Table 14, and the final concentration of each primer was 0.2 μM. The qPCR reaction system was placed on a LightCycler 480 fluorescent quantitative PCR system and amplified using the three-step method. The amplification program was pre-denatured at 95 °C for 5 min, denatured at 95 °C for 10 s, annealed at 60 °C for 10 s, and extended at 72 °C for 10 s. After the denaturation, annealing, and extension process described above were repeated for 40 times, a PCR amplification product containing the amplified target gene PD-L1 and internal reference gene GAPDH was obtained. The PCR amplification products were then incubated at 95 °C for 5 s, 65 °C for 1 min, and 97 °C for 15 s sequentially. The real-time fluorescent quantitative PCR system separately acquired the dissolution curves of the target gene PD-L1 and the internal reference gene GAPDH in the PCR amplification products to give the Ct values of the target gene PD-L1 and the internal reference gene GAPDH.

Table 14. Primer sequences

| Gene name | SEQ ID NO. | Nucleotide sequence (5' → 3') |
|---|---|---|
| Human PD-L1 | 1 | TGGCATTTGCTGAACGCATTT |
| | 2 | TGCAGCCAGGTCTAATTGTTTT |
| Mouse PD-L1 | 5 | CCGGGATGAGAGATGGAGTC |
| | 6 | TGTCGTCAGAATTCACCAGTC |

(continued)

| Gene name | SEQ ID NO. | Nucleotide sequence (5' → 3') |
|---|---|---|
| Mouse GAPDH | 7 | ATGAAGGGGTCGTTGATGGC |
| | 8 | GGGTTCCTATAAATACGGACTGC |

The inhibition rates of the dsRNA ligand conjugates of the present application against PD-L 1 mRNA were calculated using the comparative Ct (ΔΔCt) method as follows:

$$\Delta Ct\ (\text{test group}) = Ct\ (\text{target gene of test group}) - Ct\ (\text{internal reference gene of test group})$$

$$\Delta Ct\ (\text{control group}) = Ct\ (\text{target gene of control group}) - Ct\ (\text{internal reference gene of control group})$$

$$\Delta\Delta Ct\ (\text{test group}) = \Delta Ct\ (\text{test group}) - \Delta Ct\ (\text{mean of control group})$$

$$\Delta\Delta Ct\ (\text{control group}) = \Delta Ct\ (\text{control group}) - \Delta Ct\ (\text{mean of control group})$$

The test group denotes the sample groups (dsRNA ligand conjugate of the present application), the control group denotes the PBS group, and ΔCt (mean of the control group) is the arithmetic mean of ΔCt (control group) of the animals in the PBS group. Thus, each animal in the sample groups (dsRNA ligand conjugate of the present application) corresponds to a ΔΔCt (test group), and each animal in the PBS group corresponds to a ΔΔCt (control group).

The inhibition rate of the dsRNA ligand conjugate against PD-L1 mRNA of each animal in the test group = (1 - 2^(-ΔΔCt (test group))/2^(-ΔΔCt (control group))) × 100%,

and the inhibition rates of the dsRNA ligand conjugates against PD-L1 mRNA in liver tissue are shown in Table 15.

Table 15. Inhibition rate of dsRNA ligand conjugate against PD-L1 mRNA

| Group | PD-L1 expression inhibition rate (%) | |
|---|---|---|
| | Avg | SD |
| M01L01 | 59.3 | 19.6 |
| M02L01 | 67.1 | 17.7 |
| M03L01 | 78.2 | 7.8 |

**Experimental Example 4. Activity evaluation B in human PD-L1 model mice**

Male human PD-L1 mice (purchased from Shanghai Model Organisms Center, Inc.) aged 6-8 weeks were randomized by weight, with 3 mice in each group. The groups were designated as a PBS group and various sample groups (dsRNA ligand conjugate of the present application).

Each sample group (dsRNA ligand conjugate of the present application) received a 1 mg/mL solution prepared in 1× PBS (pH 7.4).

Each mouse in the PBS group was subcutaneously injected with 10 mL/kg body weight of 1× PBS (pH 7.4), and each mouse in the sample groups (dsRNA ligand conjugate of the present application) was subcutaneously injected with the corresponding solution prepared as described above. The administration volume for each animal was 10 mL/kg body weight, and the administration dose was 10 mg/kg body weight. The day of the first dose was designated as day 0. The liver was collected on days 14 and 28 after the treatment and preserved in 1.5 mL sterile EP tubes containing 1 mL of animal tissue RNA stabilizing solution (Shanghai Beyotime Biotech Inc., Cat. No. R0118). After being preserved in a refrigerator at 4 °C for 24 h, the liver was transferred to -80 °C for storage. Then, the liver tissue of each mouse was homogenized using a tissue homogenizer, and the total RNA of the liver tissue of each mouse was separately extracted using the TaKaRa MiniBEST Universal RNA Extraction Kit (TAKARA, Cat. No. 9767) according to the procedures for total RNA extraction in the instructions.

The procedures of reverse transcription to synthesize cDNA and fluorescence quantitative PCR, the primers, and the calculation of inhibition rate are shown in Experimental Example 3.

The inhibition rates of the dsRNA ligand conjugates against PD-L1 mRNA in liver tissue are shown in Table 16.

Table 16. Inhibition rate of dsRNA ligand conjugate against PD-L1 mRNA

| Group | PD-L1 expression inhibition rate (%) | | | |
|---|---|---|---|---|
| | Day 14 after treatment | | Day 28 after treatment | |
| | Avg | SD | Avg | SD |
| M07L01 | 49.0 | 7.7 | - | - |
| M08L01 | 26.7 | 2.2 | - | - |
| M10L01 | 64.6 | 5.3 | 47.0 | 8.1 |
| M11L01 | 66.7 | 2.9 | 39.2 | 18.1 |
| M12L01 | 78.6 | 9.4 | 50% | 6.2 |

**Experimental Example 5. *In vivo* activity evaluation in AAV-hPD-L1 mice**

AAV-CTM-PDL1 (full-length human PD-L1 gene, titer: 6.25 $\times$ $10^{13}$ vg/mL, purchased from Shandong WZ Bioscience Inc.) was prepared into a solution at a concentration of 5 $\times$ $10^{11}$ vg/mL in sterile PBS before injection. Male C57BL/6 mice (purchased from Shanghai Bikaikeyi Biotech Inc.) aged 4-6 weeks were injected with 200 $\mu$L of the prepared AAV-CTM-PDL1 at a concentration of 5 $\times$ $10^{11}$ vg/mL via the tail vein. On day 14 after the infection, the livers of 2-5 mice were taken to detect the expression level of PD-L1 mRNA, with the (Ct) value being 24 $\pm$ 2, indicating that the modeling was successful. The mice were randomized according to the body weight, 4 mice per group.

Each sample group (dsRNA ligand conjugate of the present application) received a 1 mg/mL solution prepared in 1$\times$ PBS (pH 7.4).

Each mouse in the PBS group was subcutaneously injected with 10 mL/kg body weight of 1 $\times$ PBS (pH 7.4), and each mouse in the sample groups was subcutaneously injected with the corresponding solution prepared as described above. The administration volume for each animal was 10 mL/kg body weight, and the administration dose was 10 mg/kg body weight. The day of the first dose was designated as day 0. The liver was collected on days 14 and 28 after the treatment and preserved in 1.5 mL sterile EP tubes containing 1 mL of animal tissue RNA stabilizing solution (Shanghai Beyotime Biotech Inc., Cat. No. R0118). After being preserved in a refrigerator at 4 °C for 24 h, the liver was transferred to -80 °C for storage. Then, the liver tissue of each mouse was homogenized using a tissue homogenizer, and the total RNA of the liver tissue of each mouse was separately extracted using the Universal RNA Extraction CZ Kit (H) (Onrew, Cat. No. RNC643-03H) on an automated nucleic acid extractor (Allsheng, Cat. No. AUTO-PURE96) according to the procedures for total RNA extraction in the instructions.

For the total RNA extracted from the liver tissue of each mouse, 500 ng of total RNA was taken as a template for reverse transcription, and 10 $\mu$L of reverse transcription reaction system was prepared according to the reverse transcription operation procedures in the kit instructions using the reagents provided by the PrimeScriptTM RT Master Mix (Perfect Real Time) (TaKaRa, Cat. No. RR036A) to perform reverse transcription on the total liver RNA. The reverse transcription conditions are as follows: the reverse transcription reaction system was incubated at 37 °C for 15 min and then incubated at 85 °C for 5 s, and after the reaction was completed, a cDNA-containing solution was obtained.

The procedures of fluorescence quantitative PCR, the primers, and the calculation of inhibition rate are shown in Experimental Example 3.

According to the test results, it is found that the dsRNA ligand conjugates of the present application exhibited good inhibition effects against PD-L1 mRNA in the liver tissue, and the inhibition rates against PD-L1 expression on days 14 or 28 after treatment were greater than 50%, preferably greater than 60% or more preferably greater than 70%.

**Experimental Example 6: *In vitro* stability evaluation in liver homogenate**

1) 1 mL of tissue lysis buffer (100 mM Tris-HCl, 1 mM $MgCl_2$, pH 6.0) was added as per 200 mg of liver tissue (C57BL/6 mice), and the tissue was ground by a tissue grinder at -30 °C. The resultant liquid, i.e., blank liver homogenate, was collected and preserved at -80 °C for later use.
2) 40 $\mu$L of stock solution (concentration: 1 mg/mL) of the dsRNA ligand conjugate to be tested was taken, and 360 $\mu$L of enzyme-free water was added to dilute the stock solution to 100 $\mu$g/mL for later use. The final concentration of the compound to be tested in the system was 10 $\mu$g/mL (1:10).

3) The blank mouse liver homogenate was taken and incubated at 37 °C for 5 min, 24 h, 42 h, or 48 h.
4) 720 μL of blank liver homogenate incubated for 48 h was pipetted into a 1.5 mL Eppendorf tube, and 80 μL of the stock solution of the test compound (100 μg/mL) was added. The mixture was thoroughly mixed by vortexing to give the 0 h sample.
5) 720 μL of blank liver homogenate incubated for 5 min was pipetted into a 1.5 mL Eppendorf tube, and 80 μL of the stock solution of the test compound (100 μg/mL) was added. The mixture was thoroughly mixed by vortexing and incubated on a shaker (37 °C, 300 rpm) for 48 h to give the 48 h sample.
6) Sample pretreatment:

(1) All the samples described above were thoroughly mixed by vortexing. 50 μL of the test sample was pipetted, and 150 μL of Clarity lysis buffer (phenomenex, Cat. No. AL0-8579) was added. The mixture was vortexed for 5 min and centrifuged at 15,000 rpm for 10 min.
(2) 1 mL of methanol and 1 mL of equilibration buffer (50 mM ammonium acetate (pH = 5.5) + 0.5% Triton_X100) were sequentially added to the Clarity OTX SPE plate (phenomenex, Cat. No. 8E-S103-EGA).
(3) 185 μL of supernatant after the centrifugation in step 1) was loaded on the SPE kit.
(4) The sample was washed 3 times with 1 mL of equilibration buffer (50 mM ammonium acetate (pH = 5.5)) and then washed 3 times with 1 mL of buffer (50 mM ammonium acetate (pH = 5.5) + 50% acetonitrile).
(5) The sample was eluted twice with 1 mL of eluent (100 mM ammonium bicarbonate (pH = 9.5) + 40% acetonitrile + 10% tetrahydrofuran).
(6) Finally, the sample was dried with nitrogen at 65 °C, and 100 μL of TE buffer (10 mM Tris-HCl + 1 mM EDTA (pH = 8.0)) was added for reconstitution. The mixture was vortexed for 5 min and centrifuged at 15,000 rpm for 10 min. The supernatant was collected and loaded for analysis.

7) The sample concentration was detected by LC-MS/MS, with the zero point sample as the reference. The internal standard peak area of the analyte was used to calculate the remaining percentage, and the data were analyzed.

According to the assay results, the dsRNA ligand conjugates of the present application were found to have good stability: the remaining amount of the sense strand after 48 h of incubation exceeded 30% or 40%, and the remaining amount of the antisense strand after 48 h of incubation exceeded 60% or 80%.

## Claims

**1.** A double-stranded ribonucleic acid (dsRNA), a pharmaceutically acceptable salt thereof, or a ligand conjugate thereof, wherein the dsRNA comprises a sense strand and an antisense strand; the sense strand comprises at least 15 consecutive nucleotides in the nucleotide sequence set forth in SEQ ID NO. 9 and has a length of not greater than 23 nucleotides, and the antisense strand comprises at least 15 consecutive nucleotides in the nucleotide sequence set forth in SEQ ID NO. 10 and has a length of not greater than 25 nucleotides; or, the sense strand comprises at least 15 consecutive nucleotides in the nucleotide sequence set forth in SEQ ID NO. 11 and has a length of not greater than 23 nucleotides, and the antisense strand comprises at least 15 consecutive nucleotides in the nucleotide sequence set forth in SEQ ID NO. 12 and has a length of not greater than 25 nucleotides; or, the sense strand comprises at least 15 consecutive nucleotides in the nucleotide sequence set forth in SEQ ID NO. 13 and has a length of not greater than 21 nucleotides, and the antisense strand comprises at least 15 consecutive nucleotides in the nucleotide sequence set forth in SEQ ID NO. 14 and has a length of not greater than 23 nucleotides; or, the sense strand comprises at least 15 consecutive nucleotides in the nucleotide sequence set forth in SEQ ID NO. 15 and has a length of not greater than 23 nucleotides, and the antisense strand comprises at least 15 consecutive nucleotides in the nucleotide sequence set forth in SEQ ID NO. 16 and has a length of not greater than 25 nucleotides; or, the sense strand comprises at least 15 consecutive nucleotides in the nucleotide sequence set forth in SEQ ID NO. 17 and has a length of not greater than 23 nucleotides, and the antisense strand comprises at least 15 consecutive nucleotides in the nucleotide sequence set forth in SEQ ID NO. 18 and has a length of not greater than 25 nucleotides; or, the sense strand comprises at least 15 consecutive nucleotides in the nucleotide sequence set forth in SEQ ID NO. 19 and has a length of not greater than 23 nucleotides, and the antisense strand comprises at least 15 consecutive nucleotides in the nucleotide sequence set forth in SEQ ID NO. 20 and has a length of not greater than 25 nucleotides; or, the sense strand comprises at least 15 consecutive nucleotides in the nucleotide sequence set forth in SEQ ID NO. 21 and has a length of not greater than 23 nucleotides, and the antisense strand comprises at least 15 consecutive nucleotides in the nucleotide sequence set forth in SEQ ID NO. 22 and has a length of not greater than 25 nucleotides; or, the sense strand comprises at least 15 consecutive nucleotides in the nucleotide sequence set forth in SEQ ID NO. 23 and has a length of not greater than 23 nucleotides, and the antisense strand comprises at least 15 consecutive nucleotides in the nucleotide sequence

set forth in SEQ ID NO. 24 and has a length of not greater than 25 nucleotides; or, the sense strand comprises at least 15 consecutive nucleotides in the nucleotide sequence set forth in SEQ ID NO. 25 and has a length of not greater than 23 nucleotides, and the antisense strand comprises at least 15 consecutive nucleotides in the nucleotide sequence set forth in SEQ ID NO. 26 and has a length of not greater than 25 nucleotides; or, the sense strand comprises at least 15 consecutive nucleotides in the nucleotide sequence set forth in SEQ ID NO. 27 and has a length of not greater than 23 nucleotides, and the antisense strand comprises at least 15 consecutive nucleotides in the nucleotide sequence set forth in SEQ ID NO. 28 and has a length of not greater than 25 nucleotides; or, the sense strand comprises at least 15 consecutive nucleotides in the nucleotide sequence set forth in SEQ ID NO. 29 and has a length of not greater than 21 nucleotides, and the antisense strand comprises at least 15 consecutive nucleotides in the nucleotide sequence set forth in SEQ ID NO. 30 and has a length of not greater than 23 nucleotides; or, the sense strand comprises at least 15 consecutive nucleotides in the nucleotide sequence set forth in SEQ ID NO. 53 and has a length of not greater than 23 nucleotides, and the antisense strand comprises at least 15 consecutive nucleotides in the nucleotide sequence set forth in SEQ ID NO. 54 and has a length of not greater than 25 nucleotides:

sense strand: 5'-AGCAAUAUGACAAUUGAAUGA-3' (SEQ ID NO. 9),
antisense strand: 5'-UCAUUCAAUUGUCAUAUUGCUAC-3' (SEQ ID NO. 10);
sense strand: 5'-AUUUACUGUCACGGUUCCCAA-3' (SEQ ID NO. 11),
antisense strand: 5'-UUGGGAACCGUGACAGUAAUAGC-3' (SEQ ID NO. 12);
sense strand: 5'-CUAUUUAUUUUGAGUCUGU-3' (SEQ ID NO. 13),
antisense strand: 5'-ACAGACUCAAAAUAAAUAGGA-3' (SEQ ID NO. 14);
sense strand: 5'-UGAAGAUAUAUUGUAGUAGAU-3' (SEQ ID NO. 15),
antisense strand: 5'-AUCUACUACAAUAUAUCUUCAAA-3' (SEQ ID NO. 16);
sense strand: 5'-UUGUCUCAUGUUUCAUCGUAA-3' (SEQ ID NO. 17),
antisense strand: 5'-UUACGAUGAAACAUGAGACAAAA-3' (SEQ ID NO. 18);
sense strand: 5'-CUGUGCAGUAUCUGUUCCAUU-3' (SEQ ID NO. 19),
antisense strand: 5'-AAUGGAACAGAUACUGCACAGAC-3' (SEQ ID NO. 20);
sense strand: 5'-AAUGAAAGGACUCACUUGGUA-3' (SEQ ID NO. 21),
antisense strand: 5'-UACCAAGUGAGUCCUUUCAUUUG-3' (SEQ ID NO. 22);
sense strand: 5'-GCUGCAUGAUCAGCUAUGGUA-3' (SEQ ID NO. 23),
antisense strand: 5'-UACCAUAGCUGAUCAUGCAGCGG-3' (SEQ ID NO. 24);
sense strand: 5'-GGUGCUUGGUCUCCUCUAUAA-3' (SEQ ID NO. 25),
antisense strand: 5'-UUAUAGAGGAGACCAAGCACCUU-3' (SEQ ID NO. 26);
sense strand: 5'-AUUGGUCAUCCCAGAACUACA-3' (SEQ ID NO. 27),
antisense strand: 5'-UGUAGUUCUGGGAUGACCAAUUC-3' (SEQ ID NO. 28);
sense strand: 5'-UAUUUAUUUUGAGUCUGUG-3' (SEQ ID NO. 29),
antisense strand: 5'-CACAGACUCAAAAUAAAUAGG-3' (SEQ ID NO. 30);
sense strand: 5'-AUUUACUGUCACGGUUCCCAA-3' (SEQ ID NO. 53),
antisense strand: 5'-UUGGGAACCGUGACAGUAAAUGC-3' (SEQ ID NO. 54);
the sense strand or the antisense strand is optionally modified.

**2.** The double-stranded ribonucleic acid, the pharmaceutically acceptable salt thereof, or the ligand conjugate thereof according to claim 1, wherein the sense strand comprises the nucleotide sequence set forth in SEQ ID NO. 9 and has a length of not greater than 23 nucleotides, and the antisense strand comprises the nucleotide sequence set forth in SEQ ID NO. 10 and has a length of not greater than 25 nucleotides; or, the sense strand comprises the nucleotide sequence set forth in SEQ ID NO. 11 and has a length of not greater than 23 nucleotides, and the antisense strand comprises the nucleotide sequence set forth in SEQ ID NO. 12 and has a length of not greater than 25 nucleotides; or, the sense strand comprises the nucleotide sequence set forth in SEQ ID NO. 13 and has a length of not greater than 21 nucleotides, and the antisense strand comprises the nucleotide sequence set forth in SEQ ID NO. 14 and has a length of not greater than 23 nucleotides; or, the sense strand comprises the nucleotide sequence set forth in SEQ ID NO. 15 and has a length of not greater than 23 nucleotides, and the antisense strand comprises the nucleotide sequence set forth in SEQ ID NO. 16 and has a length of not greater than 25 nucleotides; or, the sense strand comprises the nucleotide sequence set forth in SEQ ID NO. 17 and has a length of not greater than 23 nucleotides, and the antisense strand comprises the nucleotide sequence set forth in SEQ ID NO. 18 and has a length of not greater than 25 nucleotides; or, the sense strand comprises the nucleotide sequence set forth in SEQ ID NO. 19 and has a length of not greater than 23 nucleotides, and the antisense strand comprises the nucleotide sequence set forth in SEQ ID NO. 20 and has a length of not greater than 25 nucleotides; or, the sense strand comprises the nucleotide sequence set forth in SEQ ID NO. 21 and has a length of not greater than 23 nucleotides, and the antisense strand comprises the nucleotide sequence set forth in SEQ ID NO. 22 and has a length of not greater than 25 nucleotides; or, the sense strand

comprises the nucleotide sequence set forth in SEQ ID NO. 23 and has a length of not greater than 23 nucleotides, and the antisense strand comprises the nucleotide sequence set forth in SEQ ID NO. 24 and has a length of not greater than 25 nucleotides; or, the sense strand comprises the nucleotide sequence set forth in SEQ ID NO. 25 and has a length of not greater than 23 nucleotides, and the antisense strand comprises the nucleotide sequence set forth in SEQ ID NO. 26 and has a length of not greater than 25 nucleotides; or, the sense strand comprises the nucleotide sequence set forth in SEQ ID NO. 27 and has a length of not greater than 23 nucleotides, and the antisense strand comprises the nucleotide sequence set forth in SEQ ID NO. 28 and has a length of not greater than 25 nucleotides; or, the sense strand comprises the nucleotide sequence set forth in SEQ ID NO. 29 and has a length of not greater than 21 nucleotides, and the antisense strand comprises the nucleotide sequence set forth in SEQ ID NO. 30 and has a length of not greater than 23 nucleotides; or, the sense strand comprises the nucleotide sequence set forth in SEQ ID NO. 53 and has a length of not greater than 23 nucleotides, and the antisense strand comprises the nucleotide sequence set forth in SEQ ID NO. 54 and has a length of not greater than 25 nucleotides.

**3.** The double-stranded ribonucleic acid, the pharmaceutically acceptable salt thereof, or the ligand conjugate thereof according to claim 1, wherein the sense strand is the nucleotide sequence set forth in SEQ ID NO. 9, and the antisense strand is the nucleotide sequence set forth in SEQ ID NO. 10; or, the sense strand is the nucleotide sequence set forth in SEQ ID NO. 11, and the antisense strand is the nucleotide sequence set forth in SEQ ID NO. 12; or, the sense strand is the nucleotide sequence set forth in SEQ ID NO. 13, and the antisense strand is the nucleotide sequence set forth in SEQ ID NO. 14; or, the sense strand is the nucleotide sequence set forth in SEQ ID NO. 15, and the antisense strand is the nucleotide sequence set forth in SEQ ID NO. 16; or, the sense strand is the nucleotide sequence set forth in SEQ ID NO. 17, and the antisense strand is the nucleotide sequence set forth in SEQ ID NO. 18; or, the sense strand is the nucleotide sequence set forth in SEQ ID NO. 19, and the antisense strand is the nucleotide sequence set forth in SEQ ID NO. 20; or, the sense strand is the nucleotide sequence set forth in SEQ ID NO. 21, and the antisense strand is the nucleotide sequence set forth in SEQ ID NO. 22; or, the sense strand is the nucleotide sequence set forth in SEQ ID NO. 23, and the antisense strand is the nucleotide sequence set forth in SEQ ID NO. 24; or, the sense strand is the nucleotide sequence set forth in SEQ ID NO. 25, and the antisense strand is the nucleotide sequence set forth in SEQ ID NO. 26; or, the sense strand is the nucleotide sequence set forth in SEQ ID NO. 27, and the antisense strand is the nucleotide sequence set forth in SEQ ID NO. 28; or, the sense strand is the nucleotide sequence set forth in SEQ ID NO. 29, and the antisense strand is the nucleotide sequence set forth in SEQ ID NO. 30; or, the sense strand is the nucleotide sequence set forth in SEQ ID NO. 53, and the antisense strand is the nucleotide sequence set forth in SEQ ID NO. 54.

**4.** The double-stranded ribonucleic acid, the pharmaceutically acceptable salt thereof, or the ligand conjugate thereof according to any one of claims 1 to 3, wherein the double-stranded ribonucleic acid, the pharmaceutically acceptable salt thereof, or the ligand conjugate thereof optionally comprises an overhang at the 5' end and/or 3' end.

**5.** The double-stranded ribonucleic acid, the pharmaceutically acceptable salt thereof, or the ligand conjugate thereof according to claim 4, wherein the overhang comprises 1, 2, 3, 4, or 5 nucleotides; or, the overhang comprises 1 or 2 nucleotides; or, the overhang is 1, 2, 3, 4, or 5 nucleotides at the 5'-end and/or 3'-end of the sense strand or the antisense strand; or, the dsRNA, the pharmaceutically acceptable salt thereof, or the ligand conjugate thereof comprises an additional sequence as the overhang.

**6.** The double-stranded ribonucleic acid, the pharmaceutically acceptable salt thereof, or the ligand conjugate thereof according to claim 4 or 5, wherein the additional sequence as the overhang is selected from the group consisting of unmodified or modified A, G, C, U, and T, or is selected from the group consisting of unmodified or modified U and T; or when the overhang is 1 nucleotide, the additional sequence as the overhang is selected from the group consisting of u and dT; or when the overhang is 2 nucleotides, the additional sequence as the overhang is selected from the group consisting of uu and dTdT; or when the overhang is 2 nucleotides, the additional sequence as the overhang is selected from the group consisting of unmodified or modified GA.

**7.** The double-stranded ribonucleic acid, the pharmaceutically acceptable salt thereof, or the ligand conjugate thereof according to any one of claims 4 to 6, wherein the additional sequence as the overhang is linked to an adjacent nucleotide via a phosphate group or a phosphorothioate group; or one or more nucleotides in the additional sequence as the overhang are linked via a phosphate group or a phosphorothioate group.

**8.** The double-stranded ribonucleic acid, the pharmaceutically acceptable salt thereof, or the ligand conjugate thereof according to any one of claims 1 to 7, wherein 1, 2, 3, 4, 5, 6, 7, 8, 9, 10, 11, 12, 13, 14, 15, 16, 17, 18, 19, 20, 21, 22, or 23 nucleotides of the sense strand are modified, or a number of nucleotides within a range formed by any of the foregoing

values are modified; or 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, or 23 or more nucleotides of the sense strand are modified; or all nucleotides of the sense strand are modified; and/or 1, 2, 3, 4, 5, 6, 7, 8, 9, 10, 11, 12, 13, 14, 15, 16, 17, 18, 19, 20, 21, 22, 23, 24, or 25 nucleotides of the antisense strand are modified, or a number of nucleotides within a range formed by any of the foregoing values are modified; or 1 or more, 2 or more, 3 or more, 4 or more, 5 or more, 6 or more, 7 or more, 8 or more, 9 or more, 10 or more, 11 or more, 12 or more, 13 or more, 14 or more, 15 or more, 16 or more, 17 or more, 18 or more, 19 or more, 20 or more, 21 or more, 22 or more, 23 or more, 24 or more, or 25 or more nucleotides of the antisense strand are modified; or all nucleotides of the antisense strand are modified.

**9.** The double-stranded ribonucleic acid, the pharmaceutically acceptable salt thereof, or the ligand conjugate thereof according to any one of claims 1 to 8, wherein the modification is selected from the group consisting of glycosyl modification of nucleotides, modification of bases, modification of linkages between nucleotides, and terminal modification.

**10.** The double-stranded ribonucleic acid, the pharmaceutically acceptable salt thereof, or the ligand conjugate thereof according to claim 8, wherein the glycosyl modification of nucleotides is selected from the group consisting of 2'-dehydroxylation, 2'-fluoro, 2'-amino, 2'-alkyl, 2'-O-alkyl, 2'-O-ether, and 2'-O-alkenyl; or, the glycosyl modification of nucleotides is selected from the group consisting of 2'-dehydroxylation, 2'-fluoro, 2'-amino, 2'-methyl, 2'-ethyl, 2'-methyl-O-methyl, 2'-ethyl-O-methyl, 2'-O-methyl, 2'-O-ethyl, 2'-O-ethyl-O-methyl, and 2'-O-allyl;

the modification of linkages between nucleotides is selected from the group consisting of phosphorothioate (PS), phosphorodithioate (PS2), methylphosphonate (MP), methoxypropylphosphonate (MOP), and aminophosphonate; or, the modification of linkages between nucleotides is selected from the group consisting of phosphorothioate (PS); and/or
the terminal modification is selected from the group consisting of a 5'-end modification and a 3'-end modification; or, the terminal modification is selected from the group consisting of 5'-phosphate, 5'-methylphosphonate (5'-MP), 5'-phosphorothioate (5'-PS), and 5'-(E)-vinylphosphonate (5'-(E)-VP).

**11.** The double-stranded ribonucleic acid, the pharmaceutically acceptable salt thereof, or the ligand conjugate thereof according to any one of claims 1 to 10, wherein a phosphorothioate modification is present between the nucleotides at positions 1 and 2 of the sense strand and/or the nucleotides at positions 2 and 3 of the sense strand; and/or a phosphorothioate modification is present between the nucleotides at positions 1 and 2 of the antisense strand, the nucleotides at positions 2 and 3 of the antisense strand, the nucleotides at positions 1 and 2 of the 3' end of the antisense strand, and/or the nucleotides at positions 2 and 3 of the antisense strand.

**12.** The double-stranded ribonucleic acid, the pharmaceutically acceptable salt thereof, or the ligand conjugate thereof according to any one of claims 1 to 11, wherein 2, 3, 4, or 5 nucleotides of the sense strand are modified by 2'-fluoro, and 14, 15, 16, 17, 18, or 19 nucleotides of the sense strand are modified by 2'-O-methyl; and/or 2, 3, 4, 5, 6, or 7 nucleotides of the antisense strand are modified by 2'-fluoro, and 14, 15, 16, 17, 18, 19, 20, or 21 nucleotides of the antisense strand are modified by 2'-O-methyl.

**13.** The double-stranded ribonucleic acid, the pharmaceutically acceptable salt thereof, or the ligand conjugate thereof according to any one of claims 1 to 12, wherein 0, 1, or 2 of the nucleotides at positions 1 to 6 or 1 to 8 of the sense strand are modified by 2'-fluoro, 4, 5, 6, 7, or 8 of the nucleotides at positions 1 to 6 or 1 to 8 of the sense strand are modified by 2'-O-methyl, 2 or 3 of the nucleotides at positions 7 to 9 or 9 to 11 of the sense strand are modified by 2'-fluoro, 0 or 1 of the nucleotides at positions 7 to 9 or 9 to 11 of the sense strand is modified by 2'-O-methyl, 0 or 1 of the nucleotides at positions 10 to 19 or 12 to 21 of the sense strand is modified by 2'-fluoro, and 8, 9, 10, 11, or 12 of the nucleotides at positions 10 to 19 or 12 to 21 of the sense strand are modified by 2'-O-methyl; or
the nucleotides at positions 7, 8, and 9 of the sense strand are modified by 2'-fluoro, and 0, 1, or 2 of the nucleotides at positions 1 to 6 and/or 10 to 19 of the sense strand are also modified by 2'-fluoro; or the nucleotides at positions 9, 10, and 11 of the sense strand are modified by 2'-fluoro, and 0, 1, or 2 of the nucleotides at positions 1 to 8 and/or 12 to 21 of the sense strand are also modified by 2'-fluoro.

**14.** The double-stranded ribonucleic acid, the pharmaceutically acceptable salt thereof, or the ligand conjugate thereof according to any one of claims 1 to 13, wherein 3 or 4 of the nucleotides at positions 2, 6, 14, and 16 of the antisense strand are modified by 2'-fluoro; or 3, 4, 5, or 6 of the nucleotides at positions 2, 6, 8, 9, 14, and 16 of the antisense strand are modified by 2'-fluoro; or the nucleotides at positions 2, 6, 14, and 16 of the antisense strand are modified by

2'-fluoro, and any one or two of the nucleotides at positions 1, 3 to 5, 7 to 13, 15, 17 to 21, and/or 17 to 23 of the antisense strand are also modified by 2'-fluoro; or 14, 15, 16, 17, 18, or 19 of the nucleotides at positions 1, 3 to 5, 7 to 13, 15, 17 to 21, and/or 17 to 23 of the antisense strand are modified by 2'-O-methyl.

**15.** The double-stranded ribonucleic acid, the pharmaceutically acceptable salt thereof, or the ligand conjugate thereof according to any one of claims 1 to 14, wherein the ligand comprises at least one targeting group; or the ligand comprises one, two, three, four, or five targeting groups.

**16.** The double-stranded ribonucleic acid, the pharmaceutically acceptable salt thereof, or the ligand conjugate thereof according to claim 15, wherein the targeting group is a GalNAc group.

**17.** The double-stranded ribonucleic acid, the pharmaceutically acceptable salt thereof, or the ligand conjugate thereof according to any one of claims 1 to 16, wherein the ligand comprises the following branched groups:

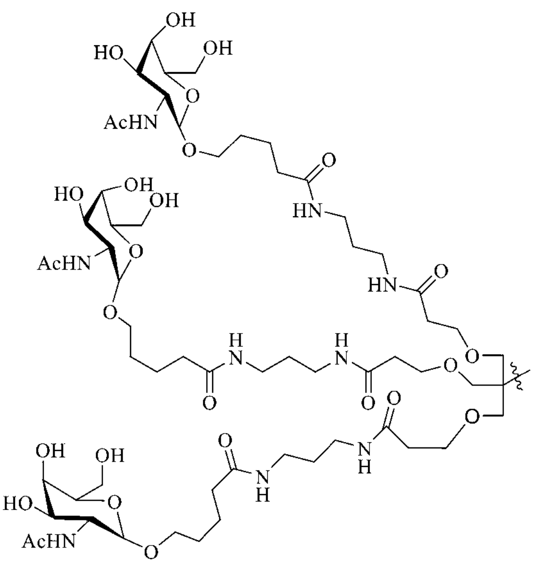

or

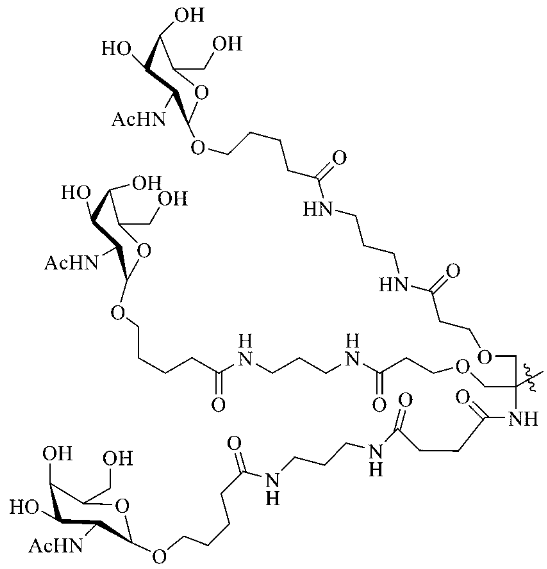

.

**18.** The double-stranded ribonucleic acid, the pharmaceutically acceptable salt thereof, or the ligand conjugate thereof according to any one of claims 1 to 16, wherein the ligand is selected from the group consisting of:

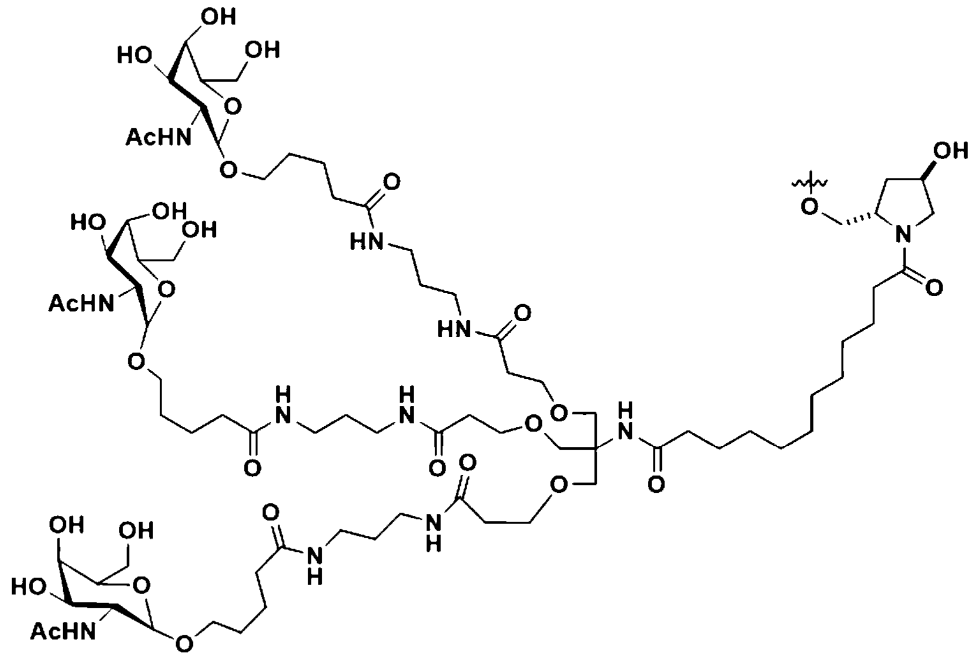

L01 ligand

and

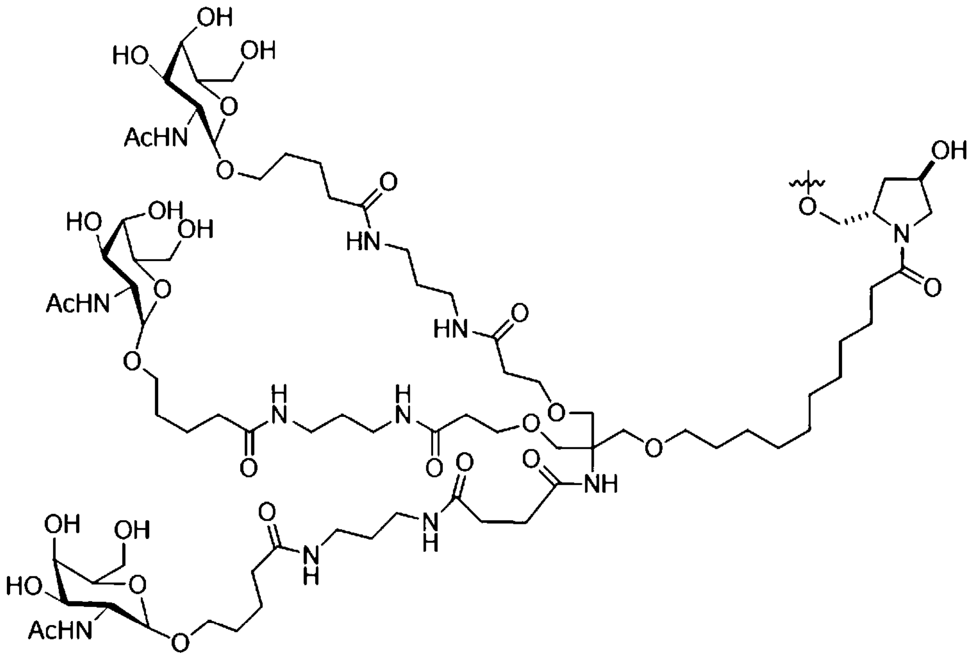

L02 ligand

**19.** The double-stranded ribonucleic acid, the pharmaceutically acceptable salt thereof, or the ligand conjugate thereof according to any one of claims 1 to 18, wherein the ligand is linked to the 5' or 3' end of the sense strand or the antisense strand; or the ligand is linked to the 5' or 3' end of the sense strand; or the ligand is linked to the 3' end of the sense strand.

**20.** The double-stranded ribonucleic acid, the pharmaceutically acceptable salt thereof, or the ligand conjugate thereof according to any one of claims 1 to 19, wherein the double-stranded ribonucleic acid comprises any one of the following sense strands: SEQ ID NO. 9, SEQ ID NO. 11, SEQ ID NO. 13, SEQ ID NO. 15, SEQ ID NO. 17, SEQ ID NO.

19, SEQ ID NO. 21, SEQ ID NO. 23, SEQ ID NO. 25, SEQ ID NO. 27, SEQ ID NO. 29, SEQ ID NO. 53, SEQ ID NO. 31, SEQ ID NO. 33, SEQ ID NO. 35, SEQ ID NO. 37, SEQ ID NO. 39, SEQ ID NO. 41, SEQ ID NO. 43, SEQ ID NO. 45, SEQ ID NO. 47, SEQ ID NO. 49, SEQ ID NO. 51, or SEQ ID NO. 55; and
the double-stranded ribonucleic acid comprises any one of the following antisense strands: SEQ ID NO. 10, SEQ ID NO. 12, SEQ ID NO. 14, SEQ ID NO. 16, SEQ ID NO. 18, SEQ ID NO. 20, SEQ ID NO. 22, SEQ ID NO. 24, SEQ ID NO. 26, SEQ ID NO. 28, SEQ ID NO. 30, SEQ ID NO. 54, SEQ ID NO. 32, SEQ ID NO. 34, SEQ ID NO. 36, SEQ ID NO. 38, SEQ ID NO. 40, SEQ ID NO. 42, SEQ ID NO. 44, SEQ ID NO. 46, SEQ ID NO. 48, SEQ ID NO. 50, SEQ ID NO. 52, or SEQ ID NO. 56.

**21.** The double-stranded ribonucleic acid, the pharmaceutically acceptable salt thereof, or the ligand conjugate thereof according to any one of claims 1 to 20, wherein the double-stranded ribonucleic acid comprises a sequence pair of the sense strand and the antisense strand as follows: SEQ ID NO. 9/SEQ ID NO. 10, SEQ ID NO. 11/SEQ ID NO. 12, SEQ ID NO. 13/SEQ ID NO. 14, SEQ ID NO. 15/SEQ ID NO. 16, SEQ ID NO. 17/SEQ ID NO. 18, SEQ ID NO. 19/SEQ ID NO. 20, SEQ ID NO. 21/SEQ ID NO. 22, SEQ ID NO. 23/SEQ ID NO. 24, SEQ ID NO. 25/SEQ ID NO. 26, SEQ ID NO. 27/SEQ ID NO. 28, SEQ ID NO. 29/SEQ ID NO. 30, SEQ ID NO. 31/SEQ ID NO. 32, SEQ ID NO. 53/SEQ ID NO. 54, SEQ ID NO. 33/SEQ ID NO. 34, SEQ ID NO. 35/SEQ ID NO. 36, SEQ ID NO. 37/SEQ ID NO. 38, SEQ ID NO. 39/SEQ ID NO. 40, SEQ ID NO. 41/SEQ ID NO. 42, SEQ ID NO. 43/SEQ ID NO. 44, SEQ ID NO. 45/SEQ ID NO. 46, SEQ ID NO. 47/SEQ ID NO. 48, SEQ ID NO. 49/SEQ ID NO. 50, SEQ ID NO. 51/SEQ ID NO. 52, or SEQ ID NO. 55/SEQ ID NO. 56.

**22.** The double-stranded ribonucleic acid, the pharmaceutically acceptable salt thereof, or the ligand conjugate thereof according to any one of claims 1 to 20, wherein:

a) the sense strand comprises 5'-asgscaauauGfAfCfaauugaaugaL01-3' (SEQ ID NO. 31), and the antisense strand comprises 5'-usCfsauuCfaauugucAfuAfuugcusasc-3' (SEQ ID NO. 32);
b) the sense strand comprises 5'-asgscaauauGfAfCfaauugaaugaL02-3' (SEQ ID NO. 31), and the antisense strand comprises 5'-usCfsauuCfaauugucAfuAfuugcusasc-3' (SEQ ID NO. 32);
c) the sense strand comprises 5'-asusuuacugUfCfAfcgguucccaaL01-3' (SEQ ID NO. 33), and the antisense strand comprises 5'-usUfsgggAfaccgugaCfaGfuaauasgsc-3' (SEQ ID NO. 34);
d) the sense strand comprises 5'-asusuuacugUfCfAfcgguucccaaL02-3' (SEQ ID NO. 33), and the antisense strand comprises 5'-usUfsgggAfaccgugaCfaGfuaauasgsc-3' (SEQ ID NO. 34);
e) the sense strand comprises 5'-csusauuuAfUfUfuugagucuguL01-3' (SEQ ID NO. 35), and the antisense strand comprises 5'-asCfsagaCfucaaaauAfaAfuagsgsa-3' (SEQ ID NO. 36);
f) the sense strand comprises 5'-csusauuuAfUfUfuugagucuguL02-3' (SEQ ID NO. 35), and the antisense strand comprises 5'-asCfsagaCfucaaaauAfaAfuagsgsa-3' (SEQ ID NO. 36);
g) the sense strand comprises 5'-usgsaagauaUfAfUfuguaguagauL01-3' (SEQ ID NO. 37), and the antisense strand comprises 5'-asUfscuaCfuacaauaUfaUfcuucasasa-3' (SEQ ID NO. 38);
h) the sense strand comprises 5'-usgsaagauaUfAfUfuguaguagauL02-3' (SEQ ID NO. 37), and the antisense strand comprises 5'-asUfscuaCfuacaauaUfaUfcuucasasa-3' (SEQ ID NO. 38);
i) the sense strand comprises 5'-ususgucucaUfGfUfuucaueguaaL01-3' (SEQ ID NO. 39), and the antisense strand comprises 5'-usUfsacgAfugaaacaUfgAfgacaasasa-3' (SEQ ID NO. 40);
j) the sense strand comprises 5'-ususgucucaUfGfUfuucaucguaaL02-3' (SEQ ID NO. 39), and the antisense strand comprises 5'-usUfsacgAfugaaacaUfgAfgacaasasa-3' (SEQ ID NO. 40);
k) the sense strand comprises 5'-csusgugcagUfAfUfcuguuccauuL01-3' (SEQ ID NO. 41), and the antisense strand comprises 5'-asAfsuggAfacagauaCfuGfcacagsasc-3' (SEQ ID NO. 42);
l) the sense strand comprises 5'-csusgugcagUfAfUfcuguuccauuL02-3' (SEQ ID NO. 41), and the antisense strand comprises 5'-asAfsuggAfacagauaCfuGfcacagsasc-3' (SEQ ID NO. 42);
m) the sense strand comprises 5'-asasugaaagGfAfCfucacuugguaL01-3' (SEQ ID NO. 43), and the antisense strand comprises 5'-usAfsccaAfgugagucCfuUfucauusug-3' (SEQ ID NO. 44);
n) the sense strand comprises 5'-asasugaaagGfAfCfucacuugguaL02-3' (SEQ ID NO. 43), and the antisense strand comprises 5'-usAfsccaAfgugagucCfuUfucauusug-3' (SEQ ID NO. 44);
o) the sense strand comprises 5'-gscsugcaugAfUfCfagcuagguaL01-3' (SEQ ID NO. 45), and the antisense strand comprises 5'-usAfsccaUfagcugauCfaUfgcagcsgsg-3' (SEQ ID NO. 46);
p) the sense strand comprises 5'-gscsugcaugAfUfCfagcuagguaL02-3' (SEQ ID NO. 45), and the antisense strand comprises 5'-usAfsccaUfagcugauCfaUfgcagcsgsg-3' (SEQ ID NO. 46);
q) the sense strand comprises 5'-gsgsugcuugGfUfCfuccucuauaaL01-3' (SEQ ID NO. 47), and the antisense strand comprises 5'-usUfsauaGfaggagacCfaAfgcaccsusu-3' (SEQ ID NO. 48);
r) the sense strand comprises 5'-gsgsugcuugGfUfCfuccucuauaaL02-3' (SEQ ID NO. 47), and the antisense

strand comprises 5'-usUfsauaGfaggagacCfaAfgcaccsusu-3' (SEQ ID NO. 48);
s) the sense strand comprises 5'-asusuggucaUfCfCfcagaacuacaL01-3' (SEQ ID NO. 49), and the antisense strand comprises 5'-usGfsuagUfucugggaUfgAfccaaususc-3' (SEQ ID NO. 50);
t) the sense strand comprises 5'-asusuggucaUfCfCfcagaacuacaL02-3' (SEQ ID NO. 49), and the antisense strand comprises 5'-usGfsuagUfucugggaUfgAfccaaususc-3' (SEQ ID NO. 50);
u) the sense strand comprises 5'-usasuuuaUfUfUfugagucugugL01-3' (SEQ ID NO. 51), and the antisense strand comprises 5'-csAfscagAfcucaaaaUfaAfauasgsg-3' (SEQ ID NO. 52);
v) the sense strand comprises 5'-usasuuuaUfUfUfugagucugugL02-3' (SEQ ID NO. 51), and the antisense strand comprises 5'-csAfscagAfcucaaaaUfaAfauasgsg-3' (SEQ ID NO. 52);
w) the sense strand comprises 5'-asusuuacugUfCfAfcgguucccaaL01-3' (SEQ ID NO. 55), and the antisense strand comprises 5'-usUfsgggAfaccgugaCfaGfuaaausgsc-3' (SEQ ID NO. 56);
x) the sense strand comprises 5'-asusuuacugUfCfAfcgguucccaaL02-3' (SEQ ID NO. 55), and the antisense strand comprises 5'-usUfsgggAfaccgugaCfaGfuaaausgsc-3' (SEQ ID NO. 56).

**23.** A pharmaceutical composition, comprising the double-stranded ribonucleic acid, the pharmaceutically acceptable salt thereof, or the ligand conjugate thereof according to any one of claims 1 to 22, and optionally a pharmaceutically acceptable carrier or excipient.

**24.** Use of the double-stranded ribonucleic acid, the pharmaceutically acceptable salt thereof, or the ligand conjugate thereof according to any one of claims 1 to 22 or the pharmaceutical composition according to claim 23 in preparing a medicament for treating and/or preventing hepatitis B virus infection.

**25.** The use according to claim 24, wherein the double-stranded ribonucleic acid, the pharmaceutically acceptable salt thereof, or the ligand conjugate thereof, or the pharmaceutical composition is used in combination with an additional therapeutic agent.

**INTERNATIONAL SEARCH REPORT**

International application No.
**PCT/CN2024/109635**

**A. CLASSIFICATION OF SUBJECT MATTER**

C12N15/113(2010.01)i; A61K31/713(2006.01)i; A61P31/20(2006.01)i

According to International Patent Classification (IPC) or to both national classification and IPC

**B. FIELDS SEARCHED**

Minimum documentation searched (classification system followed by classification symbols)

IPC: C12N, A61K, A61P

Documentation searched other than minimum documentation to the extent that such documents are included in the fields searched

Electronic data base consulted during the international search (name of data base and, where practicable, search terms used)

CNABS, CNTXT, ENTXT, VEN, WPABS, DWPI, CNKI, 万方, WANFANG, EBI, NCBI, 百度, Baidu, ISI Web of Knowledge, PUBMED, STN. 中国专利生物序列检索系统, China Patent Biological Sequence Search System: 正大天晴药业集团股份有限公司, 发明人, Inventors, 乙型病毒性肝炎, 乙肝, Hepatitis B Virus, HBV, 小干扰RNA, small interfering RNA, siRNA, RNA干扰, RNA interference, RNAi, 双链核糖核酸, dsRNA, PD-L1, PDL1, 程序性死亡配体1, B7同系物1, B7-H1, cd274, 相关序列, related sequences

**C. DOCUMENTS CONSIDERED TO BE RELEVANT**

| Category* | Citation of document, with indication, where appropriate, of the relevant passages | Relevant to claim No. |
|---|---|---|
| X | CN 114980903 A (SIRNAOMICS, INC.) 30 August 2022 (2022-08-30)<br>claims 1-23, 49-56 and 79-98, description, table 3, sequences 68-71, and table 5, sequences 170-173, and description, paragraph 115 | 1-25 (in part) |
| A | US 2018273947 A1 (ALNYLAM PHARMACEUTICALS, INC.) 27 September 2018 (2018-09-27)<br>claims 1-106 | 1-25 (in part) |
| A | US 2011251259 A1 (ALNYLAM PHARMACEUTICALS, INC.) 13 October 2011 (2011-10-13)<br>claims 1-20 | 1-25 (in part) |
| A | CN 115176009 A (ALIGOS THERAPEUTICS, INC.) 11 October 2022 (2022-10-11)<br>claims 1-28 | 1-25 (in part) |
| A | CN 108285907 A (SHANDONG UNIVERSITY) 17 July 2018 (2018-07-17)<br>claims 1-9 | 1-25 (in part) |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **10 September 2024** | **14 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

INTERNATIONAL SEARCH REPORT

International application No.
PCT/CN2024/109635

C. DOCUMENTS CONSIDERED TO BE RELEVANT

| Category* | Citation of document, with indication, where appropriate, of the relevant passages | Relevant to claim No. |
|---|---|---|
| A | WO 2023083906 A2 (F. HOFFMANN-LA ROCHE AG) 19 May 2023 (2023-05-19) claims 1-145 | 1-25 (in part) |

Form PCT/ISA/210 (second sheet) (July 2022)

**INTERNATIONAL SEARCH REPORT**

International application No.
**PCT/CN2024/109635**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/109635**

**Box No. III Observations where unity of invention is lacking (Continuation of item 3 of first sheet)**

This International Searching Authority found multiple inventions in this international application, as follows:

Invention 1: the technical solutions of claims 1-25 (in part) relating to SEQ ID NOs: 9-10.

Inventions 2-12: the technical solutions of claims 1-25 (in part) respectively relating to SEQ ID NOs: 11-12, 13-14, 15-16, 17-18, 19-20, 21-22, 23-24, 25-26, 27-28, 29-30 and 53-54.

The same technical feature of inventions 1-12 is: a small interfering nucleotide targeting PD-L1. For a person skilled in the art, a gene and a protein sequence of PD-L1 are well known in the art, and designing a small interfering nucleotide according to the gene is also a conventional technique in the art; moreover, the prior art also discloses a small interfering nucleotide targeting PD-L1 (for example, WO 2021061437 A1, 01 April 2021, table 2). Therefore, inventions 1-12 do not have a same or corresponding special technical feature, and thus do not comply with PCT Rule 13.2.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☑ As only some of the required additional search fees were timely paid by the applicant, this international search report covers only those claims for which fees were paid, specifically claims Nos.: **the technical solutions of claims 1-25 (in part) relate to SEQ ID NOs: 11-12, 13-14 and 53-54**

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

**INTERNATIONAL SEARCH REPORT**
**Information on patent family members**

International application No.
**PCT/CN2024/109635**

| Patent document cited in search report | | | Publication date (day/month/year) | Patent family member(s) | | | Publication date (day/month/year) |
|---|---|---|---|---|---|---|---|
| CN | 114980903 | A | 30 August 2022 | CA | 3151030 | A1 | 01 April 2021 |
| | | | | AU | 2020352441 | A1 | 28 April 2022 |
| | | | | IL | 291297 | A | 01 May 2022 |
| | | | | JP | 2022548085 | A | 16 November 2022 |
| | | | | EP | 4028011 | A1 | 20 July 2022 |
| | | | | EP | 4028011 | A4 | 05 April 2023 |
| | | | | US | 2022282258 | A1 | 08 September 2022 |
| | | | | BR | 112022004563 | A2 | 07 June 2022 |
| | | | | KR | 20220110723 | A | 09 August 2022 |
| | | | | WO | 2021061437 | A1 | 01 April 2021 |
| US | 2018273947 | A1 | 27 September 2018 | | None | | |
| US | 2011251259 | A1 | 13 October 2011 | JP | 2017038600 | A | 23 February 2017 |
| | | | | WO | 2011127180 | A1 | 13 October 2011 |
| | | | | EP | 2555778 | A1 | 13 February 2013 |
| | | | | EP | 2555778 | A4 | 21 May 2014 |
| | | | | US | 2014148497 | A1 | 29 May 2014 |
| | | | | US | 9422562 | B2 | 23 August 2016 |
| | | | | EP | 4385568 | A2 | 19 June 2024 |
| | | | | US | 2021155937 | A1 | 27 May 2021 |
| | | | | JP | 2019047826 | A | 28 March 2019 |
| | | | | JP | 6814788 | B2 | 20 January 2021 |
| | | | | AU | 2011237630 | A1 | 20 September 2012 |
| | | | | AU | 2011237630 | B2 | 21 January 2016 |
| | | | | EP | 3578657 | A1 | 11 December 2019 |
| | | | | EP | 3578657 | B1 | 20 March 2024 |
| | | | | JP | 2013523162 | A | 17 June 2013 |
| | | | | US | 2017067060 | A1 | 09 March 2017 |
| | | | | US | 9932593 | B2 | 03 April 2018 |
| | | | | US | 2019048352 | A1 | 14 February 2019 |
| | | | | US | 10745704 | B2 | 18 August 2020 |
| | | | | US | 8507663 | B2 | 13 August 2013 |
| | | | | CA | 2792561 | A1 | 13 October 2011 |
| | | | | CA | 2792561 | C | 26 October 2021 |
| | | | | JP | 2021078501 | A | 27 May 2021 |
| CN | 115176009 | A | 11 October 2022 | US | 2021277403 | A1 | 09 September 2021 |
| | | | | US | 11939581 | B2 | 26 March 2024 |
| | | | | WO | 2021173811 | A1 | 02 September 2021 |
| | | | | EP | 4110922 | A1 | 04 January 2023 |
| | | | | AU | 2021226366 | A1 | 08 September 2022 |
| | | | | KR | 20220148241 | A | 04 November 2022 |
| | | | | CA | 3172823 | A1 | 02 September 2021 |
| | | | | JP | 2023515218 | A | 12 April 2023 |
| | | | | TW | 202231285 | A | 16 August 2022 |
| CN | 108285907 | A | 17 July 2018 | | None | | |
| WO | 2023083906 | A2 | 19 May 2023 | CA | 3234478 | A1 | 19 May 2023 |
| | | | | KR | 20240101580 | A | 02 July 2024 |
| | | | | AU | 2022384619 | A1 | 11 April 2024 |
| | | | | IL | 312635 | A | 01 July 2024 |
| | | | | WO | 2023083906 | A3 | 13 July 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310982666
- CN 202311654515
- CN 202410114631X
- WO 2009082607 A
- WO 2014025805 A
- WO 2015006740 A
- WO 2021249484 A


### Non-patent literature cited in the description


- **EDWARD J. G. et al.** The oral toll-like receptor-7 agonist GS-9620 in patients with chronic hepatitis B virus infection.. *Journal of Hepatology*, 2015, vol. 63, 320-328
- **JANSSEN et al.** *Lancet*, 2005, vol. 365, 123-129
- **MARCELLIN et al.** *N. Engl.JMed.*, 2004, vol. 351, 1206-1217
- **BUSTER et al.** *Hepatology*, 2007, vol. 46, 388-394